# EUROPEAN PATENT APPLICATION

(11) **EP 4 166 542 A1**
(43) Date of publication of application: **19.04.2023**
(21) Application number: 21820923.7
(22) Date of filing: 10.06.2021
(51) Int. Cl.: C07D 243/36, A61K 31/403, A61P 35/00

(54) **BICYCLIC COMPOUND AND APPLICATION THEREOF**

(30) Priority: 11.06.2020 WO PCT/CN2020/095654; 28.08.2020 WO PCT/CN2020/112063; 01.06.2021 CN 202110610887
(71) Applicant: Betta Pharmaceuticals Co., Ltd, Yuhang Hangzhou Zhejiang 311100 (CN)
(72) Inventor: YANG, Rongwen, Beijing 100176 (CN); SUN, Yun, Beijing 100176 (CN); ZHANG, Jian, Beijing 100176 (CN); LIU, Xiangkai, Beijing 100176 (CN); WANG, Pingping, Beijing 100176 (CN); YI, Xuegang, Beijing 100176 (CN); MA, Teng, Beijing 100176 (CN); HUANG, Chuanlong, Beijing 100176 (CN); LAN, Hong, Beijing 100176 (CN); DING, Lieming, Beijing 100176 (CN); WANG, Jiabing, Beijing 100176 (CN)
(74) Representative: Casalonga
(86) International application number: PCT/CN2021/099316
(87) International publication number: WO 2021/249463

(57) **Abstract**

The present invention relates to compounds of formula (I). The present invention also provides compositions and formulations containing such compounds, and methods for using and preparing such compounds.

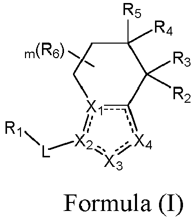

## Description

### FIELD OF THE INVENTION

The present invention relates to bicyclic compounds, pharmaceutical compositions and formulations containing such compounds, and methods for using and preparing such compounds.

### BACKGROUND OF THE INVENTION

HIFs (Hypoxia inducible factors) are members of transcription factors family, which respond to the changes of oxygen in body, known as hypoxia inducible factors; HIFs mediate the effects of hypoxia through regulating over 40 hypoxia-adaptive genes downstream. HIFs consist of three α-subunits (HIF1α, HIF2α and HIF3α) and one β-subunit (HIP1β), wherein HIF-1β is always in the nucleus; HIFα is located in the cytoplasm. In conditions of sufficient oxygen, HIFα is hydroxylated by prolyl-hydroxylases, allowing its recognition and ubiquitination by the VHL E3 ubiquitin ligase, and then degraded by the proteasome. In hypoxic conditions, HIFα can't be degraded and is stabilized in the nucleus, then binds to HIF-1β to form heterodimers to recognize and activate HRE (Hypoxia response element) within downstream gene promoters, which can upregulate the gene transcription allowing cells survive in hypoxic conditions. These genes relate to tumor angiogenesis, cell proliferation, survival, metabolism, invasion and metastasis, drug resistance, inflammation and immunity, etc. HIF-2α mediates chronic hypoxia and plays a more important role in the development of tumor, which sustainably activated under physiological hypoxia conditions.

Current studies have shown that the mechanism of HIF-2α-mediated tumorigenesis and development mainly includes: 1. Under conditions such as hypoxia or VHL mutation, HIF-2α can't be degraded and is stabilized in the nucleus, then binds to HIF-1β to form heterodimer to recognize and activate HRE (Hypoxia response element) within downstream gene promoters, which can upregulate VEGFA , CXCR4, Cyclin D1 and other cancer-related genes in the downstream and promote tumor angiogenesis; 2. HIF-2α also participates in immunosuppressive signaling by upregulating CD73 expression, Therefore, targeting HIF-2α can restore or enhance the anti-tumor immune function of mature DC cells, activate B cells and NK cells.

The activation of HIF-2α pathway is closely related to the occurrence and development of renal cell carcinoma, glioma, neuroblastoma and pheochromocytoma. VHL protein is an impotant part of E3 ubiquitin ligase, which mediates the degradation of proteins by the proteasome. The VHL gene has a 57% mutation rate or 98% loss of heterozygosity in renal cell carcinoma (RCC), causes pseudohypoxia and induces HIF-2α activation in the nucleus. Clear cell renal cell carcinoma (ccRCC) accounts for 70%-75% of primary renal cell malignancies and more than 90% of ccRCC patients have VHL protein deficiency.

In the absence of vascularization in gliomas, the unstable blood supply leads to a hypoxic microenvironment, which induces local high expression of HIF-2α and promotes tumor growth. In pheochromocytoma and paraganglioma, the 529-532 AA mutation rate of HIF-2α is as high as 81%, which affects the hydroxylation and degradation of HIF-2α directly and makes HIF-2α constantly activated.

The heterodimer formed by activated HIF-2α and HIF-1β is the key process leading the downstream activation, the binding site of the heterodimer is PAS binding domain, which is the foundation of the HIF-2α small molecular inhibitor PT2977 developed by Peloton, PT2977 exerts the anti-tumor efficacy through inhibiting the binding of HIF-2α and HIF-1β.

In view of the close relationship between HIF-2α and tumorigenesis and migration, it is necessary to develop more efficient new molecular entity.

### SUMMARY OF THE INVENTION

The present invention first provides a compoud of formula (I) or a stereoisomer, tautomer, pharmaceutically acceptable salt, prodrug, chelate, non-covalent complex, or solvate thereof, 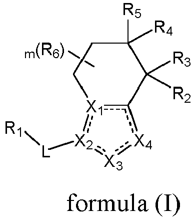 wherein,
- -̅ -̅ -̅ -̅ -̅ -̅: represents a single bond or a double bond;
- L: is a bond, -O-, -NH-, -(CR_{d}Rₑ)ₙ-, -S-, -S(=O)-, -S(=O)₂-, -C=C-, -C=C- or C₃-C₅ cycloalkyl;
- X₁ and X₂: are each independently selected from C or N;
- X₃: is CR_{f} or NR_{f};
- X₄: is CRⱼ or NRⱼ; and at least one of X₁, X₂, X₃ or X₄ is N;
- R₁: is selected from C₁-C₁₀ alkyl, C₂-C₁₀ alkenyl, C₂-C₁₀ alkynyl, C₁-C₁₀ alkoxyl, C₆-C₁₀ aryl, 5-18 membered heteroaryl, C₃-C₁₀ cycloalkyl, or 3-10 membered heterocyclyl; wherein, the 5-18 membered heteroaryl and the 3-10 membered heterocyclyl optionally including 1, 2 or 3 hetero atoms independently selected from N, O or S; the C₁-C₁₀ alkyl, C₂-C₁₀ alkenyl, C₂-C₁₀ alkynyl, C₁-C₁₀ alkoxyl, C₆-C₁₀ aryl, 5-18 membered heteroaryl, C₃-C₁₀ cycloalkyl and 3-10 membered heterocyclyl are each optionally substituted with one or more halogen, -OH, -CN, oxo, amino, C₁-C₆ alkyl, C₁-C₆ alkoxyl, C₂-C₆ alkenyl, C₃-C₅ cycloalkyl, C₂-C₆ alkynyl, C₁-C₆ haloalkyl, -C₁-C₆ alkylene-OR_{c}, -C₀-C₆ alkylene-C=O-R_{c}, -NO₂,-OR_{c}, -SR_{c}, -NRₐR_{b}, -C(=O)R_{c}, -C(=O)OR_{c}, -C(=O)NRₐR_{b}, -NC(=O)R_{c},-S(=O)R_{c}, -S(=O)₂R_{c}, -S(=O)₂NRₐR_{b}, -S(=O)(=NRₐ)R_{b}, -P(=O)RₐR_{b} and/or-P(=S)RₐR_{b};
- R₂: is selected from H, deuterium, halogen, -CN, -OH, =N-OH, C₁-C₅ haloalkyl, amino, C₁-C₁₀ alkoxyl, -O-C(=O)-C₁₋₃ alkyl, -C(=O)-O-C₁₋₃alkyl or -NRₐR_{b};
- R₃: is selected from H, deuterium, halogen, -CN, -OH, =N-OH, C₁-C₁₀ alkyl, C₂-C₁₀ alkenyl, C₁-C₅ haloalkyl, C₁-C₁₀ alkoxyl, -O-C(=O)-C₁₋₃ alkyl, -C(=O)-O-C₁₋₃ alkyl or C₂-C₁₀ alkynyl, the C₁-C₁₀ alkyl, C₂-C₁₀ alkenyl, C₁-C₅ haloalkyl, C₁-C₁₀ alkoxyl, -O-C(=O)-C₁₋₃ alkyl, -C(=O)-O-C₁₋₃ alkyl are each optionally substituted with one or more H, halogen, -CN, -OH, amino, C₁-C₅ alkyl, C₂-C₆ alkenyl and/or C₁-C₅ haloalkyl; or R₂ and R₃ together form an oxo group;
- R₄ and R₅: are each independently selected from H, halogen, -OH, C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl or 3-6 membered heterocyclyl; the C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl and 3-6 membered heterocyclyl are each optionally substituted with one or more halogen, -CN, -OH, amino, C₁-C₅ alkyl, C₂-C₆ alkenyl and/or C₁-C₅ haloalkyl; or R₄ and R₅ together with the C atom to which they are attached form a substituted or unsubstituted cyclopropyl;
- R₆: is selected from H, -CN, halogen, -OH, -NO₂, -NH₂, oxo, C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl or 3-6 membered heterocyclyl; the C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl and 3-6 membered heterocyclyl are each optionally substituted with one or more H, halogen, -CN, -OH, amino, oxo, C₁-C₅ alkyl, C₂-C₆ alkenyl and/or C₁-C₅ haloalkyl; or two R₆ together with the C atom to which they are attached form a substituted or unsubstituted C₃-C₅ cycloalkyl or 3-5 membered heterocyclyl;
- or R₆ and R₅: together with the C atom to which they are attached form a substituted or unsubstituted C₃-C₄ cycloalkyl;
- R_{d} and Rₑ: are each independently selected from H, halogen, CN, -NRₐR_{b}, C₁-C₁₀ alkyl or C₃-C₁₀ cycloalkyl; or R_{d} and Rₑ together form an oxo group;
- R_{f}: is selected from absent, H, -CN, halogen, C₁-C₁₀ alkyl, C₁-C₁₀ haloalkyl, C₃-C₁₀ cycloalkyl, oxo or -NRₐR_{b}; the C₁-C₁₀ alkyl, C₁-C₁₀ haloalkyl and C₃-C₁₀ cycloalkyl are each optionally substituted with one or more H, halogen, -CN,-OH, amino, C₁-C₅ alkyl, C₂-C₆ alkenyl and/or C₁-C₅ haloalkyl;
- Rⱼ: is selected from H, -NO₂, -CN, halogen, C₁-C₁₀ alkyl, C₂-C₁₀ alkenyl, C₂-C₁₀ alkynyl, C₁-C₁₀ haloalkyl, C₃-C₁₀ cycloalkyl, 3-10 membered heterocyclyl, C₆-C₁₀ aryl, 5-10 membered heteroaryl, -C₁-C₆ alkylene-OR_{c}, -OR_{c}, -SR_{c}, -NRₐR_{b},-C(=O)R_{c}, -C(=O)OR_{c}, -C(=O)NRₐR_{b}, -NC(=O)R_{c}, -S(=O)R_{c}, -S(=O)₂R_{c},-S(=O)₂NRₐR_{b}, -S(=O)(=NRₐ)R_{b}, P(=O)RₐR_{b}, or P(=S)RₐR_{b}; wherein, the 5-10 membered heteroaryl and 3-10 membered heterocyclyl optionally including 1, 2 or 3 heteroatoms independently selected from N, O or S; the C₁-C₁₀ alkyl, C₂-C₁₀ alkenyl, C₂-C₁₀ alkynyl, C₁-C₁₀ haloalkyl, C₃-C₁₀ cycloalkyl, 3-10 membered heterocyclyl, C₆-C₁₀ aryl and 5-10 membered heteroaryl are each optionally substituted with one or more halogen, hydroxyl, amino, P(=O)Me₂, P(=S)Me₂,-S(=O)₂-C₁-C₃ alkyl, -S(=O)₂-C₃-C₅ cycloalkyl, -S(=O)-C₁-C₃ alkyl, -S(=O)-C₃-C₅ cycloalkyl, CN, C₁-C₆ alkyl, C₁-C₆ alkoxyl and/or C₁-C₆ haloalkyl;
- Rₐ, R_{b} and R_{c}: are each independently selected from H, C₁-C₁₀ alkyl, C₁-C₁₀ haloalkyl, C₂-C₁₀ alkenyl, C₂-C₁₀ alkynyl, C₃-C₁₀ cycloalkyl, 3-10 membered heterocyclyl, C₆-C₁₀ aryl or 5-10 membered heteroaryl;
- n: is 1, 2 or 3;
- m: is 0, 1, 2, 3 or 4.

In some embodiments, wherein,
- -̅ -̅ -̅ -̅ -̅ -̅: represents a single bond or a double bond;
- L: is a bond, -O-, -CR_{d}Rₑ-, -S-, -S(=O)-, -S(=O)₂-, -C=C- or -C=C-;
- X₁: and X₂ are each independently selected from C or N;
- X₃: is CR_{f} or NR_{f};
- X₄: is CRⱼ or NRⱼ; and at least one of X₁, X₂, X₃ or X₄ is N;
- R₁: is selected from substituted or unsubstituted C₁-C₁₀ alkyl, C₂-C₁₀ alkenyl, C₂-C₁₀ alkynyl, C₁-C₁₀ alkoxyl, C₆-C₁₀ aryl, 5-18 membered heteroaryl, C₃-C₁₀ cycloalkyl, or 3-10 membered heterocyclyl; wherein, the 5-18 membered heteroaryl and 3-10 membered heterocyclyl optionally including 1, 2 or 3 heteroatoms each independently selected from N, O or S; the R₁ is optionally substituted with one or more halogen, hydroxyl, CN, oxo, amino, C₁₋₆ alkyl, C₂₋₆ alkenyl, C₃₋₅ cycloalkyl, C₂₋₆ alkynyl or C₁₋₆ haloalkyl;
- R₂: is selected from H, -OH, amino, C₁-C₁₀ alkoxyl, -O-C₁-C₃alkylacyl or -NRₐR_{b};
- R₃: is selected from H, -OH, C₁-C₁₀ alkyl, C₂-C₁₀ alkenyl, C₁-C₁₀ alkoxyl, -O-C₁₋₃ alkylacyl or C₂-C₁₀ alkynyl; or R₂ and R₃ together form an oxo group;
- R₄ and R₅: are each independently selected from H, halogen, -OH, C₁-C₆ alkyl or C₁-C₆ haloalkyl; or R₄ and R₅ and together with the C atom to which they are attached form a substituted or unsubstituted cyclopropyl;
- R₆: is selected from H, -CN, halogen, C₁-C₆ alkyl, or C₁-C₆ haloalkyl; or two R₆ together with the C atom to which they are attached form a substituted or unsubstituted C₃-C₅ cycloalkyl or 3-5 membered heterocyclyl;
or R₆ and R₅ together with the C atom to which they are attached form a substituted or unsubstituted C₃-C₄ cycloalkyl;
- R_{d} and Rₑ: are each independently selected from H, halogen, CN, -NRₐR_{b}, C₁-C₁₀ alkyl or C₃-C₁₀ cycloalkyl; or R_{d} and Rₑ together form an oxo group;
- R_{f}: is selected from H, -CN, halogen, C₁-C₁₀ alkyl, C₁-C₁₀ haloalkyl, C₃-C₁₀ cycloalkyl, oxo, or -NRₐR_{b};
- Rⱼ: is selected from H, -NO₂, -CN, halogen, C₁-C₁₀ alkyl, C₂-C₁₀ alkenyl, C₂-C₁₀ alkynyl, C₁-C₁₀ haloalkyl, C₃-C₁₀ cycloalkyl, 3-10 membered heterocyclyl, C₆-C₁₀ aryl, 5-10 membered heteroaryl, -OR_{c}, -SR_{c}, -NRₐR_{b}, -C(=O)R_{c}, -C(=O)OR_{c}, -C(=O)NRₐR_{b}, -NC(=O)R_{c}, -S(=O)R_{c}, -S(=O)₂R_{c}, -S(=O)₂NRₐR_{b},-S(=O)(=NRₐ)R_{b}, P(=O)RₐR_{b}, or P(=S)RₐR_{b}; wherein, the 5-10 membered heteroaryl and 3-10 membered heterocyclyl optionally including 1, 2 or 3 heteroatoms each independently selected from N, O or S; the Rⱼ is optionally substituted with halogen, P(=O)Me₂, P(=S)Me₂, -S(=O)₂-C₁₋₃ alkyl, -S(=O)₂-C₃₋₅ cycloalkyl, -S(=O)-C₁₋₃ alkyl, -S(=O)-C₃₋₅ cycloalkyl, CN, C₁₋₆ alkyl or C₁₋₆ haloalkyl;
- Rₐ, R_{b} and R_{c}: are each independently selected from H, C₁-C₁₀ alkyl, C₁-C₁₀ haloalkyl, C₂-C₁₀ alkenyl, C₂-C₁₀ alkynyl, C₃-C₁₀ cycloalkyl, 3-10 membered heterocyclyl, C₆-C₁₀ aryl or 5-10 membered heteroaryl;
- m: is 0, 1, 2, 3 or 4.

In some embodiments, wherein,
- L: is bond, -O-, NH-, -CR_{d}Rₑ-, -S-, -S(=O)-, -S(=O)₂-, -C=C- or -C=C-;
- X₁: and X₂ are C or N;
- X₃: is CR_{f} or NR_{f};
- X₄: is CRⱼ or NRⱼ; and at least one of X₁, X₂ or X₄ is N;
- R₁: is selected from C₁-C₁₀ alkyl, C₂-C₁₀ alkenyl, C₂-C₁₀ alkynyl, C₁-C₁₀ alkoxyl, C₆-C₁₀ aryl, C₅-C₁₈ heteroaryl, C₃-C₁₀ cycloalkyl, or C₃-C₁₀ heterocyclyl; wherein, the C₅-C₁₈ heteroaryl and C₃-C₁₀ heterocyclyl optionally including 1, 2 or 3 heteroatoms each independently selected from N, O or S; the C₁-C₁₀ alkyl, C₂-C₁₀ alkenyl, C₂-C₁₀ alkynyl, C₁-C₁₀ alkoxyl, C₆-C₁₀ aryl, C₅-C₁₈ heteroaryl, C₃-C₁₀ cycloalkyl and C₃-C₁₀ heterocyclyl are each optionally substituted with H, halogen, hydroxyl, CN, oxo, amino, C₁₋₆ alkyl, C₁₋₆ alkoxyl, C₂₋₆ alkenyl, C₃₋₅ cycloalkyl, C₂₋₆ alkynyl or C₁₋₆ haloalkyl;
- R₂: is selected from H, -OH, oxo, amino, C₁-C₁₀ alkoxyl, -O-C₁₋₃ alkylacyl or -NRₐR_{b};
- R₃: is selected from H, -OH, oxo, C₁-C₁₀ alkyl, C₂-C₁₀ alkenyl, C₂-C₁₀ alkynyl, C₁-C₁₀ alkoxyl, C₁-C₁₀ haloalkyl or -O-C₁₋₃ alkylacyl;
- R₄ and R₅: are each independently selected from H, halogen, -OH, C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl or C₃-C₆ heterocyclyl; or R₄ and R₅ and together with the C atom to which they are attached form a substituted or unsubstituted cyclopropyl;
- R₆: is selected from H, -CN, halogen, -OH, C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl or C₃-C₆ heterocyclyl; or two R₆ together with the C atom to which they are attached form a substituted or unsubstituted C₃-C₅ cycloalkyl or C₃-C₅ heterocyclyl;
- or R₆ and R₅: together with the C atom to which they are attached form a substituted or unsubstituted C₃-C₄ cycloalkyl;
- R_{d} and Rₑ: are each independently selected from H, halogen, CN, -NRₐR_{b}, C₁-C₁₀ alkyl or C₃-C₁₀ cycloalkyl; or R_{d} and Rₑ together form an oxo group;
- Rⱼ: is selected from H, -NO₂, -CN, halogen, C₁-C₁₀ alkyl, C₂-C₁₀ alkenyl, C₂-C₁₀ alkynyl, C₁-C₁₀ haloalkyl, C₃-C₁₀ cycloalkyl, C₃-C₁₀ heterocyclyl, C₆-C₁₀ aryl, C₅-C₁₀ heteroaryl, -OR_{c}, -SR_{c}, -NRₐR_{b}, -C(=O)R_{c}, -C(=O)OR_{c}, -C(=O)NRₐR_{b},-NC(=O)R_{c}, -S(=O)R_{c}, -S(=O)₂R_{c}, -S(=O)₂NRₐR_{b}, -S(=O)(=NRₐ)R_{b}, P(=O)RₐR_{b}, or P(=S)RₐR_{b}; wherein, the C₅-C₁₀ heteroaryl and C₃-C₁₀ heterocyclyl optionally including 1, 2 or 3 heteroatoms each independently selected from N, O or S; the C₁-C₁₀ alkyl, C₂-C₁₀ alkenyl, C₂-C₁₀ alkynyl, C₁-C₁₀ haloalkyl, C₃-C₁₀ cycloalkyl, C₃-C₁₀ heterocyclyl, C₆-C₁₀ aryl and C₅-C₁₀ heteroaryl are each optionally substituted with H, halogen, hydroxyl, CN, oxo, amino, C₁-C₆ alkyl, C₁₋₆ alkoxyl, C₁-C₆ haloalkyl, C₂-C₆ alkenyl, C₂-C₆ alkynyl, C₃-C₅ cycloalkyl, C₃-C₆ heterocyclyl, P(=O)Me₂, P(=S)Me₂, -S(=O)₂-C₁₋₃alkyl, -S(=O)₂-C₃₋₅ cycloalkyl,-S(=O)-C₁₋₃ alkyl or -S(=O)-C₃₋₅ cycloalkyl;
- R_{f}: is selected from absent, H, -CN, halogen, C₁-C₁₀ alkyl, C₁-C₁₀ haloalkyl, C₃-C₁₀ cycloalkyl, oxo, or -NRₐR_{b};
- Rₐ, R_{b} and R_{c}: are each independently selected from H, C₁-C₁₀ alkyl, C₁-C₁₀ haloalkyl, C₂-C₁₀ alkenyl, C₂-C₁₀ alkynyl, C₃-C₁₀ cycloalkyl, C₃-C₁₀ heterocyclyl, C₆-C₁₀ aryl or C₅-C₁₀ heteroaryl;
- m: is 0, 1, 2, 3 or 4.

Preferably, in some embodiments, the compound is represented by formula (II): 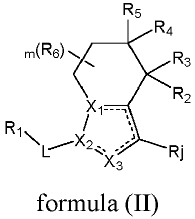 wherein, one and only one of X₁ or X₂ is N, and the other is C.

Preferably, in some embodiments, the compound is represented by formula (III-1) or formula (III-2):

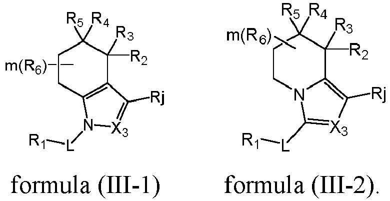

Preferably, in some embodiments, the compound is represented by formula (III-A-1), formula (III-A-2) or formula (III-A-3),

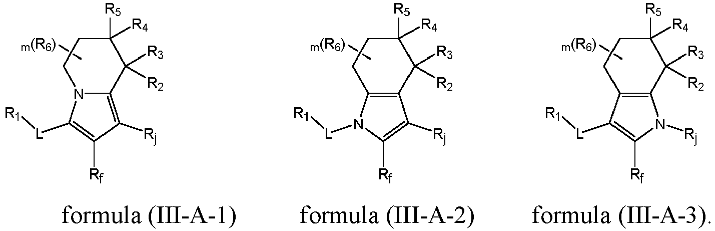

Preferably, in some embodiments, R₂ is halogen, -CN, -OH or =N-OH.

Preferably, in some embodiments, R₂ is halogen, -CN or -OH.

Preferably, in some embodiments, R₂ is F, -CN or -OH.

Preferably, in some embodiments, R₂ is halogen.

Preferably, in some embodiments, R₂ is -CN.

Preferably, in some embodiments, R₂ is -OH.

Preferably, in some embodiments, R₂ and R₃ together form an oxo group.

Preferably, in some embodiments, R₃ is selected from H, deuterium, C₁-C₁₀ alkyl or C₂-C₁₀ alkenyl; the C₁-C₁₀ alkyl and C₂-C₁₀ alkenyl are each optionally substituted with H, halogen, -CN, -OH, amino, or C₁-C₅ haloalkyl; or R₂ and R₃ together form an oxo group.

Preferably, in some embodiments, R₃ is selected from H, deuterium, C₁-C₃ alkyl or C₂-C₅ alkenyl; the C₁-C₃ alkyl and C₂-C₅ alkenyl are each optionally substituted with H, halogen, -CN, -OH, amino or C₁-C₅ haloalkyl.

Preferably, in some embodiments, R₃ is selected from H, deuterium, or C₁-C₃ alkyl, the C₁-C₃ alkyl and C₂-C₅ alkenyl are each optionally substituted with H, halogen, - CN, -OH, amino or C₁-C₅ haloalkyl.

Preferably, in some embodiments, R₃ is selected from H, deuterium or C₁-C₃ alkyl.

Preferably, in some embodiments, R₃ is H ordeuterium.

Preferably, in some embodiments, R₃ is H.

Preferably, in some embodiments, R₂ is -OH, -CN or halogen, and R₃ is H ordeuterium; or R₂ and R₃ together form an oxo group.

Preferably, in some embodiments, R₂ is -OH, -CN or -F, and R₃ is H or deuterium; or R₂ and R₃ together form an oxo group.

Preferably, in some embodiments, R₂ is -OH, and R₃ is H or deuterium; or R₂ and R₃ together form an oxo group.

Preferably, in some embodiments, the compound is represented by formula (IV-1) or formula (IV-2), 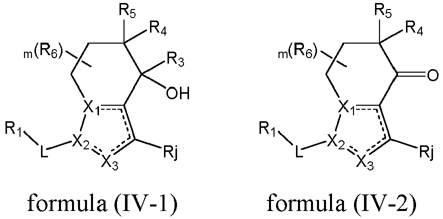 wherein, one and only one of X₁ and X₂ is N, and the other is C.

Preferably, in some embodiments, R₄ and R₅ are each independently selected from H, halogen or C₁-C₆ alkyl, the C₁-C₆ alkyl is optionally substituted with H, halogen, -CN, -OH, amino or oxo.

Preferably, in some embodiments, R₄ and R₅ are each independently selected from H, halogen or C₁-C₆ alkyl.

Preferably, in some embodiments, R₄ and R₅ are each independently selected from H or halogen.

Preferably, in some embodiments, R₄ and R₅ are each independently selected from H or F.

Preferably, in some embodiments, X₄ is CRⱼ.

Preferably, in some embodiments, the compound is represented by formula (V), 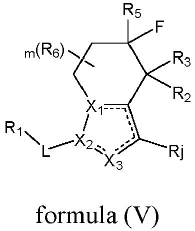 wherein, one and only one of X₁ and X₂ is N, and the other is C.

Preferably, in some embodiments, L is a bond, -CH₂-, -S(=O)₂-, -C=C-, -C=O-, -C=Cor C₃-C₅ cycloalkyl.

Preferably, in some embodiments, L is a bond.

Preferably, in some embodiments, the compound is represented by formula (VI-1), formula (VI-2), formula (VI-3), formula (VI-4) or formula (VI-5):

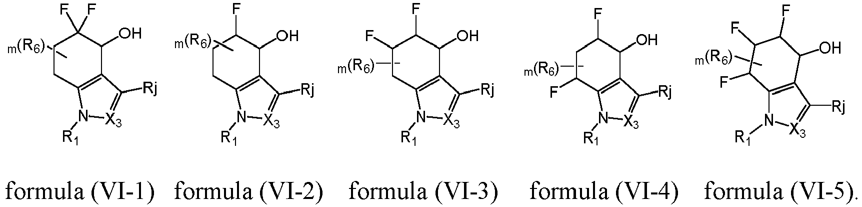

Preferably, in some embodiments, X₃ is CR_{f}.

Preferably, in some embodiments, R_{f} is selected from H, -CN, -NH₂, halogen, C₁-C₃ alkyl, cyclopropyl or C₁-C₃ haloalkyl, the C₁-C₃ alkyl, cyclopropyl or C₁-C₃ haloalkyl are each optionally substituted with H, halogen, -CN, -OH, amino, C₁-C₃ alkyl, C₂-C₄ alkenyl, or C₁-C₃ haloalkyl.

Preferably, in some embodiments, X₃ is CR_{f}, the R_{f} is selected from H, -CN, halogen, C₁-C₃ alkyl or cyclopropyl.

Preferably, in some embodiments, R_{f} is H, -CN, halogen, C₁-C₆ alkyl or C₁-C₃ haloalkyl.

Preferably, in some embodiments, R_{f} is H, -CN, halogen, C₁-C₃ alkyl or C₁-C₃ haloalkyl.

Preferably, in some embodiments, R_{f} is H, -CN or halogen.

Preferably, in some embodiments, R_{f} is H or halogen.

Preferably, in some embodiments, R_{f} is H, -CN, -F, -Cl, -Br or -CF₃.

Preferably, in some embodiments, R_{f} is H.

Preferably, in some embodiments, R₆ is H, -CN, halogen, C₁-C₆ alkyl, C₁-C₆ alkoxyl or C₁-C₆ haloalkyl, the C₁-C₆ alkyl, C₁-C₆ alkoxyl or C₁-C₆ haloalkyl are each optionally substituted with H, halogen, -CN, -OH, oxo, or amino.

Preferably, in some embodiments, R₆ is H, halogen or C₁-C₆ alkyl, the C₁-C₆ alkyl is optionally substituted with H, halogen, -CN, -OH, oxo, or amino.

Preferably, in some embodiments, R₆ is H, halogen or C₁-C₆ alkyl.

Preferably, in some embodiments, R₆ is H, halogen or C₁-C₃ alkyl.

Preferably, in some embodiments, R₆ is H or halogen.

Preferably, in some embodiments, R₆ is H, F, Cl or Br.

Preferably, in some embodiments, R₆ is H or F.

Preferably, in some embodiments, two R₆ together with the C atom to which they are attached form a substituted or unsubstituted cyclopropyl.

Preferably, in some embodiments, R₁ is C₆-C₁₀ aryl, 5-18 membered heteroaryl or C₃-C₁₀ cycloalkyl, the 5-18 membered heteroaryl optionally including 1, 2 or 3 heteroatoms each independently selected from N, O or S.

Preferably, in some embodiments, R₁ is C₆-C₁₀ aryl or 5-18 membered heteroaryl, the 5-18 membered heteroaryl optionally including 1, 2 or 3 heteroatoms each independently selected from N, O or S.

Preferably, in some embodiments, R₁ is C₆-C₁₀ aryl or 5-18 membered heteroaryl, the 5-18 membered heteroaryl optionally including 1, 2 or 3 heteroatoms independently selected from N, O or S, the C₆-C₁₀ aryl or 5-18 membered heteroaryl are each optionally substituted with one or more H, halogen, hydroxyl, CN, amino, C₁₋₄ alkyl, C₃₋₅ cycloalkyl or C₁₋₆ haloalkyl.

Preferably, in some embodiments, R₁ is C₆-C₁₀ aryl or 5-18 membered heteroaryl, the 5-18 membered heteroaryl optionally including 1, 2 or 3 heteroatoms independently selected from N, O or S, the C₆-C₁₀ aryl or 5-18 membered heteroaryl are each optionally substituted with one or more halogen, CN and/or C₁₋₆ haloalkyl.

Preferably, in some embodiments, R₁ is C₆-C₈ aryl, 5-8 membered heteroaryl or C₃-C₆ cycloalkyl, the 5-8 membered heteroaryl optionally including 1, 2 or 3 heteroatoms independently selected from N, O or S, the C₆-C₈ aryl, 5-8 membered heteroaryl or C₃-C₆ cycloalkyl are each optionally substituted with one or more halogen, -OH, -CN, oxo, amino, C₁-C₆ alkyl, C₁-C₆ alkoxyl, C₁-C₆ haloalkyl, -C₁-C₆ alkylene-OR_{c}, -C₀-C₆ alkylene-C=O-R_{c}, -NO₂, C(=O)OR_{c} and/or -S(=O)₂R_{c}.

Preferably, in some embodiments, R₁ is phenyl, 5-6 membered heteroaryl or C₃-C₆ cycloalkyl, the 5-6 membered heteroaryl optionally including 1, 2 or 3 heteroatoms independently selected from N, O or S, the phenyl, 5-6 membered heteroaryl or C₃-C₆ cycloalkyl are each optionally substituted with one or more halogen, -CN and/or C₁-C₄ haloalkyl.

Preferably, in some embodiments, R₁ is phenyl or 5-6 membered heteroaryl, the 5-6 membered heteroaryl optionally including 1, 2 or 3 heteroatoms is selected from N, O or S.

Preferably, in some embodiments, R₁ is phenyl or 6-membered heteroaryl, the 6-membered heteroaryl optionally including 1, 2 or 3 N heteroatoms.

Preferably, in some embodiments, R₁ is phenyl or pyridyl.

Preferably, in some embodiments, the R₁ is phenyl, the phenyl is optionally substituted with one or more halogen, -CN and/or C₁-C₄haloalkyl.

Preferably, in some embodiments, the R₁ is phenyl, the phenyl is optionally substituted with one or more halogen, -CN, -CHF₂, -CF₃, -CH₂CHF₂ and/or -CH₂CF₃.

Preferably, in some embodiments, the R₁ is phenyl, the phenyl is optionally substituted with one or more halogen and/or -CN.

Preferably, in some embodiments, the R₁ is pyridyl, the pyridyl is optionally substituted with one or more halogen, -CN and/or C₁-C₄haloalkyl.

Preferably, in some embodiments, the R₁ is pyridyl, the pyridyl is optionally substituted with one or more halogen, CN and/or trifluoromethyl.

Preferably, in some embodiments, the Rⱼ is selected from H, -CN, halogen, C₁-C₁₀ alkyl, C₂-C₁₀ alkenyl, C₁-C₁₀ haloalkyl, C₃-C₁₀ cycloalkyl, C₆-C₁₀ aryl, 5-10 membered heteroaryl, -S(=O)R_{c}, -C₁-C₆ alkylene-OR_{c}, -S(=O)₂R_{c} or P(=O)RₐR_{b}; the 5-10 membered heteroaryl optionally including 1, 2 or 3 heteroatoms independently selected from N, O or S; the C₁-C₁₀ alkyl, C₂-C₁₀ alkenyl, C₁-C₁₀ haloalkyl, C₃-C₁₀ cycloalkyl, C₆-C₁₀ aryl, 5-10 membered heteroaryl are each optionally substituted with one or more halogen, hydroxyl, P(=O)Me₂, P(=S)Me₂, -S(=O)₂-C₁-C₃ alkyl, - S(=O)₂-C₃-C₅ cycloalkyl, -S(=O)-C₁-C₃ alkyl, -S(=O)-C₃-C₅ cycloalkyl, CN, C₁-C₆ alkyl and/or C₁-C₆ haloalkyl;
the Rₐ, R_{b} and R_{c} are each indepentently H or C₁-C₄ alkyl.

Preferably, in some embodiments, the Rⱼ is selected from H, halogen, -CN, C₁-C₄ haloalkyl, C₃-C₅ cycloalkyl, 5-membered heteroaryl, S(=O)R_{c}, -S(=O)₂R_{c}, - S(=O)(=NRₐ)R_{b} or P(=O)Me₂; the C₃-C₅ cycloalkyl, 5-membered heteroaryl are each optionally substituted with one or more halogen, hydroxyl, C₁-C₃ alkyl, C₁-C₃ alkoxyl and/or C₁-C₃ haloalkyl; the 5-membered heteroaryl optionally including 1, 2 or 3 heteroatoms independently selected from N, O or S;
the Rₐ and R_{b} are each independently selected from H, or C₁-C₄ alkyl;
the R_{c} is selected from H, C₁-C₃ alkyl or C₁-C₃ haloalkyl.

Preferably, in some embodiments, Rⱼ is selected from halogen, CN, C₁-C₄ haloalkyl, C₃-C₆ cycloalkyl, 5-membered heteroaryl, -S(=O)R_{c}, -S(=O)₂R_{c}, -S(=O)(=NRₐ)R_{b} or P(=O)Me₂; the C₃-C₆ cycloalkyl, 5-membered heteroaryl are each optionally substituted with H, halogen, hydroxyl, C₁₋₃alkyl, C₁₋₃ alkoxyl or C₁₋₃ haloalkyl.

Preferably, in some embodiments, the Rⱼ is selected from C₁-C₄ haloalkyl, CN, - S(=O)₂R_{c} or 5-membered heteroaryl, the 5-membered heteroaryl optionally including 1, 2 or 3 heteroatoms independently selected from N, O or S; the 5-membered heteroaryl are each optionally substituted with one or more halogen, C₁-C₃ alkyl and/or C₁-C₃ haloalkyl;
the R_{c} is selected from H, C₁-C₃ alkyl or C₁-C₃ haloalkyl.

Preferably, in some embodiments, the Rⱼ is selected from C₁-C₄ haloalkyl, CN or 5-membered heteroaryl, the 5-membered heteroaryl optionally including 1, 2 or 3 heteroatoms independently selected from N, O or S; the 5-membered heteroaryl are each optionally substituted with H, halogen or C₁-C₃ alkyl.

Preferably, in some embodiments, the Rⱼ is C₁-C₄haloalkyl.

Preferably, in some embodiments, the Rⱼ is F substituted C₁-C₄ alkyl.

Preferably, in some embodiments, the Rⱼ is -CF₃, -CHF₂, -CH₂F, -CH₂CH₂F, - CH₂CHF₂ or -CH₂CF₃.

Preferably, in some embodiments, the Rⱼ is -CF₃.

Preferably, in some embodiments, the Rₐ and R_{b} are each independently selected from H, C₁-C₄ alkyl.

Preferably, in some embodiments, the R_{c} is selected from H, C₁-C₃ alkyl or C₁-C₃ haloalkyl.

Preferably, in some embodiments, the Rⱼ is selected from 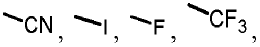 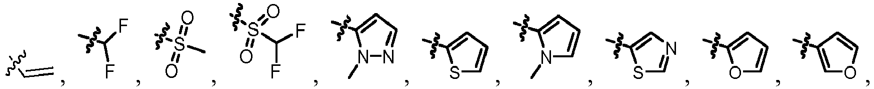 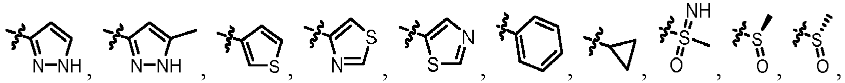 or

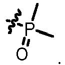

Preferably, in some embodiments, the compound is represented by formula (VII): 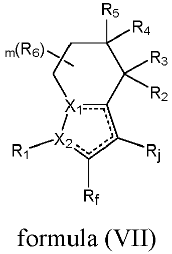 wherein,
-̅ -̅ -̅ -̅ -̅ -̅ represents a single bond or a double bond;
one and only one of X₁ and X₂ is N, and the other is C;
R₁ is selected from C₆-C₁₀ aryl, 5-18 membered heteroaryl, or C₃-C₁₀ cycloalkyl, wherein, the 5-18 membered heteroaryl and 3-10 membered heterocyclyl optionally including 1, 2 or 3 heteroatoms independently selected from N, O or S;
the R₁ is optionally substituted with one or more halogen, -OH, -CN, oxo, amino, C₁-C₆ alkyl, C₁-C₆ alkoxyl, C₂-C₆ alkenyl, C₃-C₅ cycloalkyl, C₂-C₆ alkynyl, C₁-C₆ haloalkyl, -C₁-C₆ alkylene-OR_{c}, -NO₂, -OR_{c}, -SR_{c}, -NRₐR_{b}, -C(=O)R_{c}, - C(=O)OR_{c}, -C(=O)NRₐR_{b}, -NC(=O)R_{c}, -S(=O)R_{c}, -S(=O)₂R_{c}, -S(=O)₂NRₐR_{b} or - S(=O)(=NRₐ)R_{b}, P(=O)RₐR_{b} and/or P(=S)RₐR_{b};
R₂ is -OH, -CN or halogen, and R₃ is H or deuterium; or R₂ and R₃ together form an oxo group;
R₄ and R₅ are each independently H or halogen;
R₆ is H, halogen or C₁-C₆ alkyl;
R_{f} is H, -CN, halogen, C₁-C₆ alkyl or C₁-C₃ haloalkyl;
Rⱼ is H, -CN, halogen, C₁-C₁₀ alkyl, C₂-C₁₀ alkenyl, C₁-C₁₀ haloalkyl, C₃-C₁₀ cycloalkyl, C₆-C₁₀ aryl, 5-10 membered heteroaryl, -S(=O)R_{c}, -C₁-C₆ alkylene-OR_{c}, -S(=O)₂R_{c} or P(=O)RₐR_{b}; the 5-10 membered heteroaryl optionally including 1, 2 or 3 heteroatoms independently selected from N, O or S; the C₁-C₁₀ alkyl, C₂-C₁₀ alkenyl, C₁-C₁₀ haloalkyl, C₃-C₁₀ cycloalkyl, C₆-C₁₀ aryl, 5-10 membered heteroaryl are each optionally substituted with one or more halogen, P(=O)Me₂, P(=S)Me₂, -S(=O)₂-C₁-C₃ alkyl, -S(=O)₂-C₃-C₅ cycloalkyl, -S(=O)-C₁-C₃ alkyl, -S(=O)-C₃-C₅ cycloalkyl, CN, C₁-C₆ alkyl and/or C₁-C₆ haloalkyl;
the Rₐ and R_{b} are each independently selected from H or C₁-C₄ alkyl;
the R_{c} is selected from H, C₁-C₃ alkyl or C₁-C₃ haloalkyl.

The present invention further provides a compound or solvate thereof, the compound is selected from:
1-(3-chloro-5-fluorophenyl)-5,5-difluoro-3-iodo-1,5,6,7-tetrahydro-4*H*-indol-4-one;
1-(3-chloro-5-fluorophenyl)-5,5-difluoro-3-iodo-4,5,6,7-tetrahydro-1*H*-indol-4-ol;
5,5-difluoro-3-iodo-1-(4-(trifluoromethyl)phenyl)-1,5,6,7-tetrahydro-4*H*-indol-4-one;
5,5-difluoro-3-iodo-1-(4-(trifluoromethyl)phenyl)-4,5,6,7-tetrahydro-1*H*-indol-4-ol;
5-(5,5-difluoro-4-hydroxy-3-iodo-4,5,6,7-tetrahydro-1H-indol-1-yl)-2-fluorobenzonitrile;
1-(3-chloro-5-fluorophenyl)-5,5-difluoro-3-(trifluoromethyl)-1,5,6,7-tetrahydro-4*H*-indol-4-one;
1-(3-chloro-5-fluorophenyl)-5,5-difluoro-3-(trifluoromethyl)-4,5,6,7-tetrahydro-1*H*-indol-4-ol;
1-(3,5-difluorophenyl)-5,5-difluoro-3-(trifluoromethyl)-4,5,6,7-tetrahydro-1*H-*indol-4-ol;
5,5-difluoro-1-phenyl-3-(trifluoromethyl)-4,5,6,7-tetrahydro-1*H*-indol-4-ol;
1-(3,5-difluorophenyl)-5,5-difluoro-3-(trifluoromethyl)-1,5,6,7-tetrahydro-4*H-*indol-4-one;
5,5-difluoro-1-phenyl-3-(trifluoromethyl)-1,5,6,7-tetrahydro-4*H*-indol-4-one;
3-(5,5-difluoro-4-hydroxy-3-(trifluoromethyl)-4,5,6,7-tetrahydro-1*H*-indol-1-yl)-5-fluorobenzonitrile;
4-(5,5-difluoro-4-hydroxy-3-(trifluoromethyl)-4,5,6,7-tetrahydro-1*H*-indol-1-yl)benzonitrile;
5,5-difluoro-1-(4-fluoro-3-methoxyphenyl)-3-(trifluoromethyl)-4,5,6,7-tetrahydro-1H-indol-4-ol;
1-(3-chlorophenyl)-5,5-difluoro-3-(trifluoromethyl)-4,5,6,7-tetrahydro-1*H*-indol-4-ol;
5,5-difluoro-1-(3-fluorophenyl)-3-(trifluoromethyl)-4,5,6,7-tetrahydro-1*H*-indol-4-ol;
1-(5-chloropyridin-3-yl)-5,5-difluoro-3-(trifluoromethyl)-4,5,6,7-tetrahydro-1*H-*indol-4-ol;
5,5-difluoro-1-(2-fluorophenyl)-3-(trifluoromethyl)-4,5,6,7-tetrahydro-1*H*-indol-4-ol;
3-(5,5-difluoro-4-hydroxy-3-(trifluoromethyl)-4,5,6,7-tetrahydro-1*H*-indol-1-yl)benzonitrile;
1-(3,5-dichlorophenyl)-5,5-difluoro-3-(trifluoromethyl)-4,5,6,7-tetrahydro-1*H-*indol-4-ol;
1-(4-chlorophenyl)-5,5-difluoro-3-(trifluoromethyl)-4,5,6,7-tetrahydro-1*H*-indol-4-ol;
1-(3-bromo-5-fluorophenyl)-5,5-difluoro-3-(trifluoromethyl)-4,5,6,7-tetrahydro-1*H*-indol-4-ol;
5,5-difluoro-1-(pyridin-3-yl)-3-(trifluoromethyl)-4,5,6,7-tetrahydro-1*H*-indol-4-ol;
5,5-difluoro-1-(3-fluoro-5-(trifluoromethyl)phenyl)-3-(trifluoromethyl)-4,5,6,7-tetrahydro-1*H*-indol-4-ol;
5,5-difluoro-3-(trifluoromethyl)-1-(4-(trifluoromethyl)phenyl)-4,5,6,7-tetrahydro-1*H*-indol-4-ol;
5,5-difluoro-1-(p-tolyl)-3-(trifluoromethyl)-4,5,6,7-tetrahydro-1*H*-indol-4-ol;
5,5-difluoro-1-(3-fluoro-5-hydroxyphenyl)-3-(trifluoromethyl)-4,5,6,7-tetrahydro-1*H*-indol-4-ol;
1-(3-chloro-5-fluorobenzyl)-5,5-difluoro-3-(trifluoromethyl)-1,5,6,7-tetrahydro-4*H*-indol-4-one;
1-(3-chloro-5-fluorobenzyl)-5,5-difluoro-3-(trifluoromethyl)-4,5,6,7-tetrahydro-1*H*-indol-4-ol;
5,5-difluoro-1-phenyl-3-(trifluoromethyl)-1,5,6,7-tetrahydro-4*H*-indol-4-ol;
1-((3,3-difluorocyclobutyl)methyl)-5,5-difluoro-3-(trifluoromethyl)-4,5,6,7-tetrahydro-1*H*-indol-4-ol;
(5,5-difluoro-4-hydroxy-3-(trifluoromethyl)-4,5,6,7-tetrahydro-1*H*-indol-1-yl)(3,3-difluorocyclobutyl)methanone;
1-(3,5-difluorophenyl)-5,5-difluoro-1,5,6,7-tetrahydro-4*H*-indol-4-one;
1-(3,5-difluorophenyl)-5,5-difluoro-4,5,6,7-tetrahydro-1*H*-indol-4-ol;
1-cyclohexyl-5,5-difluoro-3-(trifluoromethyl)-1,5,6,7-tetrahydro-4*H*-indol-4-one;
1-cyclohexyl-5,5-difluoro-3-(trifluoromethyl)-4,5,6,7-tetrahydro-1*H*-indol-4-ol;
1-(3,5-difluorophenyl)-3-(trifluoromethyl)-4,5,6,7-tetrahydro-1*H*-indol-4-ol;
3-fluoro-5-(3-(methylsulfonyl)-4-oxo-4,5,6,7-tetrahydro-1*H*-indol-1-yl)benzonitrile;
3-fluoro-5-(4-hydroxy-3-(methylsulfonyl)-4,5,6,7-tetrahydro-1*H*-indol-1-yl)benzonitrile;
1-(3,5-difluorophenyl)-4-hydroxy-4,5,6,7-tetrahydro-1*H*-indole-3-carbonitrile;
3-fluoro-5-(4-hydroxy-3-(trifluoromethyl)-4,5,6,7-tetrahydro-1*H*-indol-1-yl)benzonitrile;
1-(3-chloro-5-fluorophenyl)-3-(trifluoromethyl)-1,5,6,7-tetrahydro-4*H*-indol-4-one;
3-fluoro-5-(4-oxo-3-(trifluoromethyl)-4,5,6,7-tetrahydro-1*H*-indol-1-yl)benzonitrile;
1-(3,3-difluorocyclobutyl)-5,5-difluoro-3-(trifluoromethyl)-4,5,6,7-tetrahydro-1*H-*indol-4-ol;
1-(3,3-difluorocyclobutyl)-5,5-difluoro-3-(methylsulfonyl)-4,5,6,7-tetrahydro-1*H-*indol-4-ol;
2-fluoro-5-(4-hydroxy-3-(trifluoromethyl)-4,5,6,7-tetrahydro-1*H*-indol-1-yl)benzonitrile;
1-(3,5-difluorophenyl)-3-(1-methyl-1H-pyrazol-5-yl)-4,5,6,7-tetrahydro-1*H*-indol-4-ol;
(*S*)-1-(3-chloro-5-fluorophenyl)-5,5-difluoro-3-(thiophen-2-yl)-4,5,6,7-tetrahydro-1*H*-indol-4-ol;
1-(4-fluorobenzyl)-3-(1-methyl-1H-pyrazol-5-yl)-4,5,6,7-tetrahydro-1*H*-indol-4-ol;
1-(3,5-difluorophenyl)-5,5-difluoro-3-(thiophen-2-yl)-4,5,6,7-tetrahydro-1*H*-indol-4-ol;
1-(3-chloro-5-fluorophenyl)-5,5-difluoro-3-(1-methyl-1*H*-pyrrol-2-yl)-4,5,6,7-tetrahydro-1*H*-indol-4-ol;
1-(3-chloro-5-fluorophenyl)-3-cyclopropyl-5,5-difluoro-4,5,6,7-tetrahydro-1*H-*indol-4-ol;
1-(3-chloro-5-fluorophenyl)-5,5-difluoro-3-(furan-2-yl)-4,5,6,7-tetrahydro-1*H-*indol-4-ol;
5-(5,5-difluoro-4-hydroxy-3-(thiazol-5-yl)-4,5,6,7-tetrahydro-1*H*-indol-1-yl)-2-fluorobenzonitrile;
1-(3-chloro-5-fluorophenyl)-5,5-difluoro-3-(furan-3-yl)-1,5,6,7-tetrahydro-4*H-*indol-4-one;
1-(3-chloro-5-fluorophenyl)-5,5-difluoro-3-(furan-3-yl)-4,5,6,7-tetrahydro-1*H-*indol-4-ol;
1-(3-chloro-5-fluorophenyl)-5,5-difluoro-3-(1*H*-pyrazol-3-yl)-4,5,6,7-tetrahydro-1H-indol-4-ol;
1-(3-chloro-5-fluorophenyl)-5,5-difluoro-3-(5-methyl-1*H*-pyrazol-3-yl)-4,5,6,7-tetrahydro-1H-indol-4-ol;
1-(3-chloro-5-fluorophenyl)-5,5-difluoro-3-(1-methyl-1*H*-pyrazol-5-yl)-4,5,6,7-tetrahydro-1H-indol-4-ol;
1-(3-chloro-5-fluorophenyl)-5,5-difluoro-3-(thiophen-3-yl)-4,5,6,7-tetrahydro-1*H-*indol-4-ol;
1-(3-chloro-5-fluorophenyl)-5,5-difluoro-3-methyl-4,5,6,7-tetrahydro-1*H*-indol-4-ol;
1-(3-chloro-5-fluorophenyl)-5,5-difluoro-3-(thiazol-4-yl)-4,5,6,7-tetrahydro-1*H-*indol-4-ol;
1-(3-chloro-5-fluorophenyl)-5,5-difluoro-3-(thiazol-5-yl)-4,5,6,7-tetrahydro-1*H-*indol-4-ol;
(*S*)-1-(3,5-difluorophenyl)-5,5-difluoro-3-(trifluoromethyl)-4,5,6,7-tetrahydro-1*H-*indol-4-ol;
1-(3-chloro-5-fluorophenyl)-3-(difluoromethyl)-5,5-difluoro-4,5,6,7-tetrahydro-1*H*-indol-4-ol;
1-(3-chloro-5-fluorophenyl)-5,5-difluoro-3-vinyl-4,5,6,7-tetrahydro-1*H*-indol-4-ol;
1-(3-chloro-5-fluorophenyl)-5,5-difluoro-3-(hydroxymethyl)-4,5,6,7-tetrahydro-1*H*-indol-4-ol;
3-(3-chloro-5-fluorophenyl)-7-fluoro-1-(trifluoromethyl)-5,6,7,8-tetrahydroindolizin-8-ol;
5-(7,7-difluoro-8-hydroxy-1-(trifluoromethyl)-5,6,7,8-tetrahydroindolizin-3-yl)-2-fluorobenzonitrile;
(*S*)-5-(7,7-difluoro-8-hydroxy-1-(trifluoromethyl)-5,6,7,8-tetrahydroindolizin-3-yl)-2-fluorobenzonitrile;
3-(3-chloro-5-fluorophenyl)-7,7-difluoro-1-(trifluoromethyl)-5,6,7,8-tetrahydroindolizin-8-ol;
7-fluoro-3-phenyl-1-(trifluoromethyl)-5,6,7,8-tetrahydroindolizin-8-ol;
3-(3,5-difluorophenyl)-7-fluoro-1-(trifluoromethyl)-5,6,7,8-tetrahydroindolizin-8-ol;
7-fluoro-3-(4-fluoro-3-(trifluoromethyl)phenyl)-1-(trifluoromethyl)-5,6,7,8-tetrahydroindolizin-8-ol;
2-fluoro-5-(7-fluoro-8-hydroxy-1-(trifluoromethyl)-5, 6,7, 8-tetrahydroindolizin-3-yl)benzonitrile;
3-(3,5-difluorophenyl)-7,7-difluoro-1-(trifluoromethyl)-5,6,7,8-tetrahydroindolizin-8-ol;
3-(7,7-difluoro-8-hydroxy-1-(trifluoromethyl)-5,6,7,8-tetrahydroindolizin-3-yl)-5-fluorobenzonitrile;
(*E*)-3-(2-cyclohexylvinyl)-7,7-difluoro-1-(trifluoromethyl)-5,6,7,8-tetrahydroindolizin-8-ol;
3-(cyclopropylethynyl)-7,7-difluoro-1-(trifluoromethyl)-5,6,7,8-tetrahydroindolizin-8-ol;
3-(3-(difluoromethyl)-4-fluorophenyl)-7,7-difluoro-1-(trifluoromethyl)-5,6,7,8-tetrahydroindolizin-8-ol;
7,7-difluoro-1-(trifluoromethyl)-3-(3,4, 5-trifluorophenyl)-5, 6,7, 8-tetrahydroindolizin-8-ol;
7,7-difluoro-3-(4-fluoro-3-(trifluoromethyl)phenyl)-1-(trifluoromethyl)-5,6,7,8-tetrahydroindolizin-8-ol;
3-(3-(difluoromethyl)-4-fluorophenyl)-7,7-difluoro-1-(trifluoromethyl)-6,7-dihydroindolizin-8(5*H*)-one;
7,7-difluoro-1-(trifluoromethyl)-3-(3,4,5-trifluorophenyl)-6,7-dihydroindolizin-8(5*H*)-one;
7,7-difluoro-3-(4-fluoro-3-(trifluoromethyl)phenyl)-1-(trifluoromethyl)-6,7-dihydroindolizin-8(5*H*)-one;
7,7-difluoro-3-(1-phenylcyclopropyl)-1-(trifluoromethyl)-5,6,7,8-tetrahydroindolizin-8-ol;
7,7-difluoro-3-(phenylethynyl)-1-(trifluoromethyl)-5,6,7,8-tetrahydroindolizin-8-ol;
(1-(3-chloro-5-fluorophenyl)-5,5-difluoro-4-hydroxy-4,5,6,7-tetrahydro-1*H*-indol-3-yl)dimethylphosphine oxide;
(*S*)-5-(5,5-difluoro-4-hydroxy-3-(trifluoromethyl)-4,5,6,7-tetrahydro-1*H*-indol-1-yl)-2-fluorobenzonitrile;
1-(3-chloro-5-fluorophenyl)-5,5-difluoro-4-hydroxy-4,5,6,7-tetrahydro-1*H*-indole-3-carbonitrile;
1-(3-chloro-5-fluorophenyl)-5,6-difluoro-3-(trifluoromethyl)-4,5,6,7-tetrahydro-1*H*-indol-4-ol;
5,5-difluoro-1-(4-fluoro-3-(hydroxymethyl)phenyl)-3-(trifluoromethyl)-4,5,6,7-tetrahydro-1*H*-indol-4-ol;
5,5-difluoro-1-(6-fluoropyridin-2-yl)-3-(trifluoromethyl)-4,5,6,7-tetrahydro-1*H-*indol-4-ol;
5-(5,5-difluoro-4-hydroxy-4-methyl-3-(trifluoromethyl)-4,5,6,7-tetrahydro-1*H-*indol-1-yl)-2-fluorobenzonitrile;
5-(5,5-difluoro-4-hydroxy-6,6-dimethyl-3-(trifluoromethyl)-4,5,6,7-tetrahydro-1*H-*indol-1-yl)-2-fluorobenzonitrile;
2-fluoro-5-(5-fluoro-4-hydroxy-6,6-dimethyl-3-(trifluoromethyl)-4,5,6,7-tetrahydro-1*H*-indol-1-yl)benzonitrile;
(4*S*,5*S*)-1-(3-(difluoromethyl)-4-fluorophenyl)-5-fluoro-3-(trifluoromethyl)-4,5,6,7-tetrahydro-1*H*-indol-4-ol;
(4*R*,5*R*)-1-(3-(difluoromethyl)-4-fluorophenyl)-5-fluoro-3-(trifluoromethyl)-4,5,6,7-tetrahydro-1*H*-indol-4-ol;
(4*S*,5*R*)-1-(3-(difluoromethyl)-4-fluorophenyl)-5-fluoro-3-(trifluoromethyl)-4,5,6,7-tetrahydro-1*H*-indol-4-ol;
(4*R*,5*S*)-1-(3-(difluoromethyl)-4-fluorophenyl)-5-fluoro-3-(trifluoromethyl)-4,5,6,7-tetrahydro-1*H*-indol-4-ol;
1-(3-chloro-5-fluorophenyl)-2,5,5-trifluoro-3-(trifluoromethyl)-4,5,6,7-tetrahydro-1*H*-indol-4-ol;
(*S*)-2-chloro-1-(3-chloro-5-fluorophenyl)-5,5-difluoro-3-(trifluoromethyl)-4,5,6,7-tetrahydro-1*H*-indol-4-ol;
1-(3-(difluoromethyl)-4-fluorophenyl)-3-(trifluoromethyl)-4,5,6,7-tetrahydro-1*H-*indol-4-ol;
1-(3-chloro-5-fluorophenyl)-5,5-difluoro-3-(methylsulfonyl)-4,5,6,7-tetrahydro-1*H*-indol-4-ol;
3-(5,5-difluoro-4-hydroxy-3-(methylsulfonyl)-4,5,6,7-tetrahydro-1*H*-indol-1-yl)-5-fluorobenzonitrile;
3-(3-(difluoromethyl)-5,5-difluoro-4-hydroxy-4,5,6,7-tetrahydro-1*H*-indol-1-yl)-5-fluorobenzonitrile;
3-(3-((difluoromethyl)sulfonyl)-5,5-difluoro-4-hydroxy-4,5,6,7-tetrahydro-1*H-*indol-1-yl)-5-fluorobenzonitrile;
1-(3-chloro-5-fluorophenyl)-3,5,5-trifluoro-4,5,6,7-tetrahydro-1*H*-indol-4-ol;
1-(3-chloro-5-fluorophenyl)-5,5-difluoro-4-hydroxy-3-(trifluoromethyl)-4,5,6,7-tetrahydro-1*H*-indole-2-carbonitrile;
(*R*)-1-(3-chloro-5-fluorophenyl)-5,5-difluoro-3-(trifluoromethyl)-4,5,6,7-tetrahydro-1*H*-indol-4-ol;
(*S*)-1-(3-chloro-5-fluorophenyl)-5,5-difluoro-3-(trifluoromethyl)-4,5,6,7-tetrahydro-1*H*-indol-4-ol;
1-(3,5-difluorophenyl)-5,5-difluoro-3-(methylsulfonyl)-4,5,6,7-tetrahydro-1*H-*indol-4-ol;
1-(3,5-difluorophenyl)-5,5-difluoro-3-(methylsulfonyl)-1,5,6,7-tetrahydro-4*H-*indol-4-one;
3-(3-chloro-5-fluorophenyl)-6,6-difluoro-1-(trifluoromethyl)-4,5,6,7-tetrahydro-1*H*-indol-7-ol;
1-(3-chloro-4-fluorophenyl)-5,5-difluoro-3-(trifluoromethyl)-4,5,6,7-tetrahydro-1*H*-indol-4-ol;
5-(5,5-difluoro-4-hydroxy-3-(trifluoromethyl)-4,5,6,7-tetrahydro-1*H*-indol-1-yl)-2-fluorobenzonitrile;
4-(5,5-difluoro-4-hydroxy-3-(trifluoromethyl)-4,5,6,7-tetrahydro-1*H*-indol-1-yl)-2-fluorobenzonitrile;
5,5-difluoro-1-(1-methyl-1*H*-pyrrol-2-yl)-3-(trifluoromethyl)-4,5,6,7-tetrahydro-1*H*-indol-4-ol;
2-chloro-4-(5,5-difluoro-4-hydroxy-3-(trifluoromethyl)-4,5,6,7-tetrahydro-1*H-*indol-1-yl)benzonitrile;
4-(5,5-difluoro-4-hydroxy-3-(trifluoromethyl)-4,5,6,7-tetrahydro-1*H*-indol-1-yl)phthalonitrile;
5,5-difluoro-1-(furan-2-yl)-3-(trifluoromethyl)-4,5,6,7-tetrahydro-1*H*-indol-4-ol;
2-cyano-5-(5,5-difluoro-4-hydroxy-3-(trifluoromethyl)-4,5,6,7-tetrahydro-1*H-*indol-1-yl)benzoic acid;
2-acetyl-4-(5,5-difluoro-4-hydroxy-3-(trifluoromethyl)-4,5,6,7-tetrahydro-1*H-*indol-1-yl)benzonitrile;
1-(3-chloro-5-fluorophenyl)-5,6-difluoro-3-(methylsulfonyl)-4,5,6,7-tetrahydro-1*H*-indol-4-ol;
1-(4-chlorophenyl)-5,6-difluoro-3-(trifluoromethyl)-4,5,6,7-tetrahydro-1*H*-indol-4-ol;
2-fluoro-5-((4S)-5-fluoro-4-hydroxy-3-(trifluoromethyl)-4,5,6,7-tetrahydro-1*H*- indol-1-yl)benzonitrile;
5,6-difluoro-1-(5-fluoropyridin-3-yl)-3-(trifluoromethyl)-4,5,6,7-tetrahydro-1*H-*indol-4-ol;
5,5-difluoro-1-(5-fluoropyridin-3-yl)-3-(trifluoromethyl)-4,5,6,7-tetrahydro-1*H-*indol-4-ol;
(*S*)-5-(5,5-difluoro-4-hydroxy-3-(methylsulfonyl)-4,5,6,7-tetrahydro-1*H*-indol-1-yl)-2-fluorobenzonitrile;
1-(3-chloro-5-fluorophenyl)-5,5-difluoro-3-((S)-methylsulfinyl)-4,5,6,7-tetrahydro-1*H*-indol-4-ol;
1-(3-chloro-5-fluorophenyl)-5,5-difluoro-3-((R)-methylsulfinyl)-4,5,6,7-tetrahydro-1*H*-indol-4-ol;
2-chloro-5-(5,5-difluoro-4-hydroxy-3-(trifluoromethyl)-4,5,6,7-tetrahydro-1*H-*indol-1-yl)benzonitrile;
1-(4-chloro-3-nitrophenyl)-5,5-difluoro-3-(trifluoromethyl)-4,5,6,7-tetrahydro-1*H-*indol-4-ol;
3-fluoro-5-(5-fluoro-4-hydroxy-3-(trifluoromethyl)-4,5,6,7-tetrahydro-1*H*-indol-1-yl)benzonitrile;
1-(3-amino-5-fluorophenyl)-5-fluoro-3-(trifluoromethyl)-4,5,6,7-tetrahydro-1*H-*indol-4-ol;
1-(3-chloro-5-fluorophenyl)-5,7-difluoro-3-(trifluoromethyl)-4,5,6,7-tetrahydro-1*H*-indol-4-ol;
1-(3-chloro-5-fluorophenyl)-5-fluoro-3-(trifluoromethyl)-4,5,6,7-tetrahydro-1*H-*indol-4-ol;
1-(3-chloro-5-fluorophenyl)-5-fluoro-2-methyl-3-(trifluoromethyl)-4,5,6,7-tetrahydro-1*H*-indol-4-ol;
1-(3-chloro-5-fluorophenyl)-5,5-difluoro-3-phenyl-1,5,6,7-tetrahydro-4*H*-indol-4-one;
1-(3-chloro-5-fluorophenyl)-5,5-difluoro-3-phenyl-4,5,6,7-tetrahydro-1*H*-indol-4-ol;
(*S*)-2-bromo-1-(3,5-difluorophenyl)-5,5-difluoro-3-(trifluoromethyl)-4,5,6,7-tetrahydro-1*H*-indol-4-ol;
5,5-difluoro-1-(thiophen-3-yl)-3-(trifluoromethyl)-4,5,6,7-tetrahydro-1*H*-indol-4-ol;
5,5-difluoro-3-(trifluoromethyl)-1-(3,4,5-trifluorophenyl)-4,5,6,7-tetrahydro-1*H-*indol-4-ol;
1-(3,5-dichloro-4-fluorophenyl)-5,5-difluoro-3-(trifluoromethyl)-4,5,6,7-tetrahydro-1*H*-indol-4-ol;
3-(3-chloro-5-fluorophenyl)-6,7-difluoro-1-(trifluoromethyl)-5,6,7,8-tetrahydroindolizin-8-ol;
3-(3-chloro-5-fluorophenyl)-6,7-difluoro-1-(trifluoromethyl)-5,6,7,8-tetrahydroimidazo[1,5-a]pyridin-8-ol;
3-(3-chloro-5-fluorophenyl)-5,6-difluoro-1-(trifluoromethyl)-4,5,6,7-tetrahydro-1*H*-indol-7-ol;
3-(3-chloro-5-fluorophenyl)-5,6-difluoro-1-(trifluoromethyl)-4,5,6,7-tetrahydro-1*H*-indazol-7-ol;
3-chloro-5-(5,5-difluoro-4-hydroxy-3-(trifluoromethyl)-4,5,6,7-tetrahydro-1*H-*indol-1-yl)benzonitrile;
4-(5,5-difluoro-4-hydroxy-3-(trifluoromethyl)-4,5,6,7-tetrahydro-1*H*-indol-1-yl)-2-methoxybenzonitrile;
5,5-difluoro-1-(4-fluoro-3-(trifluoromethyl)phenyl)-3-(trifluoromethyl)-4,5,6,7-tetrahydro-1*H*-indol-4-ol;
4-(5,5-difluoro-4-hydroxy-3-(trifluoromethyl)-4,5,6,7-tetrahydro-1*H*-indol-1-yl)-2-(trifluoromethyl)benzonitrile;
5,5-difluoro-1-(3-fluoro-4-methoxyphenyl)-3-(trifluoromethyl)-4,5,6,7-tetrahydro-1*H*-indol-4-ol;
5,5-difluoro-1-(pyridin-4-yl)-3-(trifluoromethyl)-4,5,6,7-tetrahydro-1*H*-indol-4-ol;
5,5-difluoro-1-(4-fluorophenyl)-3-(trifluoromethyl)-4,5,6,7-tetrahydro-1*H*-indol-4-ol;
5,5-difluoro-1-(3-(methylsulfonyl)phenyl)-3-(trifluoromethyl)-4,5,6,7-tetrahydro-1*H*-indol-4-ol;
5,5-difluoro-1-(2-methoxypyridin-4-yl)-3-(trifluoromethyl)-4,5,6,7-tetrahydro-1*H-*indol-4-ol;
4-(5,5-difluoro-4-hydroxy-3-(trifluoromethyl)-4,5,6,7-tetrahydro-1*H*-indol-1-yl)picolinonitrile;
1-(3,4-difluorophenyl)-5,5-difluoro-3-(trifluoromethyl)-4,5,6,7-tetrahydro-1*H-*indol-4-ol;
5,5-difluoro-1-(2-fluoropyridin-4-yl)-3-(trifluoromethyl)-4,5,6,7-tetrahydro-1*H-*indol-4-ol;
1-(3-cyano-4-fluorophenyl)-5,5-difluoro-4-hydroxy-4,5,6,7-tetrahydro-1*H*-indole-3-carbonitrile;
(*S*)-4-(5,5-difluoro-4-hydroxy-3-(trifluoromethyl)-4,5,6,7-tetrahydro-1*H*-indol-1-yl)phthalonitrile;
1-(3-(difluoromethyl)-4-fluorophenyl)-5,5-difluoro-3-(trifluoromethyl)-4,5,6,7-tetrahydro-1*H*-indol-4-ol;
5,5-difluoro-1-(4-fluoro-3-methylphenyl)-3-(trifluoromethyl)-4,5,6,7-tetrahydro-1*H*-indol-4-ol;
5,5-difluoro-1-(6-fluoropyridin-3-yl)-3-(trifluoromethyl)-4,5,6,7-tetrahydro-1*H-*indol-4-ol;
5,5-difluoro-1-(5-fluoropyridin-2-yl)-3-(trifluoromethyl)-4,5,6,7-tetrahydro-1*H-*indol-4-ol;
1-(2-chloropyridin-4-yl)-5,5-difluoro-3-(trifluoromethyl)-4,5,6,7-tetrahydro-1*H-*indol-4-ol;
(*S*)-1-(3-(difluoromethyl)-4-fluorophenyl)-5,5-difluoro-3-(trifluoromethyl)-4,5,6,7-tetrahydro-1*H*-indol-4-ol;
(*R*)-1-(3-(difluoromethyl)-4-fluorophenyl)-5,5-difluoro-3-(trifluoromethyl)-4,5,6,7-tetrahydro-1*H*-indol-4-ol;
(*R*)-5-(5,5-difluoro-4-hydroxy-3-(trifluoromethyl)-4,5,6,7-tetrahydro-1*H*-indol-1-yl)-2-fluorobenzonitrile;
5-(5,5-difluoro-4-hydroxy-3-(trifluoromethyl)-4-vinyl-4,5,6,7-tetrahydro-1*H*-indol-1-yl)-2-fluorobenzonitrile;
5,5-difluoro-1-(4-fluorophenyl)-3-(trifluoromethyl)-4,5,6,7-tetrahydro-1*H*-indole-4-carbonitrile;
(*S*)-5-(5,5-difluoro-4-hydroxy-3-(trifluoromethyl)-4,5,6,7-tetrahydro-1*H*-indol-1-yl)nicotinonitrile;
5-(5,5-difluoro-4-hydroxy-6-methyl-3-(trifluoromethyl)-4,5,6,7-tetrahydro-1*H-*indol-1-yl)-2-fluorobenzonitrile;
5-((4*S*)-5,5-difluoro-4-hydroxy-6-methyl-3-(trifluoromethyl)-4,5,6,7-tetrahydro-1*H*-indol-1-yl)-2-fluorobenzonitrile;
(*E*)-5,5-difluoro-1-styryl-3-(trifluoromethyl)-4,5,6,7-tetrahydro-1*H*-indol-4-ol;
(*E*)-5,5-difluoro-1-styryl-3-(trifluoromethyl)-1,5,6,7-tetrahydro-4*H*-indol-4-one;
1-(phenylsulfonyl)-3-(trifluoromethyl)-4,5,6,7-tetrahydro-1*H*-indol-4-ol;
(*E*)-1-(2-cyclohexylvinyl)-5,5-difluoro-3-(trifluoromethyl)-1,5,6,7-tetrahydro-4*H-*indol-4-one;
5,5-difluoro-1-(phenylsulfonyl)-3-(trifluoromethyl)-1,5,6,7-tetrahydro-4*H*-indol-4-one;
5,5-difluoro-1-(phenylsulfonyl)-3-(trifluoromethyl)-4,5,6,7-tetrahydro-1*H*-indol-4-ol;
(*E*)-1-(2-cyclohexylvinyl)-5,5-difluoro-3-(trifluoromethyl)-4,5,6,7-tetrahydro-1*H-*indol-4-ol;
5-(5,5-difluoro-4-oxo-3-(trifluoromethyl)-4,5,6,7-tetrahydro-1*H*-indol-1-yl)-2-fluorobenzonitrile;
5-(5,5-difluoro-4-oxo-3-(trifluoromethyl)-4,5,6,7-tetrahydro-1*H*-indol-1-yl)-2-fluorobenzaldehyde;
1-(3-(difluoromethyl)-4-fluorophenyl)-5,5-difluoro-3-(trifluoromethyl)-1,5,6,7-tetrahydro-4H-indol-4-one;
2-fluoro-5-(5-fluoro-4-oxo-3-(trifluoromethyl)-4,5,6,7-tetrahydro-1*H*-indol-1-yl)benzaldehyde;
1-(3-(difluoromethyl)-4-fluorophenyl)-5-fluoro-3-(trifluoromethyl)-1,5,6,7-tetrahydro-4*H*-indol-4-one;
3-fluoro-5-(5-fluoro-4-hydroxy-3-(methylsulfonyl)-4,5,6,7-tetrahydro-1*H*-indol-1-yl)benzonitrile;
3-(3-chloro-5-fluorophenyl)-7,7-difluoro-1-(trifluoromethyl)-5,6,7,8-tetrahydroimidazo[1,5-a]pyridin-8-ol;
3-(3-chloro-5-fluorophenyl)-6,6-difluoro-1-(trifluoromethyl)-4,5,6,7-tetrahydro-1*H*-indazol-7-ol;
(*S*)-1-(3,5-difluorophenyl)-5,5-difluoro-3-(methylsulfonyl)-2-(trifluoromethyl)-4,5,6,7-tetrahydro-1*H*-indol-4-ol;
(*S*)-2-fluoro-5-(4,5,5-trifluoro-3-(trifluoromethyl)-4,5,6,7-tetrahydro-1*H*-indol-1-yl)benzonitrile;
(*R*)-2-fluoro-5-(4,5,5-trifluoro-3-(trifluoromethyl)-4,5,6,7-tetrahydro-1*H*-indol-1-yl)benzonitrile;
(*Z*)-5-(5,5-difluoro-4-(hydroxyimino)-3-(trifluoromethyl)-4,5,6,7-tetrahydro-1*H-*indol-1-yl)-2-fluorobenzonitrile; or
(*S*)-5-(5,5-difluoro-4-hydroxy-3-(trifluoromethyl)-4,5,6,7-tetrahydro-1*H*-indol-1-yl-4-d)-2-fluorobenzonitrile.

The present inention also provides the preparation method of the compound, both of the compound of the formula (I) or the compound of the specific example can be prepared by the known organic synthesis technology, for example, it is obtained by a preparation method similar to that in the specific example.

The present invention also provides a pharmaceutical composition including a therapeutically effective amount of at least one of the above compounds and a pharmaceutically acceptable excipient, such as hydroxypropyl methyl cellulose. In some compositions, the weight ratio of compound to excipient is about 0.001~10.

The present invention additionally provided a method of treating a subject suffering from a disease or disorder mediated by HIF-2α, including administering a therapeutically effective amount of a compound of formula (I) or a pharmaceutically acceptable salt thereof.

In some respects, the disease or disorder is selected from VHL syndrome, autoimmune diseases, inflammatory diseases, neurodegenerative diseases, cardiovascular disorders, renal disorders, viral infections or obesity. In some respects, the disease or disorder is selected from rheumatoid arthritis, osteoarthritis, atherosclerosis, psoriasis, systemic lupus erythematosus, multiple sclerosis, inflammatory bowel disease, asthma, chronic obstructive airway disease, pneumonia, dermatitis, alopecia, nephritis, vasculitis, atherosclerosis, alzheimer's haimer's disease, hepatitis, primary biliary cirrhosis, sclerosing cholangitis, diabetes (including type I diabetes), acute rejection of transplanted organs. In some aspects, the disease or disorder is cancer, including hematology cancer, lymphoma, multiple myeloma, digestive system tumor, reproductive system tumor, brain tumor, nervous system tumor neoplasm.

In some respects, The disease or disorder is glioma, pheochromocytoma, paraganglioma, colon, rectum, prostate (eg castrate resistant prostate cancer), lung cancer (eg non-small cell lung cancer and/or small cell lung cancer), pancreas, liver, kidney, cervix, uterus, stomach, ovary, breast (eg basal or basal-like breast cancer and/or triple negative breast cancer), skin (eg melanoma), nervous system (including brain, meninges, and central nervous system, including neuroblastoma, glioblastoma, meningioma and medulloblastoma) tumors or cancers.

In some respects, the disease or disorder is VHL syndrome. In some respects, the disease or disorder is kidney cancer. In some respects, the subject is human.

In some respects, the compound is administered intravenously, intramuscularly, parenterally, nasally or orally. In some aspect, the compound is administered orally.

The present invention also provides the use of the compound of formula (I) or a pharmaceutically acceptable salt thereof in the preparation of a medicament for treating a disease or disorder mediated by HIF-2α.

The present invention also provides the compound of formula (I) or a pharmaceutically acceptable salt thereof for treatment. Further provides the compound of formula (I) or a pharmaceutically acceptable salt thereof for treating a subject suffering from a disease or disorder mediated by HIF-2α.

The present invention can be embodied in other specific forms without departing from its spirit or essential attributes. The present invention encompasses all combinations of the preferred aspects of the invention mentioned herein. It is to be understood that the subject matter disclosed in the "summary of the invention" is not an exhaustive or complete disclosure of the full scope of the present technology or any of its embodiments thereof, and that any and all embodiments of the present invention may be employed in conjunction with any other one or more embodiments to describe additional embodiments. It should also be understood that each individual element of an embodiment is its own independent embodiment. Furthermore, any element of an embodiment is intended to describe additional embodiments in combination with any and all other elements from any embodiment.

In the present invention, unless otherwise indicated, the term "halogen", means fluoro, chloro, bromo or iodo. The preferred halogen group includes F, Cl or Br.

In the present invention, unless otherwise indicated, the term "alkyl" includes straight or branched chain which saturated monovalent hydrocarbon radicals. For example, alkyl radicals include methyl, ethyl, propyl, isopropyl, cyclcopropyl, n-butyl, isobutyl, sec-butyl, t-butyl, cyclcobutyl, n-pentyl, 3- (2-methyl) butyl, 2-pentyl, 2-methylbutyl, neopentyl, cyclcopentyl, n- hexyl, 2-hexyl, 2-methylpentyl or cyclohexyl. Similary, "C₁-C₆", as in C₁₋₆ alkyl is defined to identify a group containing 1, 2, 3, 4, 5 or 6 carbon atoms arranged in a straight or branched chain.

The term "alkoxyl" refers to the oxygen ethers formed from the previously described straight chain, branched chain or cyclic alkyl groups.

The term "alkylene" refers to a divalent alkyl linking group. Alkylene formally refers to an alkane which two C-H bonds are replaced by the point of attachment to the rest of the compound. Similarly, "C₁-C₄" in C₁-C₄ alkylene refers to alkylene containing 1, 2, 3 or 4 carbon atoms.

The term "haloalkyl" refers to an alkyl group in which one or more H has been replaced by the halogen atom. The term "haloalkoxyl" refers to -O-haloalkyl group.

The term "oxo" or "oxo group" refers to the oxygen atom as a divalent substituent, forming a carbonyl group when attached to a carbon, or forming a sulfinyl or sulfonyl or N-oxide group when attached to a heteroatom.

In the present invention, unless otherwise indicated, the term "aromatic ring", "aromatic ring" or "aromatic heterocycle" refers to a carbocycle or heterocycle having one or more polyunsaturated rings having aromatic character (i.e., having (4n+2) delocalized πelectrons, wherein, n is an integer).

The term "aryl" refers to a substituted or unsubstituted stable aromatic hydrocarbon group having 6 to 10 ring carbon atoms, which may contain 1 or more aromatic rings (eg fused and bicyclic). The aryl ring does not contain heretoatoms. Examples of aryl include, but not limited to phenyl, naphthyl, indenyl, etc.

The term "heteroaryl" refers to a monocyclic or polycyclic (e.g. fused bicyclic) aromatic heterocycle having at least one heteroatom ring member, the heteroatom is selected from N, O and/or S. "5-18 membered" in 5-18 membered heteroaryl refers to heteroaryl containing 5-18 carbon atoms and N, O and/or S as ring atoms. Examples of such heteroaryl include, but are not limited to, pyridyl, pyrimidinyl, pyrrolyl, imidazolyl, thiazolyl, thienyl, benzimidazole, benzothienyl, benzofuranyl, and the like.

The term "cycloalkyl" refers to a ring system having at least one cyclized alkyl group. "C₃-C₁₀" in the term C₃-C₁₀ cycloalkyl means that the cycloalkyl group may have 3, 4, 5, 6, 7, 8, 9 or 10 ring atoms. The cycloalkylcan include monocyclic or polycyclic rings (e.g. with 2, 3 or 4 fused rings, spiro rings, paracyclic rings, etc.). In some embodiments, cycloalkyl include but are not limited to cyclopropyl, cyclobutyl, cyclopentyl, etc.; in some embodiments, cycloalkyl also includes moieties with one or more aromatic rings fused to the cyclized alkyl ring, such as benzo or thienyl derivatives of cyclohexane.

The term "cycloalkenyl" refers to a ring system containing at least one cyclized alkenyl group, the cycloalkenyl having one or more carbon-carbon double bonds. "C₃-C₁₀" in the term C₃-C₁₀ cycloalkenyl means that the cycloalkenyl group may have 3, 4, 5, 6, 7, 8, 9 or 10 ring-forming atoms. The cycloalkenyl group may include monocyclic or polycyclic rings (e.g. with 2, 3 or 4 fused rings, spiro rings, bridged rings, etc.). In some embodiments, cycloalkenyl groups include, but are not limited to, cyclohexenyl, cyclohexadiene, cycloheptatrienyl, etc.; in some embodiments, cycloalkenyl groups also include aromatic compounds having one or more cycloalkenyl rings fused parts of family rings, such as benzo or thienyl derivatives of cyclohexene rings, etc.

The term "heterocyclyl" refers to a cyclized alkyl or cyclized alkenyl ring system, which have at leat one heteroatom, the heteroatom is selected from N, O and/or S. The heterocyclyl group may include monocyclic or polycyclic rings (e.g. with 2, 3 or 4 fused rings, spiro rings, bridged rings, etc.). The heterocyclyl can be linked to other parts of the compound by ring carbon atoms or ring heteroatmos. The definition of heterocyclyl also includes one or more aromatic rings fused to cyclized alkyl or cyclized alkenyl rings, such as benzo or thienyl derivatives of piperidine, morpholine, etc. In some embodiments, heterocyclyl includes, but is not limited to, pyrrolidinyl, pyrrolinyl, tetrahydrothienyl, tetrahydrofuranyl, piperidinyl, morpholinyl, azacycloheptane, dihydrobenzofuranyl, and the like.

The term "composition", as used herein, is intended to encompass a product including the specified ingredients in the specified amounts, as well as any product which results, directly or indirectly, from combinations of the specified ingredients in the specified amounts. Accordingly, pharmaceutical compositions containing the compounds of the present invention as the active ingredient as well as methods of preparing the instant compounds are also part of the present invention. Furthermore, some of the crystalline forms for the compounds may exist as polymorphs and as such are intended to be included in the present invention. In addition, some of the compounds may form solvates with water (i.e., hydrates) or common organic solvents and such solvates are also intended to be encompassed within the scope of this invention.

The "compound" of the present invention includes the compound of formula (I), and all pharmaceutically acceptable forms. Such pharmaceutically acceptable forms include salts, solvates, non-covalent complexes, chelates, stereoisomers isomers (including diastereomers, enantiomers and racemates), geometric isomers, isotopically labeled compounds, tautomers, prodrugs, or any mixture of all of the above.

The "enantiomers" are a pair of stereoisomers that are non-superimposable, mirror-image stereoisomers, and a 1:1 mixture of a pair of enantiomers is a "racemic" mixture.

When specifying the stereochemistry of the compounds of the present invention, the conventional RS system (eg (1S,2S) to specify a single stereoisomer of known relative and absolute configuration with two chiral centers) is used. Optically active (R)- and (S)-isomers can be prepared using optically active starting material synthesis or chiral reagents, or resolved using conventional techniques (eg, separation on a chiral SFC or HPLC column).

The "diastereomers" are stereoisomers that have at least two asymmetric atoms, but which are not mirror images of each other. When the compound is a pure enantiomer, the stereochemistry at each chiral carbon can be specified by R or S.

Resolved compounds of unknown absolute configuration can be designated (+) or (-) depending on the direction in which they rotate plane-polarized light (right or left) at the wavelength of the sodium D line. Alternatively, resolved compounds can be defined by the corresponding retention times of the corresponding enantiomers/diastereoisomers by chiral HPLC.

Those skilled in the art will recognize that the compound of the present invention may contain one or more chiral centers, and may thereby give rise to diastereomers and optical isomers. The above formula (I) does not precisely define a certain position of the compound and therefore can exist in different isomeric forms. Unless otherwise specified, the present invention includes all possible stereoisomers of the compound represented by formula (I) and pharmaceutically acceptable salts thereof, such as elimination mixtures, optically pure forms and mixtures of isomers in any ratio. Further, mixtures of stereoisomers and isolated specific stereoisomers are also included in the present invention. In the synthesis process for preparing such compounds, or during the use of racemization or epimerization methods well known to those of ordinary skill in the art, the resulting product may be a mixture of stereoisomers.

Prodrugs of the compounds of the present invention are included within the scope of the present invention. Generally, the prodrugs refer to functional derivatives that are easily converted into the desired compound in vivo. Therefore, the term "administration" of the present invention includes the administration of a compound disclosed herein, or a prodrug compound that is not explicitly disclosed but is converted in vivo after administration to a subject. Conventional methods for the selection and preparation of suitable prodrug derivatives have been described in books such as ((Design of Prodrugs)) (Design of Prodrugs, ed. H. Bundgaard, Elsevier, 1985).

Clearly, the definition of variables of any substituent or position-specific in a molecule is independent of other positions in the molecule. It is easy to understand that those of ordinary skill in the art can select the substituents or substituted forms of the compounds of the present invention by means of the prior art and the methods, to provide chemically stable and easily synthesized compounds.

Clearly, the definition of variables of any substituent or position-specific in a molecule is independent of the definition of variables of any substituent or position-specific in other molecules. It is easy to understand that those of ordinary skill in the art can select the substituents or substituted forms according to the prior art of the discipline, the compound of the present invention that is chemically stable and easily synthesized by using the prior art of the discipline or by the method described in the present invention can be provided.

When the compound of formula (I) and pharmaceutically acceptable salt thereof is in the form of solvates or polymorphs, the present invention includes any possible solvates and polymorphs. The type of solvent that forms the solvate is not particularly limited as long as the solvent is pharmacologically acceptable. For example, water, ethanol, propanol, acetone and the like can be used.

The term "pharmaceutically acceptable salts" refers to salts prepared from pharmaceutically acceptable non-toxic bases or acids. When the compound of the present invention is acidic, its corresponding salt can be conveniently prepared from pharmaceutically acceptable non-toxic bases, including inorganic bases and organic bases. Salts derived from such inorganic bases include aluminum, ammonium, calcium, copper (ic and ous), ferric, ferrous, lithium, magnesium, manganese (ic and ous), potassium, sodium, zinc and the like salts. Particularly preferred are the ammonium, calcium, magnesium, potassium and sodium salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, *N*',*N*'-dibenzylethylenediamine, diethylamine, 2-diethylamino, ethanol, 2-dimethylaminoethanol, ethanolamine, ethylenediamine, N-ethylmorpholine, N-ethylpiperidine, reduced glucosamine, amino, glucose, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

When the compound of the present invention is basic, its corresponding salt can be conveniently prepared from pharmaceutically acceptable non-toxic acids, including inorganic and organic acids. Such acids include, for example, acetic acid, benzenesulfonic acid, benzoic acid, camphorsulfonic acid, citric acid, ethanesulfonic acid, formic acid, fumaric acid, gluconic acid, glutamic acid, hydrobromic acid, hydrochloric acid, isethionic acid, lactic acid, maleic acid, malic acid, mandelic acid, methanesulfonic acid, mucic acid, nitric acid, pamoic acid, pantothenic acid, phosphoric acid, succinic acid, sulfuric acid, oxalic acid, propionic acid, glycolic acid, hydroiodic acid, perchloric acid, cyclohexamic acid, salicylic acid, 2-naphthalenesulfonic acid, saccharinic acid, trifluoroacetic acid, tartaric acid, p-toluenesulfonic acid and the like. Preferred are citric acid, hydrobromic acid, formic acid, hydrochloric acid, maleic acid, phosphoric acid, sulfuric acid and tartaric acids, particularly preferred are formic acid and hydrochloric acid. Since the compounds of Formula (I) are intended for pharmaceutical use they are preferably provided in substantially pure form, for example at least 60% pure, more suitably at least 75% pure, especially at least 98% pure (% are on a weight for weight basis).

The pharmaceutical compositions of the present invention contain a compound represented by Formula (I) (or a pharmaceutically acceptable salt thereof) as an active ingredient, a pharmaceutically acceptable carrier and optionally other therapeutic ingredients or adjuvants. The compositions include compositions suitable for oral, rectal, topical, and parenteral (including subcutaneous, intramuscular, and intravenous) administration, although the most suitable route in any given case will depend on the particular host, and nature and severity of the conditions for which the active ingredient is being administered. The pharmaceutical compositions may be conveniently presented in unit dosage form and prepared by any of the methods well known in the art of pharmacy.

In practice, the compounds represented by Formula (I) or a prodrug, or a metabolite, or pharmaceutically acceptable salts thereof, of the present invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). Thus, the pharmaceutical compositions of the present invention can be presented as discrete units suitable for oral administration such as capsules, cachets or tablets each containing a predetermined amount of the active ingredient. Further, the pharmaceutical compositions of the present invention can be presented as a powder, as granules, as a solution, as a suspension in an aqueous liquid, as a non-aqueous liquid, as oil-in-water emulsion, or as a water-in- oil liquid emulsion. In addition to the common dosage forms set out above, the compound represented by Formula (I), or a pharmaceutically acceptable salt thereof, may also be administered by controlled release means and/or delivery devices. The compositions may be prepared by any of the methods of pharmacy. In general, such methods include a step of bringing into association the active ingredient with the carrier that constitutes one or more necessary ingredients. In general, the compositions are prepared by uniformly and intimately mixing the active ingredient with liquid carriers or finely divided solid carriers or both. The product can then be conveniently shaped into the desired presentation.

Thus, the pharmaceutical compositions of this invention may include a pharmaceutically acceptable carrier and a compound, or a pharmaceutically acceptable salt thereof. The compounds of Formula (I), or pharmaceutically acceptable salts thereof, can also be included in pharmaceutical compositions in combination with one or more other therapeutically active compounds.

The pharmaceutical carrier employed can be, for example, a solid, liquid, or gas. Examples of solid carriers include such as lactose, terra alba, sucrose, talc, gelatin, agar, pectin, acacia, magnesium stearate, and stearic acid. Examples of liquid carriers include such as sugar syrup, peanut oil, olive oil, and water. Examples of gaseous carriers include such as carbon dioxide and nitrogen. In preparing the compositions for oral dosage form, any convenient pharmaceutical media may be employed. For example, water, glycols, oils, alcohols, flavoring agents, preservatives, coloring agents, and the like may be used to form oral liquid preparations such as suspensions, elixirs and solutions; while carriers such as starches, sugars, microcrystalline cellulose, diluents, granulating agents, lubricants, binders, disintegrating agents, and the like may be used to form oral solid preparations such as powders, capsules and tablets. Because of their ease of administration, tablets and capsules are the preferred oral dosage units whereby solid pharmaceutical carriers are employed. Optionally, tablets may be coated by standard aqueous or nonaqueous techniques.

A tablet containing the composition of this invention may be prepared by compression or molding, optionally with one or more accessory ingredients or adjuvants. Compressed tablets may be prepared by compressing, in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Each tablet preferably contains from about 0.05mg to about 5g of the active ingredient and each cachet or capsule preferably containing from about 0.05mg to about 5g of the active ingredient. For example, a formulation intended for the oral administration to humans may contain from about 0.5mg to about 5g of active agent, compounded with an appropriate and convenient amount of carrier material which may vary from about 5 to about 95 percent of the total composition. Unit dosage forms will generally contain between from about 1 mg to about 2g of the active ingredient, typically 25mg, 50mg, 100mg, 200mg, 300mg, 400mg, 500mg, 600mg, 800mg, or 1000mg.

Pharmaceutical compositions of the present invention suitable for parenteral administration may be prepared as solutions or suspensions of the active compounds in water. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions of the present invention suitable for injectable use include sterile aqueous solutions or dispersions. Furthermore, the compositions can be prepared in the form of sterile powders for the extemporaneous preparation of such sterile injectable solutions or dispersions. In all cases, the final injectable form must be sterile and must be effectively fluid for easy syringeability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), vegetable oils, and suitable mixtures thereof.

Pharmaceutical compositions of the present invention can be in a form suitable for topical administration, for example, aerosol, emulsion, ointment, lotion, dusting powder, or other similar dosage forms. Further, the pharmaceutical compositions provided by the present invention can be in a form suitable for use in transdermal devices. These formulations may be prepared, utilizing a compound represented by Formula (I) of this invention, or a pharmaceutically acceptable salt thereof, via conventional processing methods. As an example, emulsion or ointment is prepared by admixing hydrophilic material and water, together with about 5wt% to about 10wt% of the compound, to produce a emulsion or ointment having a desired consistency.

Pharmaceutical compositions of this invention can be prepared in the form of a solid is carrier suitable for rectal administration. It is preferable that the mixture forms unit dose suppositories. Suitable carriers include cocoa butter and other materials commonly used in the art. The suppositories may be conveniently formed by first mixing the composition with the softened or melted carrier(s) followed by chilling and shaping in molds.

In addition to the aforementioned carrier ingredients, the pharmaceutical formulations described above may include, as appropriate, one or more additional carrier ingredients such as diluents, buffers, flavoring agents, binders, surface-active agents, thickeners, lubricants, preservatives (including antioxidants) and the like. Furthermore, other adjuvants can also include osmotic agent that regulate the drug and blood isotonicity. Compositions containing a compound described by Formula (I), or pharmaceutically acceptable salts thereof, may also be prepared in powder or liquid concentrate form.

Generally, to treat the conditions or disorder indicated above, the drug is administered at a dosage level of approximately 0.01 mg/kg to 150 mg/kg of body weight per day, or 0.5 mg to 7 g per patient per day. However, it is understood that specific dose level and treatment regimens for any particular subject will depend upon a variety of factors, including age, body weight, general medical condition, sex, diet, time of administration, route of administration, rate of excretion, concomitant drug use and the severity of the particular disease being treated.

### EXAMPLES

In order to make the present invention easier to understand, the present invention was described in detail with reference to the examples. These examples are only for illustrative purposes and are not limited to the scope of application of the present invention. The experimental methods in the present invention, unless otherwise specified, all are conventional methods. The experimental materials used in the present invention are purchased from the market unless otherwise specified.

Unless otherwise specified, all parts and percentages are by weight and all temperatures are in degrees Celsius.

The following abbreviations have been used in the examples:
CuI: Cuprous iodide;
DAST: Diethylaminosulfur trifluoride;
DCM: Dichloromethane;
DMF: Dimethylformamide;
DMSO: Dimethyl sulfoxide;
EA: Ethyl Acetate;
ESI-MS: Electrospray ionization mass spectrometry;
K₂CO₃: Potassium carbonate;
NaH: Sodium hydride;
LDA: Lithium diisopropylamide;
LiHMDS: Lithium bis(trimethylsilyl)amide;
m-CPBA: m-chloroperoxybenzoic acid;
MtBE: Methyl tert-butyl ether;
Na₂SO₄: Sodium sulfate;
NaBH₄: Sodium borohydride;
NBS: N-bromosuccinimide;
NF SI: N-Fluorobisbenzenesulfonamide;
NIS: N-Iodosuccinimide;
NMP: N-Methylpyrrolidone;
PE: Petroleum ether;
Pd₂(dba)₃: Tris(dibenzylideneacetone)dipalladium;
Pd(dppf)Cl₂: 1,1'-Bis(diphenylphosphino)ferrocene]palladium dichloride;
Pd(PPh₃)₄: Tetrakis(triphenylphosphine)palladium;
Prep-TLC: Preparative Thin Layer Chromatography;
SelectFlour: 1-Chloromethyl-4-fluoro-1,4-azabicyclo[2.2.2]octanebis(tetrafluoro borate);
TBAF: Tetrabutylammonium fluoride;
TEA: Triethylamine;
THF: Tetrahydrofuran;
Xantphos: 4,5-Bis(diphenylphosphine)-9,9-dimethylxanthene;
TLC: Thin layer chromatography;
ESI-MS: Electrospray ionization mass spectrometry;
LCMS or LC-MS: liquid chromatography mass spectrometry;
¹H NMR: hydrogen-1 nuclear magnetic resonance;
[(R,R)-Ts-DPEN]RuCl(p-cymene): Chlorine {[(1R,2R)-(-)-2-amino-1,2-diphenylethyl](4-toluenesulfonyl)amino}(P-isopropyltoluene)ruthenium(II).

### Synthesis of intermediate M1 (5,5-difluoro-3-iodo-L5,6,7-tetrahydro-4H-indol-4-one)

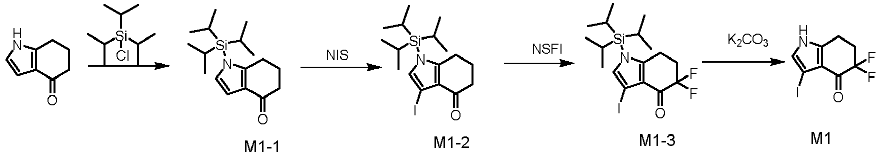

### Step 1: Synthesis of compound M1-1

6,7-dihydro-1*H*-indol-4(5*H*)-one (13.5 g) was dissolved in DMF (100 mL) under N₂ protection, 60% NaH (4.4 g) was added slowly in an ice bath, after stirred for 0.5 h, triisopropylchlorosilane (21.2 g) was added. The reaction mixture was stirred at 0 °C for 3 h, the reaction mixture was poured into a mixture of water and ice (500 mL), extracted with EA (500 mL^{∗}2), washed with saturated sodium chloride solution (500 mL^{∗}2), dried, concentrated to give a crude product, which was purified by column chromatography (EA/PE=1:10) to afford the target compound M1-1 (20.3 g). ESI-MS m/z: 292.10[M+H]⁺.

### Step 2: Synthesis of compound M1-2

Compound M1-1 (13.5 g) was dissolved in DMF (100 mL) under N₂ protection, NIS (11.5 g) was added in portions, stirred at room temperature for 7 h, the reaction mixture was poured into a mixture of water and ice (500 mL), extracted with EA (500 mL^{∗}2), washed with saturated sodium chloride solution (500 mL^{∗}2), dried, concentrated to give a crude product, which was purified by column chromatography (EA/PE=1:10) to afford the target compound M1-2 (8.7 g). ESI-MS m/z: 418.10 [M+H]⁺.

### Step 3: Synthesis of compound M1-3

Compound M1-2 (5.5 g) was dissolved in THF (50 mL), cooled to -78°C, a solution og LiHMDS in THF (1.0 M, 35.6 mL) was added slowly, after stirred at room temperature for 0.5 h, NFSI (8.72 g) in THF (50 mL) was added, and stirring was continued at room temperature for 2 h, the reaction was quenched by the addition of saturated ammonium chloride, extracted twice with EA, washed twice with saturated sodium chloride solution, dried, concentrated to give a crude product, which was purified by column chromatography (EA/PE=1:10) to afford the target compound M1-3 (2.1 g).ESI-MS m/z: 454.10 [M+H]⁺.

### Step 4: Synthesis of compound M1

Compound M1-3 (2.1 g) was dissolved in a mixture of THF (20 mL) and water (20 mL), K₂CO₃ (1.27 g) was added under room temperature, stirred at room temperature for 3 h. The reaction mixture was poured into water, extracted with EA, the organic layer was washed with sodium chloride solution, dried over Na₂SO₄, concentrated to give a crude product, which was purified by column chromatography (100%DCM) to afford the target compound M1 (0.5 g).ESI-MS m/z: 297.99 [M+H]⁺.¹H NMR (500 MHz, DMSO-d6): δ 12.05 (s, 1H), 7.12 (d, J = 2.3 Hz, 1H), 2.98 (t, J = 6.2 Hz, 2H), 2.60-2.53 (m, 2H).

### Synthesis of intermediate M2 (5,5-difluoro-3-(trifluoromethyl)-1,5,6,7-tetrahydro-4H-indol-4-one)

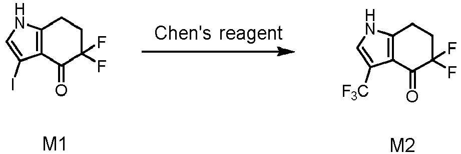

M1 (12 g), cuprous iodide (7.8 g), methyl fluorosulfonyl difluoroacetate (24 g) were added into DMF (120 mL), the atmosphere was evacuated and replaced with nitrogen, the reaction was heated to 130 °C under nitrogen protection, stirred overnight, TLC and LCMS showed that no starting material remained, the temperature was cooled down, the reaction mixture was diluted with EA, the organic layer was washed with saturated sodium chloride solution, dried over Na₂SO₄, concentrated to give a crude product, which was purified by column chromatography (EA/PE=1: 5) to afford the target compound M2(8.4 g). ESI-MS m/z: 238.00 [M-H]⁻. ¹H NMR (500 MHz, DMSO) δ 12.37 (s, 1H), 7.56 (s, 1H), 3.03 (t, *J* = 6.1 Hz, 2H), 2.68 - 2.55 (m, 2H).

### Synthesis of intermediate M3 (5-fluoro-3-(trifluoromethyl)-1,5,6,7-tetrahydro-4H-indol-4-one)

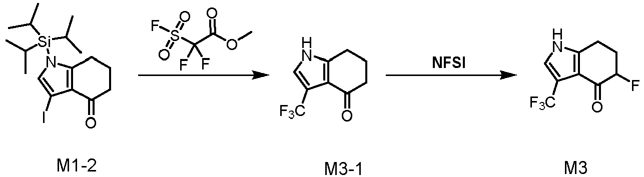

### Step 1: Synthesis of compound M3-1

Compound M1-2 (834 mg), cuprous iodide (380 mg), NMP (20 mL) and methyl fluorosulfonyl difluoroacetate (1.15 g) were added to 50 mL single-neck bottle sequentially, heated to 120°C, stirred for 6h, the reaction mixture was diluted with EA, washed three times with water and once with saturated brine, dried, concentrated to give a crude product, which was purified by column chromatography (DCM =100%) to afford the target compound M 3-1 (264 mg). ESI-MS m/z: 204.08[M+H]⁺.

### Step 2: Synthesis of compound M3

Compound M3-1 (264 mg) was dissolved in ultra-dry THF (20 mL) under N₂ protection, cooled to -78 °C, a solution of LDA in THF (1 M, 2.5 mL) was added slowly, after stirred for 0.5 h, NFSI (491 mg) in THF (5 mL) was added, stirring was continued at -78°C for 2 h. The reaction was quenched by the addition of saturated ammonium chloride, extracted twice with EA, washed twice with saturated sodium chloride solution, dried, concentrated to give a crude product, which was purified by column chromatography (DCM =100%) to afford the target compound M3 (147 mg). ESI-MS m/z: 222.12[M+H]⁺.

### Synthesis of intermediate M4 (3-bromo-7-fluoro-1-(trifluoromethyl)-6,7-indoline-8(5H)-one) and intermediate M5 (3-bromo-7,7-difluoro-1-(trifluoromethyl)-6,7-indoline-8(5H)-one)

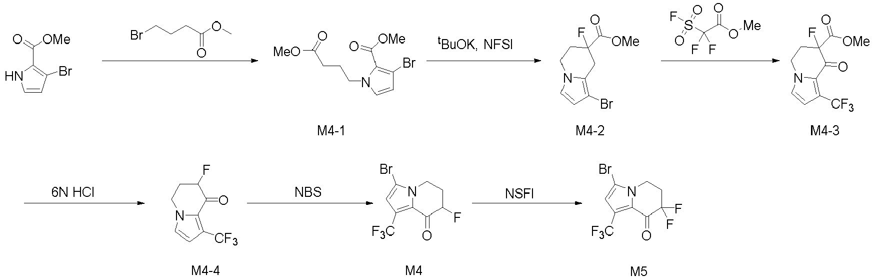

### Step 1: Synthesis of compound M4-1

3-bromo-1H-pyrrole-2-carboxylate methylester (20.4 g) and methyl 4-bromobutyrate (19.8 g) were dissolved in DMF (100 mL) under N₂ protection, cesium carbonate (65.2 g) was added, after stirred at room temperature for 4 h, the reaction solution was poured into a mixture of ice and water (500 mL), extracted with EA, washed with saturated sodium chloride solution (500 mL^{∗}2), dried over anhydrous sodium sulfate, concentrated to give a crude product, which was purified by column chromatography (EA/PE=1:10) to afford the target compound M4-1 (28.3 g). ESI-MS m/z: 304.10[M+H]⁺.

### Step 2: Synthesis of compound M4-2

Compound M4-1 (15.2 g) was dissolved in THF (100 mL) under N₂ protection, solution of potassium tert-butoxide in THF (1.0 M, 60 mL) was added dropwise in an ice bath, stirred at 0°C for 3h and then cooled to -78°C, solution of NFSI (18.9 g) in THF (50 mL) was added, stirring at -78°C was continued for another 1h, the reaction was quenched by the addition of saturated ammonium chloride, extracted with EA, washed with saturated sodium chloride solution, dried over anhydrous sodium sulfate, concentrated to give a crude product, which was purified by column chromatography (EA/PE=1:5) to afford the target compound M4-2 (9.1 g). ESI-MS m/z: 277.15 [M+H]⁺.

### Step 3: Synthesis of compound M4-3

Compound M4-2 (8.3 g) was dissolved in DMF (100 mL) under N₂ protection, palladium acetate (3.3 g), CuI (11.4 g) and methyl fluorosulfonyl difluoroacetate (23 g) were added, stirred at 100°C for 3h, extracted with EA, the organic layer was washed with saturated sodium chloride solution, dried over Na₂SO₄, concentrated to give a crude product, which was purified by column chromatography (EA/PE=1:4) to afford the target compound M4-3 (4.23 g). ESI-MS m/z: 279.20 [M+H]⁺.

### Step 4: Synthesis of compound M4-4

Compound M4-3 (4.23 g) was dissolved in a mixture of ethanol (20 mL) and 6 M aqueous hydrochloric acid (20 mL), the reaction mixture was heated at reflux for 3h. Concentrated under reduced pressure to remove ethanol, the residue was extracted with EA (100 mL^{∗}2), the organic layer was washed with saturated sodium chloride solution (100 mL^{∗}2), dried over Na₂SO₄, concentrated to give a crude product, which was purified by column chromatography (EA/PE=1:4) to afford the target compound M4-4 (2.1 g). ESI-MS m/z: 221.20 [M+H]⁺.

### Step 5: Synthesis of compound M4

Compound M4-4 (2.1 g) was dissolved in acetonitrile (20 mL), NBS (1.8 g) was added at room temperature, the reaction mixture was heated at reflux for 4h, quenched by the addition of water, extracted with EA, washed with saturated sodium chloride solution, dried over anhydrous sodium sulfate, concentrated to give a crude product, which was purified by column chromatography (EA/PE=1:3) to afford the target compound M4 (2.5 g). ESI-MS m/z: 300.10 [M+H]⁺.

### Step 6: Synthesis of compound M5

Compound M4 (1.3 g) was dissolved in THF (50 mL), cooled to -78°C, solution of LiHMDS in THF (1.0 M, 6 mL) was added dropwise, after stirred for 0.5h, solution of NFSI (1.5 g) in THF (10 mL) was added dropwise, continued stirring for 2h, the reaction was quenched by the addition of saturated ammonium chloride, extracted twice with EA, washed twice with saturated sodium chloride solution, dried, concentrated to give a crude product, which was purified by column chromatography (EA/PE=1:3) to afford the target compound M5 (2.1 g).ESI-MS m/z: 318.14 [M+H]⁺.

### Synthesis of intermediate M6 (5,6-difluoro-3-(trifluoromethyl)-1,5,6,7-tetrahydro-4H-indol-4-one)

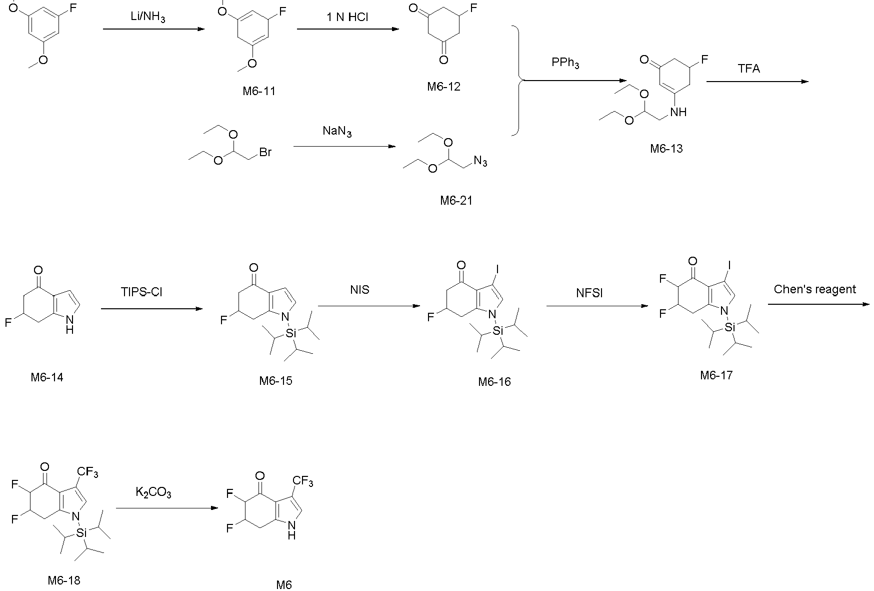

### Step 1: Synthesis of compound M6-11

1,3-dimethoxy-5-fluorobenzene (15.6 g) was dissolved in tert-butanol (25 mL) and THF (15 mL) under N₂ protection, the mixture was added to liquid ammonia solution (650 mL), metal lithium (1.75 g) was added in batches, the blue solution was stirred at -65°C for 3h, solid ammonium chloride was added, until the blue color was disappeared, the temperature was allowed to rise to evaporate and remove the liquid ammonia, the residue was diluted with petroleum ether, washed with water, and washed with saturated brine, dried and concentrated to afford the target product M6-11 (10 g).

### Step 2: Synthesis of compound M6-12

Compound M6-11 (10 g) was dissolved in THF (100 mL), hydrochloric acid (1 N, 100 mL) was added, stirred at room temperature overnight, diluted with EA, extracted three times, dried and concentrated to afford the target product M6-12 (10 g). ESI-MS m/z: 129.10 [M-H]⁻.

### Step 3: Synthesis of compound M6-21

Compound 2-bromo-1,1-dimethoxyethane (82.4 mL) was dissolved in DMSO (14 mL), sodium azide (1.95 g) and potassium iodide (0.33 g) were added, heated to 90 °C and stirred for 5 days, cooled to room temperature, the reaction was diluted with water and ether, the water layer was extracted three times with ether, dried and concentrated to afford M6-21 (30 g).

### Step 4: Synthesis of compound M6-13

Compound M6-21 (5.73 g) was dissolved in THF (150 mL), M6-12 (3.86 g) and triphenylphosphine (11.8 g) were added, heated to reflux overnight, cooled to room temperature, concentrated, and purified by column chromatography to afford M6-13 (7.24 g).

### Step 5: Synthesis of compound M6-14

Compound M6-13 (7.24 g) was dissolved in dichloromethane (100 mL), the reaction mixture was cooled to 0 °C, trifluoroacetic acid (5 mL) was added, stirred at room temperature overnight, concentrated, and purified by column chromatography to afford M6-14 (5 g).

### Step 6: Synthesis of compound M6-15

Refer to the preparation method of compound M1-1 to obtain M6-15.

### Step 7: Synthesis of compound M6-16

Refer to the preparation method of compound M1-2 to obtain M6-16.

### Step 8: Synthesis of compound M6-17

Refer to the preparation method of step 2 in compound M3 to obtain M6-17.

### Step 9: Synthesis of compound M6-18

Refer to the preparation method of intermediate M2 to obtain M6-18.

### Step 10: Synthesis of compound M6

Refer to the preparation method of step 4 in intermediate M1 to obtain M6.

### Example 1 and example 2 Synthesis of 1-(3-chloro-5-fluorophenyl)-5,5-difluoro-3-iodo-1,5,6,7-tetrahydro-4H-indol-4-one ( compound A13) and 1-(3-chloro-5-fluorophenyl)-5,5-difluoro-3-iodo-4,5,6,7-tetrahydro-1H-indol-4-ol ( compound A2)

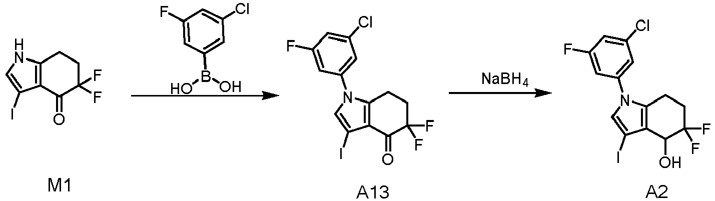

### Step 1: Synthesis of compound A13

Compound M1 (300 mg) was dissolved in DCM (20 mL), copper acetate (90 mg), 3-chloro-5-fluorophenylboronic acid (350 mg) and TEA (404 mg) were added, stirred at room temperature overnight. The reaction mixture was diluted with DCM, washed twice with saturated brine, dried, concentrated to give a crude product, which was purified by column chromatography (EA/PE=1:5) to afford the target compound A13 (255 mg). ESI-MS m/z: 426.09[M+H]⁺.

### Step 1: Synthesis of compound A2

Compound A13 (43 mg) was dissolved in methanol (2 mL), NaBH₄ (8 mg) was added in an ice bath, stirred at 0°C for 1h. Ice water was added and the mixture was extracted with EA, the organic layer was washed with with brine, dried over Na₂SO₄, concentrated to give a crude product, which was purified by Prep-TLC(EA/PE=1:5) to afford the target compound A2 (34 mg). ESI-MS m/z: 409.90[M+H-H₂O]⁺.

Using a method substantially similar to example 1, such as replacing 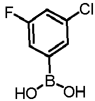 with 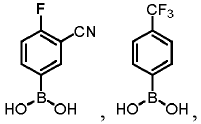 etc., to prepare the following example.

| **Example number** | **Structure** | **Chemical name** | **ESI-MS** |
|---|---|---|---|
| 3 | 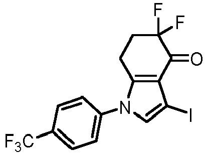 | 5,5-difluoro-3-iodo-1-(4-(trifluoromethyl)phenyl)-1,5,6,7-tetrahydro-4H-indol-4-one | [M+H]⁺: 442.06 |
| 4 | 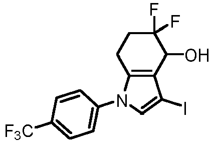 | 5,5-difluoro-3-iodo-1-(4-(trifluoromethyl)phenyl)-4,5,6,7-tetrahydro-1H-indol-4-ol | [M+H-H₂O]⁺: 425.90 |
| 5 | 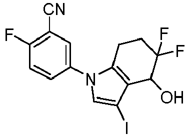 | 5 -(5,5-difluoro-4-hydroxy-3 - iodo-4,5,6,7-tetrahydro-1H-indol-1-yl)-2-fluorobenzonitrile | [M+H-H₂O]⁺: 401.01 |

### Example 6 and example 7 Synthesis of 1-(3-chloro-5-fluorophenyl)-5,5-difluoro-3-(trifluoromethyl)-15,6,7-tetrahydro-4H-indol-4-one ( compound A18) and 1-(3-chloro-5-fluorophenyl)-5,5-difluoro-3-(trifluoromethyl)-4 5,6,7-tetrahydro-1H-indol-4-ol ( compound A1)

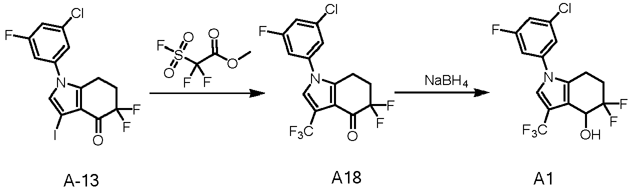

### Step 1: Synthesis of compound A18

Compound A13 (255 mg) was dissolved in DMF (5 mL) under nitrogen protection, Pd₂(dba)₃ (165 mg), CuI (114 mg) and methyl fluorosulfonyldifluoroacetate (230 mg) were added, stirred at 100°C for 3 hours, the reaction mixture was extracted with ethyl acetate, the organic layer was washed with brine, dried over Na₂SO₄, and concentrated to obtain the crude product, which was purified by column chromatography (EA/PE=1:5) to obtain the target compound A18 (176 mg). ESI-MS m/z: 368.20 [M+H]⁺.

### Step 2: Synthesis of compound A1

Compound A18 (147 mg) was dissolved in methanol (4 mL), NaBH₄(30 mg) was added in an ice bath, after stirring at 0 °C for 1 hour, ice water was added, the mixture was extracted with ethyl acetate, the organic layer was washed with brine, dried over Na₂SO₄, concentrated to obtain crude product, which was purified by Prep-TLC (EA/PE=1:5) to obtain the target compound A1 (118 mg). ESI-MS m/z:352.11[M+H-H₂O]⁺.

Using a method substantially similar to example 1-example 7, such as replacing 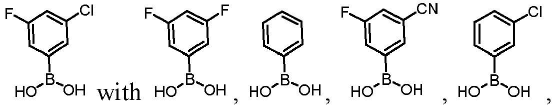 etc., to prepare the following example.

| **Example number** | **Structure** | **Chemical name** | **ESI-MS** |
|---|---|---|---|
| 8 | 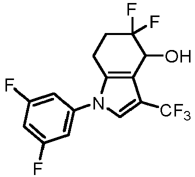 | 1-(3,5-difluorophenyl)-5,5-difluoro-3-(trifluoromethyl)-4,5,6,7-tetrahydro-1H-indol-4-ol | [M+H-H₂O]⁺: 336.05 |
| 9 | 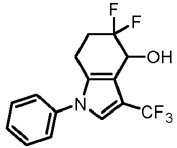 | 5,5-difluoro-1-phenyl-3-(trifluoromethyl)-4,5,6,7-tetrahydro-1H-indol-4-ol | [M+H-H₂O]⁺: 300.10 |
| 10 | 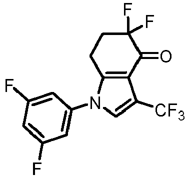 | 1-(3,5-difluorophenyl)-5,5-difluoro-3-(trifluoromethyl)-1,5,6,7-tetrahydro-4H-indol-4-one | [M+H]⁺: 352.11 |
| 11 | 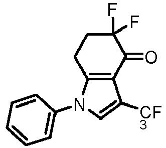 | 5,5-difluoro-1-phenyl-3-(trifluoromethyl)-1,5,6,7-tetrahydro-4H-indol-4-one | [M+H]⁺: 316.12 |
| 12 | 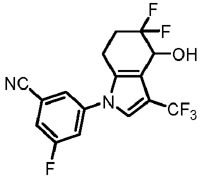 | 3-(5,5-difluoro-4-hydroxy-3- (trifluoromethyl)-4,5,6,7-tetrahydro-1H-indol-1-yl)-5-fluorobenzonitrile | [M+H-H₂O]⁺: 343.10 |
| 13 | 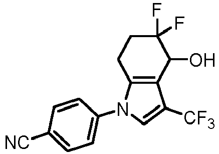 | 4-(5,5-difluoro-4-hydroxy-3-(trifluoromethyl)-4,5,6,7-tetrahydro-1H-indol-1-yl)benzonitrile | [M+Na⁺-H₂O]⁺: 382.98 |
| 14 | 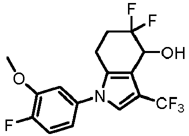 | 5,5-difluoro-1-(4-fluoro-3-methoxyphenyl)-3-(trifluoromethyl)-4,5 ,6,7-tetrahydro-1H-indol-4-ol | [M+H-H₂O]⁺: 348.08 |
| 15 | 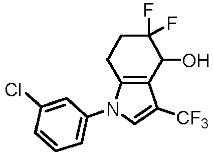 | 1-(3-chlorophenyl)-5,5-difluoro-3-(trifluoromethyl)-4,5,6,7-tetrahydro-1H-indol-4-ol | [M+H-H₂O]⁺: 334.04/336.04 |
| 16 | 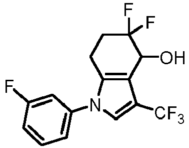 | 5,5-difluoro-1-(3-fluorophenyl)-3-(trifluoromethyl)-4,5,6,7-tetrahydro-1H-indol-4-ol | [M+H-H₂O]⁺: 318.15 |
| 17 | 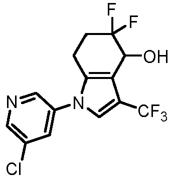 | 1-(5-chloropyridin-3-yl)-5,5-difluoro-3-(trifluoromethyl)-4,5,6,7-tetrahydro-1H-indol-4-ol | [M+H]⁺: 353.11 |
| 18 | 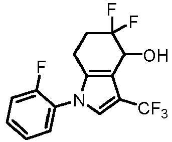 | 5,5-difluoro-1-(2-fluorophenyl)-3-(trifluoromethyl)-4,5,6,7-tetrahydro-1H-indol-4-ol | [M+H-H₂O]⁺: 318.12 |
| 19 | 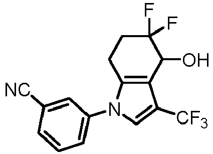 | 3-(5,5-difluoro-4-hydroxy-3- (trifluoromethyl)-4,5,6,7-tetrahydro-1H-indol-1-yl)benzonitrile | [M+H-H₂O]⁺: 325.08 |
| 20 | 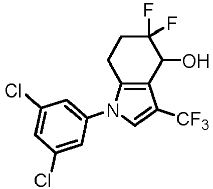 | 1-(3,5-dichlorophenyl)-5,5-difluoro-3-(trifluoromethyl)-4,5,6,7-tetrahydro-1H-indol-4-ol | [M+H-H₂O]⁺: 368.11 |
| 21 | 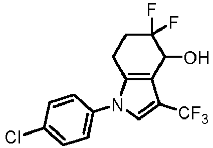 | 1-(4-chlorophenyl)-5,5-difluoro-3-(trifluoromethyl)-4,5,6,7-tetrahydro-1H-indol-4-ol | [M+H-H₂O]⁺: 334.11 |
| 22 | 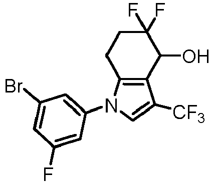 | 1-(3-bromo-5-fluorophenyl)-5,5 - difluoro-3-(trifluoromethyl)-4,5,6,7-tetrahydro-1H-indol-4-ol | [M+H-H₂O]⁺: 395.92 |
| 23 | 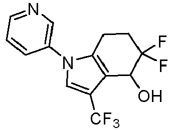 | 5,5-difluoro-1-(pyridin-3-yl)-3 - (trifluoromethyl)-4,5,6,7-tetrahydro-1H-indol-4-ol | [M+H]⁺: 319.10 |
| 24 | 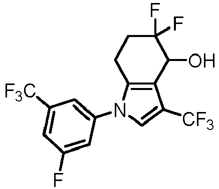 | 5,5-difluoro-1-(3-fluoro-5-(trifluoromethyl)phenyl)-3-(trifluoromethyl)-4,5 ,6,7-tetrahydro-1H-indol-4-ol | [M+H-H₂O]⁺: 386.00 |
| 25 | 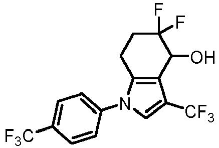 | 5,5-difluoro-3-(trifluoromethyl)-1-(4-(trifluoromethyl)phenyl)-4,5,6,7-tetrahydro-1H-indol-4-ol | [M+H-H₂O]⁺: 368.25 |
| 26 | 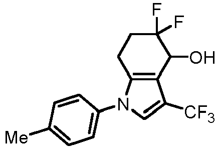 | 5,5-difluoro-1-(p-tolyl)-3-(trifluoromethyl)-4,5,6,7-tetrahydro-1H-indol-4-ol | [M+H-H₂O]⁺: 314.16 |
| 27 | 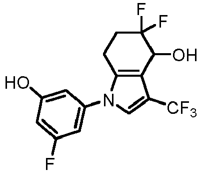 | 5,5-difluoro-1-(3-fluoro-5-hydroxyphenyl)-3-(trifluoromethyl)-4,5,6,7-tetrahydro-1H-indol-4-ol | [M+H-H₂O]⁺: 334.00 |

### Example 28 and example 29 Synthesis of 1-(3-chloro-5-fluorobenzyl)-5,5-difluoro-3-(trifluoromethyl)-15,6,7-tetrahydro-4H-indol-4-one ( compound A19) and 1-(3-chloro-5-fluorobenzyl)-5,5-difluoro-3-(trifluoromethyl)-4,5,6,7-tetrahydro-1H-indol-4-ol ( compound A3)

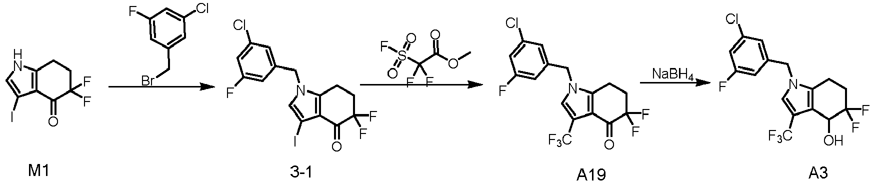

### Step 1: Synthesis of compound 3-1

Intermediate M1 (300 mg) was dissolved in DMF (10 mL), K₂CO₃ (276 mg) and 3-chloro-5-fluorobenzyl bromide (245 mg) were added, stirred at room temperature for two hours. The reaction mixture was poured into water, extracted twice with ethyl acetate, washed twice with saturated brine, dried, and concentrated to obtain the crude product, which was purified by column chromatography (EA/PE=1:7) to obtain the target compound 3-1 (307 mg). ESI-MS m/z: 440.10 [M+H]⁺.

### Step 2: Synthesis of compound A19

Compound 3-1 (307 mg) was dissolved in DMF (5 mL) under nitrogen protection, Pd₂(dba)₃ (192 mg), CuI(133 mg) and methyl fluorosulfonyldifluoroacetate (265 mg) were added, stirred at 100 °C for three hours, the reaction solution was extracted with ethyl acetate, the organic layer was washed with brine, dried over Na₂SO₄, and concentrated to obtain the crude product, which was purified by column chromatography (EA/PE=1:10) to obtain the target compound A19 (186 mg). ESI-MS m/z: 382.20 [M+H]⁺.

### Step 3: Synthesis of compound A3

Compound A19 (186 mg) was dissolved in methanol (4 mL), NaBH₄ (56 mg) was added in an ice bath, after stirring at 0 °C for 1 hour, ice water was added, the mixture was extracted with ethyl acetate, the organic layer was washed with brine, dried over Na₂SO₄ and concentrate to obtain the crude product, which was purified by Prep-TLC (EA/PE=1:5) to obtain the target compound A3 (158 mg). ESI-MS m/z: 366.22[M+H-H₂O]⁺.

Using a method substantially similar to example 1-example 28, such as replacing 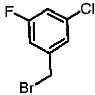 with 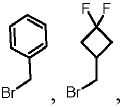 etc., to prepare the following example.

| **Example number** | **Structure** | **Chemical name** | **ESI-MS** |
|---|---|---|---|
| 30 | 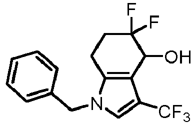 | 5,5 -difluoro-1-phenyl-3-(trifluoromethyl)-1,5,6,7-tetrahydro-4H-indol-4-ol | [M+H-H₂O]⁺ : 314.14 |
| 31 | 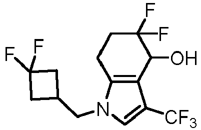 | 1-((3,3-difluorocyclobutyl)methyl)-5,5-difluoro-3-(trifluoromethyl)-4,5,6,7-tetrahydro-1H-indol-4-ol | [M+H-H₂O]⁺ : 328.10 |
| 32 | 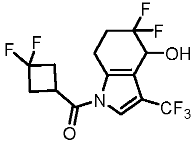 | (5,5-difluoro-4-hydroxy-3-(trifluoromethyl)-4,5,6,7-tetrahydro-1H-indol-1-yl)(3,3-difluorocyclobutyl)methanone | [M+H-H₂O]⁺ : 342.07 |

### Example 33 and example 34 Synthesis of 1-(3,5-difluorophenyl)-5,5-difluoro-1,5,6,7-tetrahydro-4H-indol-4-one ( compound A12) and 1-(3,5-difluorophenyl)-5,5-difluoro-4,5,6,7-tetrahydro-1H-indol-4-ol ( compound A4)

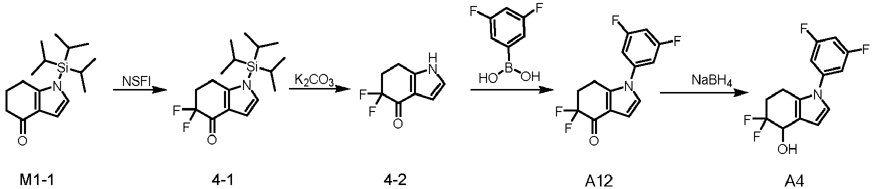

### Step 1: Synthesis of compound 4-1

Compound M1-1 (2.91 g) was dissolved inTHF (40 mL) under nitrogen protection, cooled to -78 °C, LiHMDS in tetrahydrofuran solution (1.0 M, 28 mL) was added slowly and dropwised, after stirred for 0.5 h, a solution of NFSI (6.6 g) in tetrahydrofuran (40 mL) was added slowly and dropwise, kept stirring at -78 °C for two hours. The reaction mixture was quenched by the addition of saturated aqueous ammonium chloride solution, extracted twice with ethyl acetate, washed twice with saturated brine, dried, and concentrated to obtain the crude compound 4-1 (2.8 g), which was directly used in the next step.

### Step 2: Synthesis of compound 4-2

The crude compound 4-1 (2.8 g) was dissolved in a mixed solvent of THF (20 mL) /H₂O (20 mL), K₂CO₃ (2.76 g) was added at room temperature, and stirring was continued for three hours. The reaction mixture was extracted with ethyl acetate, the organic layer was washed with brine, dried over Na₂SO₄, and concentrated to obtain the crude product, which was purified by column chromatography (DCM = 100%) to obtain the target compound 4-2 (512 mg). ESI-MS m/z: 172.12[M+H]⁺.

### Step 3: Synthesis of compound A12

Compound 4-2 (171 mg) was dissolved in DCM (50 mL), copper acetate (90 mg), 3,5-difluorophenylboronic acid (316 mg), TEA (404 mg) were added, stirred at room temperature overnight, the reaction mixture was diluted with dichloromethane, washed twice with saturated brine, dried, filtered, and concentrated to obtain the crude product, which was purified by column chromatography (EA/PE=1:5) to obtain the target compound A12 (170 mg). ESI-MS m/z: 284.08[M+H]⁺.

### Step 4: Synthesis of compound A4

Compound A12 (170 mg) was dissolved in methanol (4 mL), NaBH₄ (46 mg) was added in an ice bath, after stirred at 0°C for 1 hour, ice water was added, the mixture was extracted with ethyl acetate, and the organic layer was washed with brine, dried over Na₂SO₄ and concentrate to obtain the crude product, which was purified by Prep-TLC (EA/PE=1:5) to obtain the target compound A4 (137 mg). ESI-MS m/z:
268.13MS[M+H-H₂O]⁺.

### Example 35 and example 36 Synthesis of 1-cyclohexyl-5,5-difluoro-3-(trifluoromethyl)-15,6,7-tetrahydro-4H-indol-4-one ( compound A20) and 1-cyclohexyl-5,5-difluoro-3-(trifluoromethyl)-4,5,6,7-tetrahydro-1H-indol-4-ol

### ( compound A5)

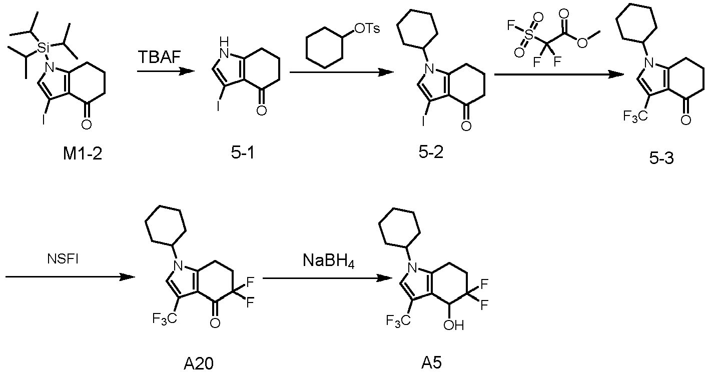

### Step 1: Synthesis of compound 5-1

Compound M1-2 (19 g), THF (150 mL) and TBAF (13.09 g) were added to a 500 ml single-necked flask sequentially, and the reaction was carried out at room temperature for 30 min. The residue was obtained by concentrating under reduced pressure, PE/EA (V: V=1:1, 100ml) was added to the residue, suction filtration after stirring, and the obtained filter cake was rinsed twice with EA to give the compound 5-1 (5.2g).

### Step 2: Synthesis of compound 5-2

Compound 5-1 (500 mg), cesium carbonate (2.5 g), cyclohexyl 4-methylbenzenesulfonate (1.2g) and DMF (10 mL) were added to a 50 ml single-necked bottle sequentially, the temperature was raised to 100 °C, and stirred overnight. The reaction mixture was diluted with EA, washed with water 3 times and with saturated sodium chloride solution once, dried, concentrated to obtain the crude product, which was purified by column chromatography to obtain the target compound 5-2 (230mg).

### Step 3: Synthesis of compound 5-3

Using the preparation method similar to compound A19, replacing compound 3-1with compound 5-2, the target compound 5-3 was prepared.

### Step 4: Synthesis of compound A20

Using the preparation method similar to Step 3 of intermediate M1, replacing compound M1-2 with compound 5-3, the target compound A20 was prepared.

### Step 5: Synthesis of compound A5

Using the preparation method similar to compound A4, replacing compound A12 with compound A20, the target compound A5 was prepared. LCMS: 306.18[M+H-H₂O]⁺.

### Example 37 Synthesis of 1-(3,5-difluorophenyl)-3-(trifluoromethyl)-4,5,6,7-tetrahydro-1H-indol-4-ol ( compound A6)

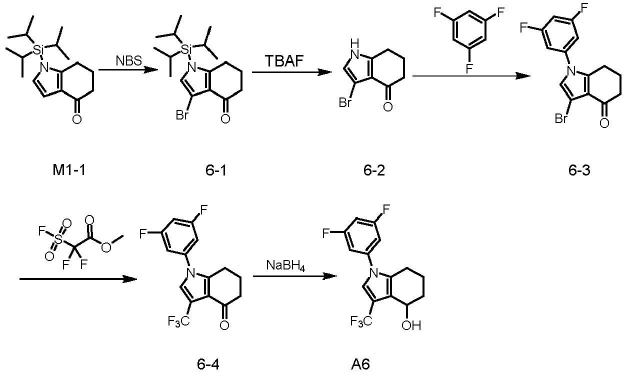

### Step 1: Synthesis of compound 6-1

Using the preparation method similar to intermediate M4, replacing M4-4 with M1-1, the target compound 6-1 was prepared.

### Step 2: Synthesis of compound 6-2

TBAF (21 mL, 1 M in THF) was added into the solution of compound 6-1 (2.6 g) in THF (30 mL) at room temperature, stirred for 1 hour, the reaction solution was concentrated, diluted with water, extracted with EA, and concentrated to obtain crude product, the crude product was purified by silica gel column (EA: PE=70%) to obtain the target compound 6-2 (1.1 g).

### Step 3: Synthesis of compound 6-3

Potassium carbonate (968 mg), 1,3,5-trifluorobenzene (617 mg) were added into a solution of compound 6-2 (500 mg) in DMF (10 mL) at room temperature, then warmed to 100°C and stirred overnight, the reaction mixture was quenched with water, diluted with ethyl acetate, and concentrated to obtain the crude product, which was purified by column chromatography (EA:PE=17%) to obtain the target compound 6-3 (400 mg).

### Step 4: Synthesis of compound 6-4

Compound 6-3 (100 mg), cuprous iodide (111.18 mg), NMP (3 mL) and methyl fluorosulfonyl difluoroacetate (112.15 mg) were added to a 25ml single-neck bottle sequentially, the temperature was raised to 120°C, stirred for 6h, the reaction mixture was diluted with EA, washed three times with water, washed once with saturated brine, dried, and concentrated to obtain the crude product, which was purified by Prep-TLC (DCM:PE=2:1) to obtain the target compound 6-4 (9.8mg). ESI-MS m/z: 332.06[M+H]⁺.

### Step 5: Synthesis of compound A6

Using the preparation method similar to M3, replacing compound M3-1 with compound 6-4, the target compound A6 was prepared. LCMS: [M+H-H₂O]⁺: 300.09.

### Example 38 and example 39 Synthesis of 3-fluoro-5-(3-(methylsulfonyl)-4-oxo-4,5,6,7-tetrahydro-1H-indol-1-yl)benzonitrile ( compound A24) and 3-fluoro-5-(4-hydroxy-3-(methylsulfonyl)-4,5,6,7-tetrahydro-1H-indol-1-yl)benzonitrile ( compound A8)

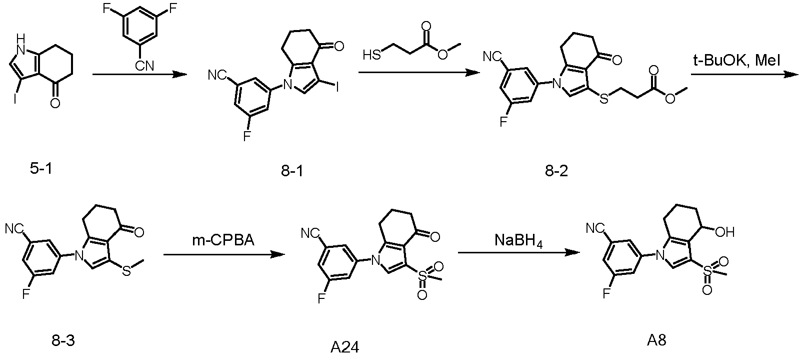

### Step 1: Synthesis of compound 8-1

Otassium carbonate (1000 mg) and 3,5-difluorobenzonitrile (700 mg) were added to a solution of compound 5-1 (500 mg) in DMF (10 mL) at room temperature, then warmed to 100°C and stirred overnight, the reaction mixture was quenched with water, diluted with ethyl acetate, and concentrated to obtain the crude product, which was purified by column chromatography (EA:PE=17%) to obtain the target compound 8-1 (350 mg).

### Step 2: Synthesis of compound 8-2

Compound 8-1 (380 mg), methyl 3-sulfanyl propionate (240 mg), Xantphos (116 mg) and Pd₂(dba)₃ (89 mg) were added to toluene (10 mL), stirred at 70°C overnight, concentrated to obtain a residue, the residue was purified by column chromatography to obtain the target compound 8-2 (232 mg).

### Step 3: Synthesis of compound 8-3

Potassium tert-butoxide (43 mg) was added to a solution of compound 8-2 (120 mg) in THF (10 mL) at -78°C, stirred until the starting material disappeared, and iodomethane (92 mg) was added, stirred for 5 hours, the reaction was quenched with saturated ammonium chloride, extracted with ethyl acetate, and concentrated to obtain the crude product, which was purified by column chromatography to obtain the target compound 8-3 (71 mg).

### Step 4: Synthesis of compound A24

*m*-CPBA (223 mg) was added to a solution of compound 8-3 (130 mg) in dichloromethane (10 mL) at room temperature, stirred at room temperature for 10 hours, the reaction mixture was extracted with aqueous sodium thiosulfate solution and diluted with dichloromethane, washed with aqueous sodium bicarbonate solution, and saturated brine, dried, and concentrated to obtain the crude product, which was purified by column chromatography to obtain the target compound A24 (71 mg). ESI-MS m/z: 333.20 [M+H]⁺.

### Step 5: Synthesis of compound A8

Using the preparation method similar to compound A2, replacing compound A13 with compound 8-4, the target compound 8 was prepared. ESI-MS m/z: 317.11[M+H-H₂O]⁺.

### Example 40 Synthesis of 1-(3,5-difluorophenyl)-4-hydroxy-4,5,6,7-tetrahydro-1H-indole-3-carbonitrile ( compound A25)

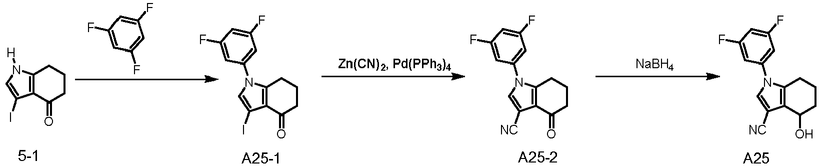

### Step 1: Synthesis of compound A25-1

Potassium carbonate (900 mg), 1,3,5-trifluorobenzene (600 mg) were added to a solution of compound 5-1 (450 mg) in DMF (10 mL) at room temperature, then warmed to 100°C and stirred overnight, the reaction mixture was quenched with water, diluted with ethyl acetate, and concentrated to obtain the crude product, which was purified by column chromatography (EA:PE=17%) to obtain the target compound A25-1 (400 mg).

### Step 2: Synthesis of compound A25-2

Compound A25-1 (200 mg) was dissolved in DMF (5 mL), Zn(CN)₂ (117 mg) and Pd(PPh₃)₄ (58 mg) were added under nitrogen protection, the reaction mixture was stirred at 120 °C for 2 hours, extracted with ethyl acetate, the organic layer was washed with brine, dried over Na₂SO₄, and concentrated to obtain the crude product, which was purified by column chromatography (EA/PE=1:3) to obtain the target compound A25-2 (120 mg).

### Step 3: Synthesis of compound A25

Compound A25-2 (100 mg) was dissolved in a mixed solvent of THF (2 mL) and H₂O (1 mL), NaBH₄ (18 mg) was added in an ice bath, stirred at 0°C for 1 hour. The reaction solution was added ice water, and extracted with ethyl acetate, the organic layer was washed with brine, dried over Na₂SO₄, concentrated to obtain the crude product, which was purified by Prep-TLC (EA/PE=1:3) to obtain the target compound A25 (15 mg). ESI-MS m/z: 257.10 [M+H-H₂O]⁺.

Using the method substantially similar to example 1 - example 40 to obtain the following compounds.

| **Example number** | **Structure** | **Chemical name** | **ESI-MS** |
|---|---|---|---|
| 41 | 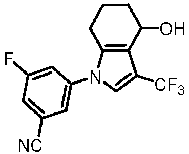 | 3-fluoro-5-(4-hydroxy-3-(trifluoromethyl)-4,5,6,7-tetrahydro-1H-indol-1-yl)benzonitrile | [M+H⁻H₂O]⁺: 307.12 |
| 42 | 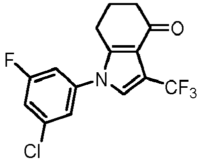 | 1-(3-chloro-5-fluorophenyl)-3-(trifluoromethyl)-1,5,6,7-tetrahydro-4H-indol-4-one | [M+H]⁺: 332.06 |
| 43 | 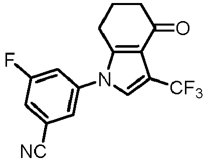 | 3-fluoro-5-(4-oxo-3-(trifluoromethyl)-4,5,6,7-tetrahydro-1H-indol-1-yl)benzonitrile | [M+H]⁺: 323.12 |
| 44 | 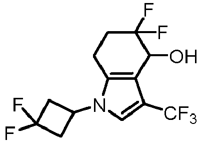 | 1-(3,3-difluorocyclobutyl)-5,5-difluoro-3-(trifluoromethyl)-4,5,6,7-tetrahydro-1H-indol-4-ol | [M+H-H₂O]⁺ : 314.08 |
| 45 | 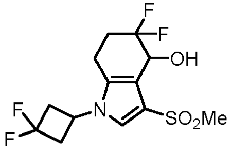 | 1-(3,3-difluorocyclobutyl)-5,5-difluoro-3-(methylsulfonyl)-4,5,6,7-tetrahydro-1H-indol-4-ol | [M+H-H₂O]⁺ : 324.08 |
| 46 | 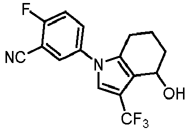 | 2-fluoro-5-(4-hydroxy-3-(trifluoromethyl)-4,5 ,6,7-tetrahydro-1H-indol-1-yl)benzonitrile | [M+H-H₂O]⁺: 307.10 |

### Example 47 Synthesis of 1-(3,5-difluorophenyl)-3-(1-methyl-1H-pyrazol-5-yl)-4,5,6,7-tetrahydro-1H-indol-4-ol ( compound A7)

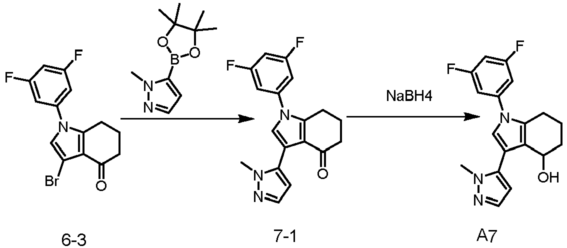

### Step 1: Synthesis of compound 7-1

Pd(dppf)Cl₂ (12.52 mg), K₂CO₃ (42.38 mg), 1-methyl-5-(4,4,5,5-tetramethyl-1,3,2-dioxaboran-2-yl)pyrazole (38.28 mg) were added to a mixed solution of compound 6-3 (50 mg) in dioxane (2 mL)/H₂O (0.5 mL) at room temperature, the atmosphere was replaced with nitrogen three times, and microwaved at 100 °C for 1h, the reaction mixture was diluted with water, extracted with ethyl acetate, dried, and concentrated to obtain the target compound 7-1 (40 mg).

### Step 2: Synthesis of compound A7

Using the preparation method similar to compound A2, replacing compound A13 with compound 7-1, the target compound A7 was prepared. ESI-MS m/z: 312.14[M+H]⁺.

### Example 48 Synthesis of (S)-1-(3-chloro-5-fluorophenyl)-5,5-difluoro-3-(thiophen-2-yl)-4,5,6,7-tetrahydro-1H-indol-4-ol ( compound A65)

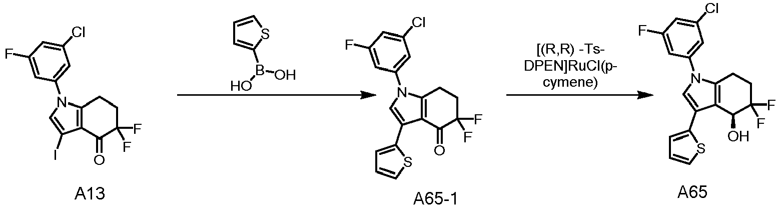

### Step 1: Synthesis of compound A65-1

Compound A13 (100 mg), 2-thiopheneboronic acid (36 mg), Pd(PPh₃)₄ (30 mg), potassium carbonate (97 mg) were added into dioxane (5 mL) and water (1 mL), the reaction was carried out at 90 °C overnight under N₂ protection, and then diluted with ethyl acetate, washed with saturated brine, dried over sodium sulfate, concentrated, and prepared by Prep-TLC to obtain the target product A65-1 (60 mg). ESI-MS m/z: [M+H]⁺: 382.05.

### Step 2: Synthesis of compound A65

Compound A65-1 (60 mg) was dissolved in dichloromethane (2 mL), cooled in an ice-water bath, [(R,R)-Ts-DPEN]RuCl(p-cymene) (10 mg), TEA (32 mg) ) and formic acid (23 mg) were added, stirred at room temperature overnight, the reaction mixture was diluted with dichloromethane, washed with saturated brine, the organic phase was spin-dried, and purified by Prep-TLC to obtain the target product A65 (23 mg). ESI-MS m/z: [M+H-H₂O]⁺: 366.11. ¹H NMR (500 MHz, DMSO-*d*₆) δ 7.53 - 7.45 (m, 3H), 7.43 - 7.36 (m, 2H), 7.33 (d, *J* = 3.5 Hz, 1H), 7.08 (dd, *J* = 5.1, 3.5 Hz, 1H), 6.01 (d, *J* = 6.5 Hz, 1H), 5.75 (s, 1H), 4.60 (d, *J* = 6.2 Hz, 1H), 2.90 (ddd, *J* = 17.0, 11.3, 6.3 Hz, 1H), 2.80 (dd, *J* = 16.2, 6.4 Hz, 1H), 2.19 - 2.11 (m, 1H).

### Example 49 Synthesis of 1-(4-fluorobenzyl)-3-(1-methyl-1H-pyrazol-5-yl)-4,5,6,7-tetrahydro-1H-indol-4-ol ( compound A79)

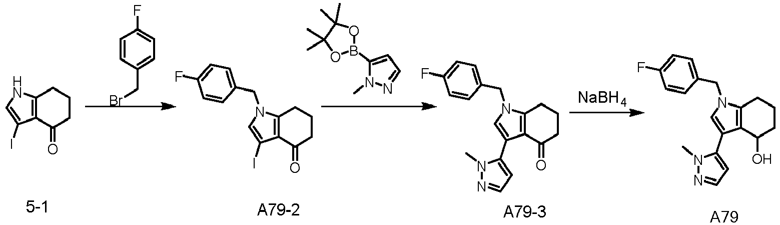

### Step 1: Synthesis of compound A79-1

Compound M1-2 (19 g), THF (150 mL) and TBAF (13.09 g) were added to a 500 ml single-necked flask sequentially, the reaction was carried out at room temperature for 30 min. Concentrated under reduced pressure to get a residue, and PE/EA (V: V=1:1, 100ml) was added to the residue, suction filtration after stirring, and the obtained filter cake was rinsed twice with EA to obtain compound A79-1 (5.2 g).

### Step 2: Synthesis of compound A79-2

Compound A79-1 (300 mg) was dissolved in DMF (10 mL), K₂CO₃ (276 mg) and 4-fluorobenzyl bromide (260 mg) were added, the mixture was stirred at room temperature for two hours. The reaction mixture was poured into water, washed with ethyl acetate, extracted twice with EA, washed twice with saturated brine, dried, and concentrated to obtain the crude product, which was purified by column chromatography (EA/PE=1:7) to obtain the target compound A79-2 (200 mg).

### Step 3: Synthesis of compound A79-3

Pd(dppf)Cl₂ (44 mg), K₂CO₃ (112 mg), 1-methyl-5-(4,4,5,5-tetramethyl-1,3,2-dioxaboran-2-yl)pyrazole (112 mg) were added into a mixed solution of compound A79-2 (100 mg) in dioxane (2 mL)/H₂O (0.5 mL), the atmosphere was replaced with nitrogen three times. The reaction was carried out at 100°C for 6 hours, the reaction mixture was diluted with water, extracted with ethyl acetate, dried, concentrated to obtain the target compound A79-3 (40 mg).

### Step 4: Synthesis of compound A79

Using the preparation method similar to compound A2, it was prepared by reduction of sodium borohydride. LCMS: 308.21 [M+H-H₂O]⁺.

Using a method substantially similar to example 1-49, such as replacing 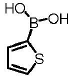 with 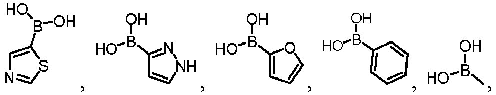 etc., to prepare the following example.

| **Example number** | **Structure** | **Chemical name** | **ESI-MS** |
|---|---|---|---|
| 50 | 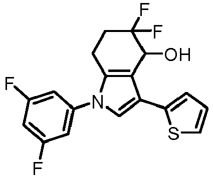 | 1-(3,5-difluorophenyl)-5,5-difluoro-3-(thiophen-2-yl)-4,5,6,7-tetrahydro-1H-indol-4-ol | [M+H-H₂O]⁺: 350.10 |
| 51 | 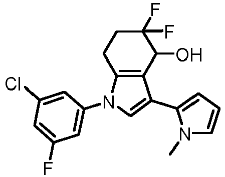 | 1-(3-chloro-5-fluorophenyl)-5,5-difluoro-3-(1-methyl-1H-pyrrol-2-yl)-4,5,6,7-tetrahydro-1H-indol-4-ol | [M+H]⁺ : 381.10 |
| 52 | 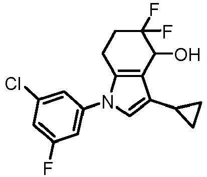 | 1-(3-chloro-5-fluorophenyl)-3-cyclopropyl-5,5-difluoro-4,5,6,7-tetrahydro-1H-indol-4-ol | [M+H-H₂O]⁺ : 324.10 |
| 53 | 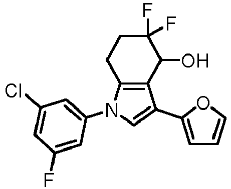 | 1-(3-chloro-5-fluorophenyl)-5,5-difluoro-3-(furan-2-yl)-4,5,6,7-tetrahydro-1H-indol-4-ol | [M+H-H₂O]⁺: 350.18 |
| 54 | 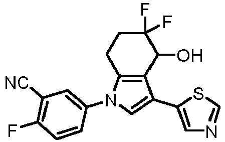 | 5-(5,5-difluoro-4-hydroxy-3 - (thiazol-5-yl)-4,5,6,7-tetrahydro-1H-indol-1-yl)-2-fluorobenzonitrile | [M+H]⁺: 376.30 |
| 55 | 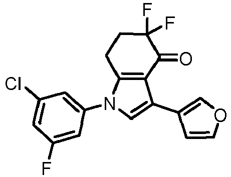 | 1-(3-chloro-5-fluorophenyl)-5,5-difluoro-3-(furan-3-yl)-1,5,6,7-tetrahydro-4H-indol-4-one | [M+H]⁺ : 366.17 |
| 56 | 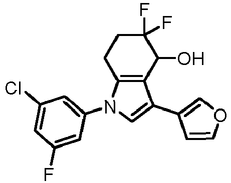 | 1-(3-chloro-5-fluorophenyl)-5,5-difluoro-3-(furan-3-yl)-4,5,6,7-tetrahydro-1H-indol-4-ol | [M+H-H₂O]⁺: 350.18 |
| 57 | 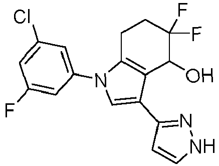 | 1-(3-chloro-5-fluorophenyl)-5,5-difluoro-3-(1H-pyrazol-3-yl)-4,5,6,7-tetrahydro-1H-indol-4-ol | [M+H-H₂O]⁺: 350.11 |
| 58 | 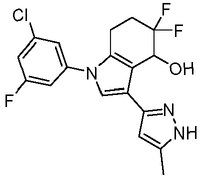 | 1-(3-chloro-5-fluorophenyl)-5,5-difluoro-3-(5-methyl-1H-pyrazol-3-yl)-4,5,6,7-tetrahydro-1H-indol-4-ol | [M+H-H₂O]⁺: 365.24 |
| 59 | 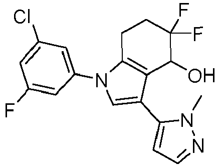 | 1-(3-chloro-5-fluorophenyl)-5,5-difluoro-3-(1-methyl-1H-pyrazol-5-yl)-4,5,6,7-tetrahydro-1H-indol-4-ol | [M+H]⁺: 382.16 |
| 60 | 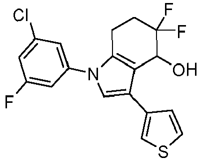 | 1-(3-chloro-5-fluorophenyl)-5 ,5-difluoro-3-(thiophen-3-yl)-4,5,6,7-tetrahydro-1H-indol-4-ol | [M+H-H₂O]⁺: 366.08 |
| 61 | 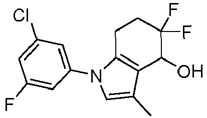 | 1-(3-chloro-5-fluorophenyl)-5,5-difluoro-3-methyl-4,5,6,7-tetrahydro-1H-indol-4-ol | [M+H-H₂O]⁺: 298.13 |
| 62 | 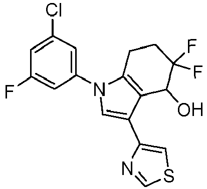 | 1-(3-chloro-3-fluorophenyl)-5,5-difluoro-3-(thiazol-4-yl)-4,5,6,7-tetrahydro-1H-indol-4-ol | [M+H-H₂O]⁺: 367.00 |
| 63 | 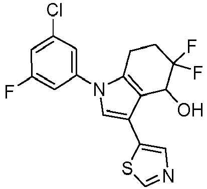 | 1-(3-chloro-5-fluorophenyl)-5,5-difluoro-3-(thiazol-5-yl)-4,5,6,7-tetrahydro-1H-indol-4-ol | [M+H-H₂O]⁺: 367.00 |

### Example 64 Synthesis of (S)-1-(3,5-difluorophenyl)-5,5-difluoro-3-(trifluoromethyl)-4,5,6,7-tetrahydro-1H-indol-4-ol ( compound A9)

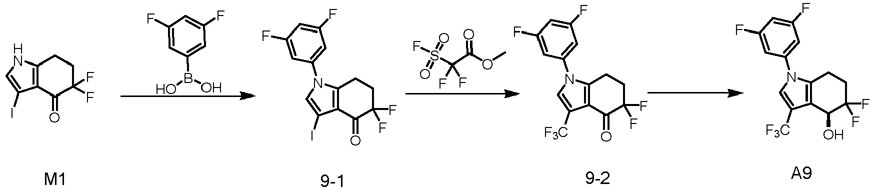

### Step 1: Synthesis of compound 9-1

Compound M1 (300 mg) was dissolved in DCM (20 mL), copper acetate (90 mg), 3,5-difluorophenylboronic acid (350 mg) and TEA (404 mg) were added, and the mixture was stirred at room temperature overnight. The reaction mixture was diluted with dichloromethane, washed twice with saturated brine, dried, concentrated to obtain the crude product, which was purified by column chromatography (EA/PE=1:5) to obtain the target compound 9-1 (230 mg). ESI-MS m/z: 409.11 [M+H]⁺.

### Step 2: Synthesis of compound 9-2

Compound 9-1 (230 mg) was dissolved in DMF (5 mL) under nitrogen protection, Pd₂(dba)₃ (165 mg), CuI (114 mg) and methyl fluorosulfonyldifluoroacetate (230 mg) were added, stirred at 100 °C for 3 hours, extracted with ethyl acetate, the organic layer was washed with brine, dried over Na₂SO₄, concentrated to obtain the crude product, the crude product was purified by column chromatography (EA/PE=1:5) to obtain the target compound 9-2 (150 mg). ESI-MS m/z: 352.20 [M+H]⁺.

### Step 3: Synthesis of compound A9

Compound 9-2 (150 mg) was dissolved in dichloromethane (5 mL), formic acid (0.03 mL) and triethylamine (1.05 mL) were added, the reaction mixture was sparged with nitrogen, [(R,R)-Ts-DPEN]RuCl(p-cymene)] (12 mg) was added, the reaction mixture was stirred at room temperature under nitrogen protection overnight, concentrated, and the crude product was purified by Prep-TLC (EA/PE=1:5) to obtain the target compound A9 (100 mg). LC-MS: 336.20[M+H-H₂O]⁺.

### Example 65 Synthesis of 1-(3-chloro-5-fluorophenyl)-3-(difluoromethyl)-5,5-difluoro-4,5,6,7-tetrahydro-1H-indol-4-ol ( compound A29)

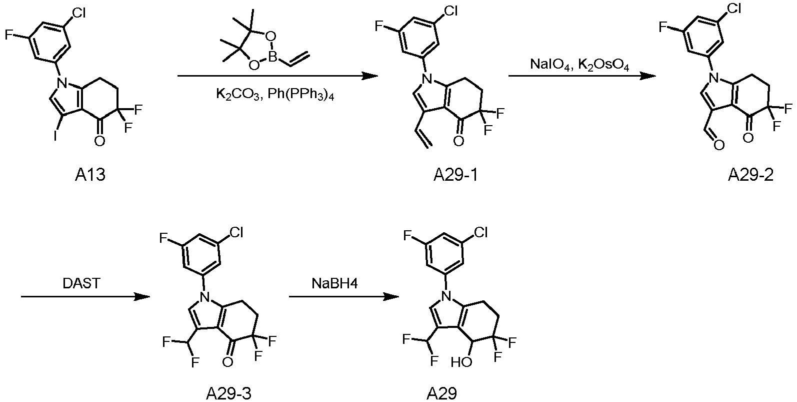

### Step 1: Synthesis of compound A29-1

Compound A13 (400 mg) was dissolved in a mixed solvent of dioxane (5 mL) and H₂O (5 mL) under nitrogen protection, Pd(PPh₃)₄ (109 mg), K₂CO₃ (390 mg) and vinyl borate pinacol ester (217 mg), stirred at 90 °C overnight, extracted with ethyl acetate, the organic layer was washed with brine, dried over Na₂SO₄, and concentrated to obtain the crude product, which was purified by column chromatography (EA/PE=1 :5) to obtained the target compound A29-1 (210 mg). ESI-MS m/z:
326.03 [M+H]⁺.

### Step 2: Synthesis of compound A29-2

Compound A29-1 (210 mg) was dissolved in a mixed solvent of THF (5 mL) and H₂O (5 mL), NaIO₄ (414 mg) and potassium osmate (20 mg) were added, stirred at room temperature for 2 hours, extracted with ethyl acetate, the organic layer was washed with brine, dried over Na₂SO₄, and concentrated to obtain the crude product, which was purified by Prep-TLC (EA/PE=1:4) to obtain the target compound A29-2 (45 mg). ESI-MS m/z: 328.10[M+H]⁺.

### Step 3: Synthesis of compound A29-3

Compound A29-2 (80 mg) was dissolved in dichloromethane (4 mL) under nitrogen protection, and DAST (98 mg) was added in an ice bath, after the dropwise addition, the mixture was warmed to room temperature and stirred overnight. The reaction mixture was quenched with saturated sodium bicarbonate, extracted with ethyl acetate, the organic layer was washed with brine, dried over Na₂SO₄, and concentrated to obtain the crude product, which was purified by Prep-TLC (EA/PE=1:4) to obtain the target compound A29-3 (45 mg). ESI-MS m/z: 350.05[M+H]⁺.

### Step 4: Synthesis of compound A29

Compound A29-3 (45 mg) was dissolved in a mixed solvent of THF (5 mL) and H₂O (5 mL), NaBH₄ (20 mg) was added in an ice bath, the reaction was carried out at 90°C for 0.5 hour, extracted with ethyl acetate, the organic layer was washed with brine, dried over Na₂SO₄, and concentrated to obtain the crude product, which was purified by Prep-TLC (EA/PE=1:3) to obtain the target compound A29 (30 mg). ESI-MS m/z: 334.04[M+H-H₂O]⁺.

### Example 66 Synthesis of 1-(3-chloro-5-fluorophenyl)-5,5-difluoro-3-vinyl-4,5,6,7-tetrahydro-1H-indol-4-ol ( compound D12)

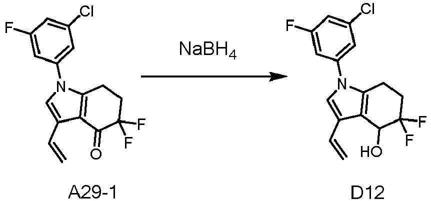

Compound D12 was obtained by reduction of A29-1 with sodium borohydride using a preparation method similar to that of compound A2. ESI-MS m/z: [M+H-H₂O]⁺: 310.17.

### Example 67 Synthesis of 1-(3-chloro-5-fluorophenyl)-5,5-difluoro-3-(hydroxymethyl)-4.5.6.7-tetrahydro-1H-indol-4-ol ( compound D20)

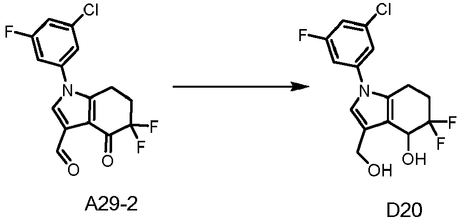

Compound D20 was obtained by reduction of A29-2 with sodium borohydride using a similar preparation method to compound A2. ESI-MS m/z: [M+H-H₂O]⁺: 314.00.

### Example 68 Synthesis of 3-(3-chloro-5-fluorophenyl)-7-fluoro-1-(trifluoromethyl)-5,6,7,8-tetrahydroindolizin-8-ol ( compound D70)

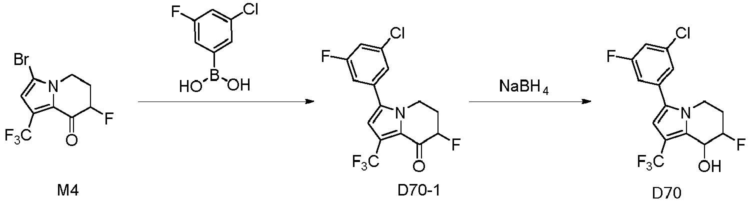

### Step 1: Synthesis of compound D70-1

Compound M4 (300 mg) and (3-chloro-5-fluorophenyl)boronic acid (260 mg) were dissolved in a mixed solvent of 1,4-dioxane (5 mL) and water (1 mL) under nitrogen protection, Pd(PPh₃)₄ (110 mg) and potassium carbonate (276 mg) were added, the reaction mixture was stirred in an oil bath at 90°C for 0.5 hours, the reaction solution was extracted twice with ethyl acetate, washed twice with saturated brine, dried and concentrate to obtain the crude product, which was purified by column chromatography (EA/PE=1:2) to obtain the target compound D70-1(185 mg). ESI-MS m/z: 350.09[M+H]⁺.

### Step 2: Synthesis of compound D70

Compound D70-1 (120 mg) was dissolved in methanol (4 mL), NaBH₄ (76 mg) was added in an ice bath, after stirred at 0°C for 1 hour, ice water was added, the mixture was extracted with ethyl acetate, and the organic layer was washed with brine, dried over Na₂SO₄, and concentrated to obtain the crude product, which was purified by Prep-TLC (EA/PE=1:2) to obtain the target compound D70 (96 mg). ESI-MS m/z: 334.01[M+H]⁺.

### Example 69 and Example 70 Synthesis of 5-(7,7-difluoro-8-hydroxy-1-(trifluoromethyl)-5,6,7,8-tetrahydroindolizin-3-yl)-2-fluorobenzonitrile ( compound D79) and (S)-S-(7,7-difluoro-8-hydroxy-1-(trifluoromethyl)-5,6,7,8-tetrahydroindolizin-3-yl)-2-fluorobenzonitrile ( compound D76)

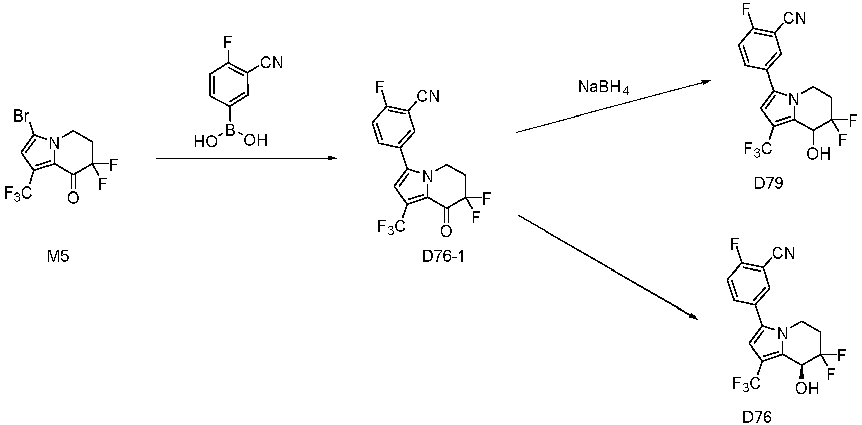

### Step 1: Synthesis of compound D76-1

Compound M5 (317 mg) and (3-cyano-4-fluorophenyl)boronic acid (248 mg) were dissolved in a mixed solvent of 1,4-dioxane (5 mL) and water (1 mL) under nitrogen protection, Pd(PPh₃)₄ (110 mg) and potassium carbonate (276 mg) were added, the reaction mixture was stirred in an oil bath at 90°C for 0.5 h, extracted twice with ethyl acetate, washed twice with saturated brine, dried and concentrated to obtain the crude product, which was purified by column chromatography (EA/PE=1:2) to obtain the target compound D76-1 (215 mg). ESI-MS m/z: 358.45[M+H]⁺.

### Step 2: Synthesis of compound D79

Compound D76-1 (100 mg) was dissolved in methanol (4 mL), NaBH₄ (38 mg) was added in an ice bath, after stirring at 0°C for 1 hour, ice water was added, the reaction mixture was extracted with ethyl acetate, the organic layer was washed with brine, dried over Na₂SO₄, concentrated to give the crude product, which was purified by Prep-TLC (EA/PE=1:3) to give compound D79 (72 mg). ESI-MS m/z: 343.11[M+H-H₂O]⁺.

### Step 3: Synthesis of compound D76

Compound D76-1 (100 mg) was dissolved in dichloromethane (5 mL), formic acid (0.03 mL) and triethylamine (1.05 mL) were added, the reaction mixture was sparged with nitrogen, [(R,R)-Ts-DPEN]RuCl(p-cymene) (12 mg) was stirred at room temperature under nitrogen protection overnight, concentrated, the crude product was purified by Prep-TLC (EA/PE=1:3) to obtain the target compound D76 (68 mg). LC-MS m/z:343.09[M+H-H₂O]⁺.

Using a method substantially similar to example 1-example 70, such as replacing 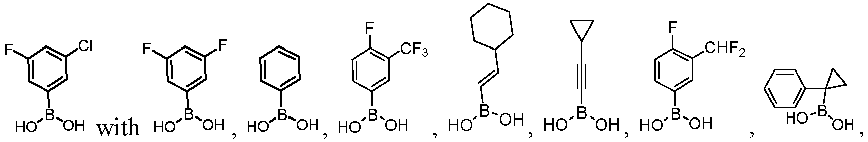 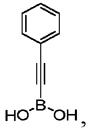 etc., to prepare the following example.

| **Example number** | **Structure** | **Chemical name** | **ESI-MS** |
|---|---|---|---|
| 71 | 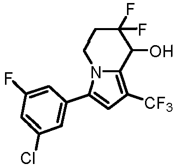 | 3-(3-chloro-5-fluorophenyl)-7,7-difluoro-1-(trifhioromethyl)-5,6,7,8-tetrahydroindolizin-8-ol | [M+H-H₂O]⁺ : 352.03 |
| 72 | 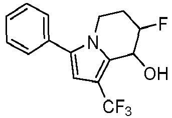 | 7-fluoro-3-phenyl-1-(trifluoromethyl)-5,6,7,8-tetrahydroindolizin-8-ol | [M+H-H₂O]⁺ : 282.04 |
| 73 | 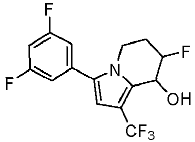 | 3-(3,5-difluorophenyl)-7-fluoro-1 - (trifluoromethyl)-5,6,7,8-tetrahydroindolizin-8-ol | [M+H-H₂O]⁺ : 318.08 |
| 74 | 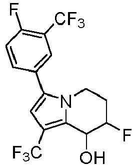 | 7-fluoro-3-(4-fluoro-3-(trifluoromethyl)phenyl)-1 - (trifluoromethyl)-5,6,7,8-tetrahydroindolizin-8-ol | [M+H-H₂O]⁺ : 368.23 |
| 75 | 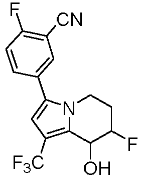 | 2-fluoro-5-(7-fluoro-8-hydroxy-1-(trifluoromethyl)-5,6,7,8-tetrahydroindolizin-3-yl)benzonitrile | [M+H-H₂O]⁺ : 325.00 |
| 76 | 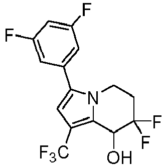 | 3-(3,5-difluorophenyl)-7,7-difluoro-1-(trifluoromethyl)-5,6,7,8-tetrahydroindolizin-8-ol | [M+H-H₂O]⁺ : 335.97 |
| 77 | 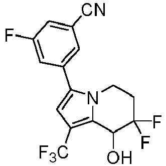 | 3-(7,7-difluoro-8-hydroxy-1-(trifluoromethyl)-5,6,7,8-tetrahydroindolizin-3-yl)-5-fluorobenzonitrile | [M+H-H₂O]⁺ : 343.13 |
| 78 | 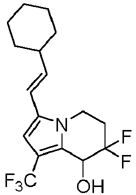 | (E)-3-(2-cyclohexylvinyl)-7,7-difluoro-1-(trifluoromethyl)-5,6,7,8-tetrahydroindolizin-8-ol | [M+H-H₂O]⁺ : 332.16 |
| 79 | 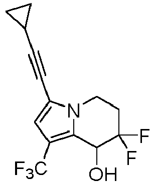 | 3-(cyclopropylethynyl)-7,7-difluoro-1-(trifluoromethyl)-5,6,7,8-tetrahydroindolizin-8-ol | [M+H-H₂O]⁺ : 228.08 |
| 80 | 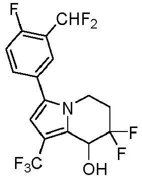 | 3-(3-(difluoromethyl)-4-fluorophenyl)-7,7-difluoro-1-(trifluoromethyl)-5,6,7,8-tetrahydroindolizin-8-ol | [M+H-H₂O]⁺ : 368.16 |
| 81 | 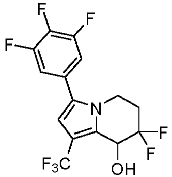 | 7,7-difluoro-1-(trifluoromethyl)-3-(3,4,5-trifluorophenyl)-5,6,7,8-tetrahydroindolizin-8-ol | [M+H-H₂O]⁺ : 354.03 |
| 82 | 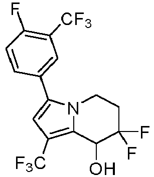 | 7,7-difluoro-3-(4-fluoro-3-(trifluoromethyl)phenyl)-1 - (trifluoromethyl)-5,6,7,8-tetrahydroindolizin-8-ol | [M+H-H₂O]⁺ : 386.15 |
| 83 | 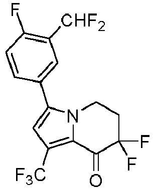 | 3-(3-(difluoromethyl)-4-fluorophenyl)-7,7-difluoro-1-(trifluoromethyl)-6,7-dihydroindolizin-8(5H)-one | [M+H-H₂O]⁺ : 383.02 |
| 84 | 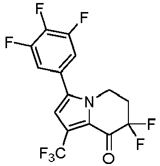 | 7,7-difluoro-1-(trifluoromethyl)-3-(3,4,5-trifluorophenyl)-6,7-dihydroindolizin-8(5H)-one | [M+H]⁺ : 370.05 |
| 85 | 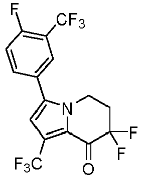 | 7,7-difluoro-3-(4-fluoro-3-(trifluoromethyl)phenyl)-1 - (trifluoromethyl)-6,7-dihydroindolizin-8(5H)-one | [M+H]⁺ : 402.06 |
| 86 | 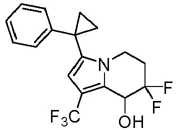 | 7,7-difluoro-3-(1-phenylcyclopropyl)-1-(trifluoromethyl)-5,6,7,8-tetrahydroindolizin-8-ol | [M+H-H₂O]⁺ : 358.14 |
| 87 | 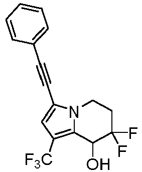 | 7,7-difluoro-3-(phenylethynyl)-1-(trifluoromethyl)-5,6,7,8-tetrahydroindolizin-8-ol | [M+H-H₂O]⁺ : 324.13 |

### Example 88 Synthesis of 1-(3,5-difluorophenyl)-5,5-difluoro-3-(trifluoromethyl)-4,5,6,7-tetrahydro-1H-indazol-4-ol ( compound D80)

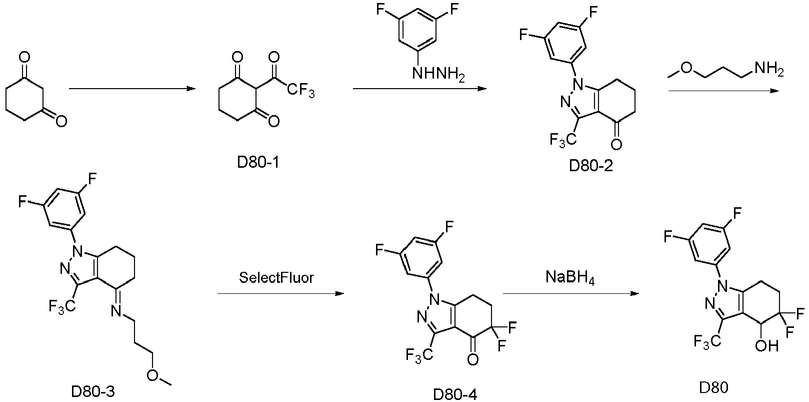

### Step 1: Synthesis of compound D80-1

Carbonyldiimidazole (32.4 g) was dissolved in 100 mL of chloroform under nitrogen protection, a solution (50 mL) of trifluoroacetic anhydride (32.4 g) in chloroform was added slowly and dropwise in an ice bath, the temperature was warmed to room temperature, a mixture of cyclohexane-1,3-dione(11.2 g) and imidazole (6.8 g) in chloroform(50 mL) was added, continued stirring for 2 h, the reaction mixture was washed with 1M dilute hydrochloric acid solution and saturated brine respectively, dried over anhydrous sodium sulfate, concentrated to obtain a crude product, and the crude product was purified by column chromatography (EA/PE=1:4) to obtain the target compound D80-1 (13.3 g). ESI-MS m/z:209.15[M+H]⁺.

### Step 2: Synthesis of compound D80-2

Compound D80-1 (2.08 g) was dissolved in ethanol (100 mL) under nitrogen protection, (3,5-difluorophenyl)hydrazine (1.44 g) was added, after refluxed for 3 hours, the reaction solution was concentrated to obtain the crude product, which was purified by column chromatography (EA/PE=1:3) to obtain the target compound D80-2(1.9 g). ESI-MS m/z: 317.45 [M+H]⁺.

### Step 3: Synthesis of compound D80-3

Compound D80-2 (316 mg), 3-methoxypropylamine (534 mg) and pivalic acid (21 mg) were dissolved in a mixture of toluene (20 mL) and cyclohexane (10 mL) under nitrogen protection, refluxed overnight; the reaction solution was spin-dried to obtain the crude target compound D80-3(326 mg), which was directly used in the next step without purification. ESI-MS m/z: 388.41 [M+H]⁺.

### Step 4: Synthesis of compound D80-4

Compound D80-3 (326 mg) was dissolved in acetonitrile (20 mL), SelectFlour (656 mg) was added and the reaction was refluxed for 6 hours, cooled to room temperature, the reaction mixture was treated with 1M dilute hydrochloric acid (20 mL), and stirred at room temperature for 1 hour, concentrated under reduced pressure to obtain a residue, the residue was partitioned between ethyl acetate and water, the organic layer was washed with brine, dried over Na₂SO₄, concentrated to give crude product, which was purified by column chromatography (EA/PE=1:3) to obtain the target compound D80-4 (213 mg). ESI-MS m/z: 353.10 [M+H]⁺.

### Step 5: Synthesis of compound D80

Compound D80-4 (100 mg) was dissolved in methanol (4 mL), NaBH₄ (42 mg) was added in an ice bath, after stirred at 0 °C for 1 hour, ice water was added, the reaction mixture was extracted with ethyl acetate, the organic layer was washed with brine, dried over Na₂SO₄, and concentrated to obtain the crude product, which was purified by Prep-TLC (EA/PE=1:3) to obtain the target compound 80 (81 mg). ESI-MS m/z:337.23[M+H-H₂O]⁺.

Using a method substantially similar to example 88, substituting different aromatic hydrazine and the following example is obtained.

| **Example number** | **Structure** | **Chemical name** | **ESI-MS** |
|---|---|---|---|
| 89 | 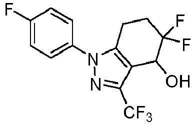 | 5,5-difluoro-1-(4-fluorophenyl)-3-(trifluoromethyl)-4,5,6,7-tetrahydro-1H-indazol-4-ol | [M+H]⁺ : 337.05 |
| 90 | 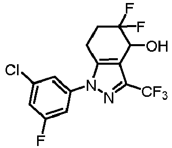 | 1-(3-chloro-5-fluorophenyl)-5,5-difluoro-3-(trifluoromethyl)-4,5,6,7-tetrahydro-1H-indazol-4-ol | [M+H]⁺: 371.03 |
| 91 | 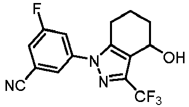 | 3-fluoro-5-(4-hydroxy-3- (trifluoromethyl)-4,5,6,7-tetrahydro-1H-indazol-1-yl)benzonitrile | [M-H]⁺: 322.04 |
| 92 | 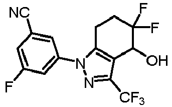 | 3-(5,5-difluoro-4-hydroxy-3-(trifluoromethyl)-4,5,6,7-tetrahydro-1H-indazol-1-yl)-5-fluorobenzonitrile | [M+H]⁺: 361.95 |
| 93 | 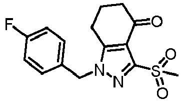 | 1-(4-fluorobenzyl)-3-(methylsulfonyl)-1,5,6,7-tetrahydro-4H-indazol-4-one | [M+H]⁺: 323.18 |

### Example 94 Synthesis of (1-(3-chloro-5-fluorophenyl)-5,5-difluoro-4-hydroxy-4,5,6,7-tetrahydro-1H-indol-3-yl)dimethylphosphine oxide ( compound A50)

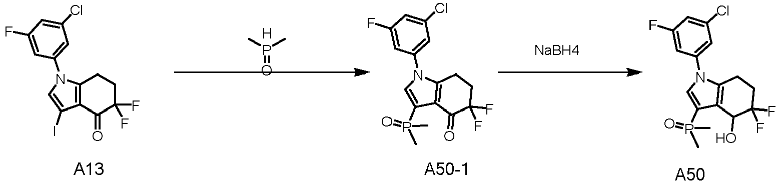

### Step 1: Synthesis of compound A50-1

Compound A13 (864 mg), dimethylphosphine oxide (200 mg), K₃PO₄ (520 mg), Xantphos (118 mg) and palladium acetate (46 mg) were added to DMF (15 mL), and the reaction mixture was stirred at 150 °C overnight under nitrogen protection, after the reaction was completed, the reaction mixture was cooled, added to water, extracted with ethyl acetate, the organic phase was washed with saturated brine, dried over sodium sulfate, concentrated, and purified by column chromatography to obtain the target product A50-1 (300 mg).

### Step 2: Synthesis of compound A50

Using a similar preparation method to compound A2, it was obtained by sodium borohydride reduction reaciton. ESI-MS m/z: 360.05 [M+H-H₂O]⁺. ¹H NMR (500 MHz, DMSO-*d*₆) δ 7.57 - 7.43 (m, 4H), 6.21 (d, *J* = 4.8 Hz, 1H), 4.84 (q, *J* = 7.1 Hz, 1H), 2.87 - 2.73 (m, 2H), 2.34-2.14 (m, 2H), 1.68 (d, *J* = 13.6 Hz, 6H).

### Example 95 Synthesis of (S)-5-(5,5-difluoro-4-hydroxy-3-(trifluoromethyl)-4,5,6,7-tetrahydro-1H-indol-1-yl)-2-fluorobenzonitrile (A61)

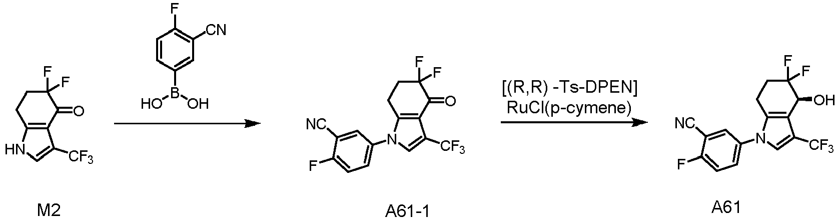

### Step 1: Synthesis of compound A61-1

Compound M2 (214 mg) was dissolved in DCM (10 mL), copper acetate (108 mg), 4-fluoro-3-cyanophenylboronic acid (100 mg), TEA (0.25 mL) were added, the reaction mixture was stirred overnight under oxygen atmosphere, diluted with dichloromethane, washed twice with saturated brine, dried, filtered, and concentrated to obtain the crude product, which was purified by column chromatography (EA/PE=1:3) to obtain the target compound A61-1 (120 mg). ESI-MS m/z: 359.05 [M+H]⁺.

### Step 2: Synthesis of compound A61

Compound A61-1 (120 mg) was dissolved in dichloromethane (3 mL), formic acid (0.04 mL) and triethylamine (0.1 mL) were added, the reaction mixture was sparged with nitrogen, [(R,R)-Ts-DPEN]RuCl(p-cymene) (22 mg) was added, the reaction mixture was stirred at room temperature overnight under nitrogen protection, concentrated, and the crude product was purified by Prep-TLC (EA/PE=1:3) to obtain the target compound A61 (90 mg, ee>96%). ESI-MS m/z: 343.05 [M+H-H₂O]⁺.

### Example 96 Synthesis of 1-(3-chloro-5-fluorophenyl)-5,5-difluoro-4-hydroxy-4,5,6,7-tetrahydro-1H-indole-3-carbonitrile ( compound A73)

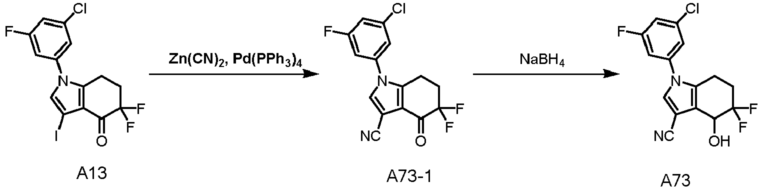

### Step 1: Synthesis of compound A73-1

Compound A13 (212 mg) was dissolved in DMF (5 mL), Zn(CN)₂ (117 mg) and Pd(PPh₃)₄ (58 mg) were added under nitrogen protection, the reaction mixture was stirred at 120 °C for 2 hours, extracted with ethyl acetate, the organic layer was washed with brine, dried over Na₂SO₄, and concentrated to obtain the crude product, which was purified by column chromatography (EA/PE=1:3) to obtain the target compound A73-1 (137 mg). ESI-MS m/z: 325.08 [M+H]⁺.

### Step 2: Synthesis of compound A73

Compound A73-1 (100 mg) was dissolved in a mixed solvent of THF (2 mL) and H₂O (1 mL), NaBH₄ (18 mg) was added in an ice bath, stirred at 0°C for 1 hour. Ice water was added, the reaction mixture was extracted with ethyl acetate, the organic layer was washed with brine, dried over Na₂SO₄, and concentrated to obtain the crude product, which was purified by Prep-TLC (EA/PE=1:3) to obtain the target compound A73 (13 mg). ESI-MS m/z: 309.12 [M+H-H₂O]⁺.

### Example 97 Synthesis of 1-(3-chloro-5-fluorophenyl)-5,6-difluoro-3-(trifluoromethyl)-4 5,6,7-tetrahydro-1H-indol-4-ol (A90)

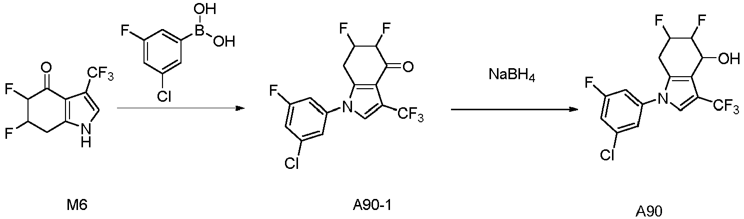

### Step 1: Synthesis of compound A90-1

Compound M6 (214 mg) was dissolved in DCM (10 mL), copper acetate (108 mg), 3-chloro-5-fluorophenylboronic acid (105 mg), TEA (0.25 mL) were added, the reaction mixture was stirred overnight under oxygen atmosphere, diluted with dichloromethane, washed twice with saturated brine, dried, filtered, and concentrated to obtain the crude product, which was purified by column chromatography (EA/PE=1:3) to obtain the target compound A90-1 (83 mg). ESI-MS m/z: 368.07 [M+H]⁺.

### Step 2: Synthesis of compound A90

Compound A90-1 (104 mg) was dissolved in methanol (4 mL), NaBH₄ (42 mg) was added in an ice bath, after stirring at 0°C for 1 hour, ice water was added, the reaction mixture was extracted with ethyl acetate, and the organic layer was washed with brine , dried over Na₂SO₄, and concentrated to obtain the crude product, which was purified by Prep-TLC (EA/PE=1:3) to obtain the target compound A90 (63 mg). ESI-MS m/z: 352.07 [M+H-H₂O]⁺.

### Example 98 Synthesis of 5,5-difluoro-l-(4-fluoro-3-(hydroxymethyl)phenyl)-3-(trifluoromethyl)-4,5,6,7-tetrahydro-1H-indol-4-ol ( compound D37)

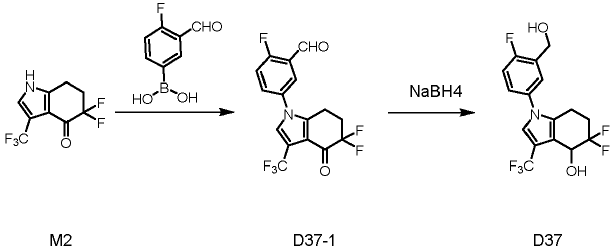

### Step 1: Synthesis of compound D37-1

Compound M2 (2.14 g) was dissolved in DCM (20 mL), copper acetate (1.08 g), 4-fluoro-3-aldolphenylboronic acid (1.0 g), TEA (2.48 mL) were added, the reaction mixture was stirred at room temperature overnight under oxygen atmosphere, diluted with dichloromethane, washed twice with saturated brine, dried, filtered, and concentrated to obtain the crude product, which was purified by column chromatography (EA/PE=1:3) to obtain the target compound D37-1 (1.1 g). ESI-MS m/z: 362.08 [M+H]⁺.

### Step 2: Synthesis of compound D37

Compound D37-1 (350 mg) was dissolved in MeOH (5 mL), sodium borohydride (0.15 g) was added in portions, stirred at room temperature for 6 hours, monitored by LC-MS and TLC, after the reaction was completed, the reaction solution was poured into water, extracted with ethyl acetate, dried and spin-dried, the crude product was purified by column chromatography to give D37 (100 mg). ESI-MS m/z: 348.05 [M+H-H₂O]⁺.

### Example 99 Synthesis of 5,5-difluoro-1-(6-fluoropyridin-2-yl)-3-(trifluoromethyl)-4,5,6,7-tetrahydro-1H-indol-4-ol ( compound D40)

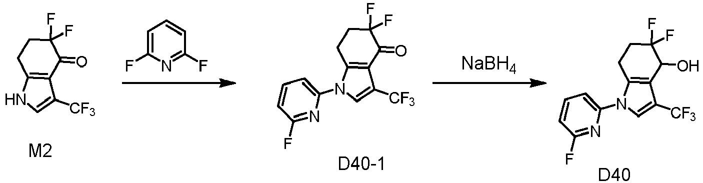

### Step 1: Synthesis of compound D40-1

Potassium carbonate (866 mg) and 2,6-difluoropyridine (481 mg) were added to a solution of compound M2 (500 mg) in DMF (10 mL) at room temperature, then warmed to 100°C and stirred overnight, the reaction mixture was quenched by the addition of water, diluted with ethyl acetate, and concentrated to obtain the crude product, which was purified by column chromatography (EA:PE=17%) to obtain the target compound D40-1 (400 mg). ESI-MS m/z: 335.05 [M+H]⁺.

### Step 2: Synthesis of compound D40

Using a similar preparation method to compound A2, it was obtained by sodium borohydride reduction reaciton. ESI-MS m/z: 319.25 [M+H-H₂O]⁺.

### Example 100 Synthesis of 5-(5,5-difluoro-4-hydroxy-4-methyl-3-(trifluoromethyl)-4,5,6,7-tetrahydro-1H-indol-1-yl)-2-fluorobenzonitrile ( compound D46)

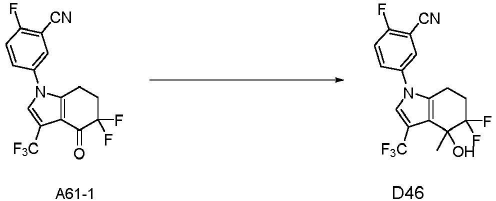

### Step 1: Synthesis of compound D46

Compound A61-1 (36 mg) was dissolved in tetrahydrofuran (5 mL) under nitrogen protection, methylmagnesium chloride (3.0 M, 0.3 mL) was added dropwise in an ice bath, the reaction mixture was stirred at 0 °C for 3 hours, and quenched with 1 M dilute hydrochloric acid, the reaction solution was extracted twice with ethyl acetate, washed twice with saturated brine, dried, and concentrated to obtain the crude product, which was purified by column Pre-TLC (EA/PE=1:3) to obtain the target compound D46 (13 mg). ESI-MS m/z: 357.18[M+H-H₂O]⁺. ¹H NMR (500 MHz, DMSO-*d*₆) δ 8.20-8.17 (m, 1H), 7.94-7.91 (m, 1H), 7.69 (t, *J* = 9.0 Hz, 1H), 7.61 (s, 1H), 2.83 - 2.59 (m, 2H), 2.45 - 2.16 (m, 2H), 1.53 (s, 3H).

### Example 101 Synthesis of 5-(5,5-difluoro-4-hydroxy-6,6-dimethyl-3-(trifluoromethyl)-4,5,6,7-tetrahydro-1H-indol-1-yl)-2-fluorobenzonitrile ( compound D49)

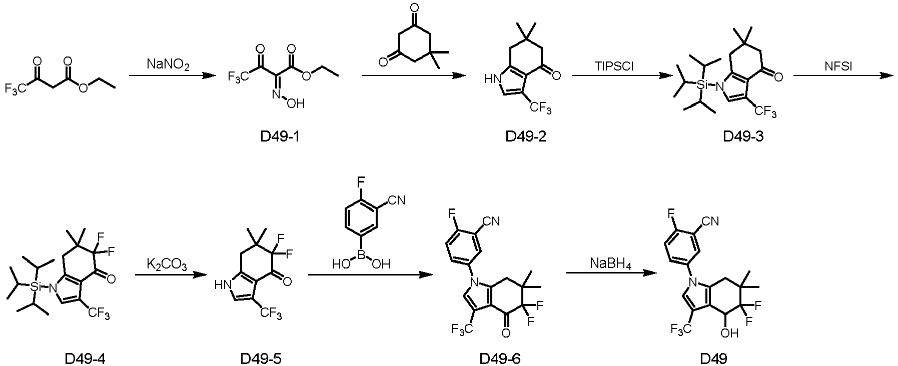

### Step 1: Synthesis of compound D49-1

Ethyl trifluoroacetoacetate (25.02 g) was dissolved in glacial acetic acid (38 mL) in an ice bath, an aqueous solution (25 mL) of sodium nitrite (9.37 g) was added in portions, and then the reaction was stirred at 20°C for 2 hours, concentrated under reduced pressure, water and ethyl acetate were added, the layers were separated, the organic layer was washed with brine, dried over Na₂SO₄, and concentrated to obtain the target compound D49-1 (22 g). ESI-MS m/z: 212.03 [M-H]⁻.

### Step 2: Synthesis of compound D49-2

5,5-dimethyl-1,3-cyclohexanedione (6.0 g) was dissolved in glacial acetic acid (30 mL), heated to 60 °C, an aqueous solution(20 mL) of compound D49-1 (9.12 g) was added, zinc powder (5.59 g) was added in batches, heated to 90 °C and stirred for 16 hours after the addition, water and ethyl acetate were added to the reaction system, separated, and the aqueous phase was extracted twice with ethyl acetate, the combined organic phases were washed twice with saturated aqueous sodium bicarbonate solution, dried over Na₂SO₄, and concentrated to obtain the crude product, which was purified by column chromatography (EA/PE=1:3) to obtain the target compound D49-2 (2.2 g, 22.2%). ESI-MS m/z: 232.10[M+H]⁺.

### Step 3: Synthesis of compound D49-3

Compound D49-2 (2.2 g) was dissolved in tetrahydrofuran (30 mL) under nitrogen protection, sodium hydride (0.457 g) was added in portions in an ice bath, the reaction was carried out at 0 °C for 0.5 h, and then a solution of triisopropylchlorosilane (2.75 g) in tetrahydrofuran (5 mL) was added, and the stirring was continued for 1 hour. The reaction solution was poured into ice water to quench, extracted with ethyl acetate, washed with water, dried over Na₂SO₄, and concentrated to obtain the crude product, which was purified by column chromatography (EA/PE=1:3) to obtain the target compound D49-3 (2.93 g, 79.5%). ESI-MS m/z: 388.10[M+H]⁺.

### Step 4: Synthesis of compound D49-4

Compound D49-3 (1.05 g) was dissolved in tetrahydrofuran (20 mL) under nitrogen protection, cooled to -78 °C, and LiHMDS in tetrahydrofuran solution (1.0 M, 7.33 mL) was added slowly and dropwise, after stirring for half an hour, a solution of NFSI (1.79 g) in tetrahydrofuran (10 mL) was added dropwise, and kept stirring at - 78 °C for two hours. The reaction mixture was quenched by the addition of saturated aqueous ammonium chloride solution, extracted twice with ethyl acetate, and washed twice with saturated brine, dried and concentrated to obtain the crude product, which was purified by column chromatography (DCM = 100%) to obtain the target compound D49-4 (0.73 g, 55.2%). ESI-MS m/z: 424.05[M+H]⁺.

### Step 5: Synthesis of compound D49-5

The crude compound D49-4 (0.73 g) was dissolved in a mixed solvent of THF (20 mL)/water (20 mL), K₂CO₃ (0.48 g) was added at room temperature, and stirring was continued for three hours. The reaction mixture was extracted with ethyl acetate, the organic layer was washed with brine, dried over Na₂SO₄, and concentrated to obtain the crude product, which was purified by column chromatography (DCM = 100%) to obtain the target compound D49-5 (402 mg). ESI-MS m/z: 268.12[M+H]⁺.

### Step 6: Synthesis of compound D49-6

Compound D49-5 (402 mg) was dissolved in DCM (20 mL), copper acetate (275 mg), 3,5-difluorophenylboronic acid (494 mg), TEA compound (304 mg) were added, the reaction mixture was stirred at room temperature overnight, diluted with dichloromethane, washed twice with saturated brine, dried, filtered, and concentrated to obtain the crude product, which was purified by column chromatography (EA/PE=1:3) to obtain the target compound D49-6 (512 mg). ESI-MS m/z: 387.08[M+H]⁺.

### Step 7: Synthesis of compound D49

Compound D49-6 (50 mg) was dissolved in methanol (4 mL), NaBH₄ (15 mg) was added in an ice bath, after stirring at 0°C for 1 hour, ice water was added, the reaction mixture was extracted with ethyl acetate, and the organic layer was washed with brine, dried over Na₂SO₄, concentrated to obtain the crude product, which was purified by Prep-TLC (EA/PE=1:4) to obtain the target compound D49 (37 mg, 73.7%). ESI-MS m/z: 371.10[M+H-H₂O]⁺.

### Example 102 Synthesis of 2-fluoro-5-(5-fluoro-4-hydroxy-6,6-dimethyl-3-(trifluoromethyl)-4,5,6,7-tetrahydro-1H-indol-1-yl)benzonitrile ( compound D50)

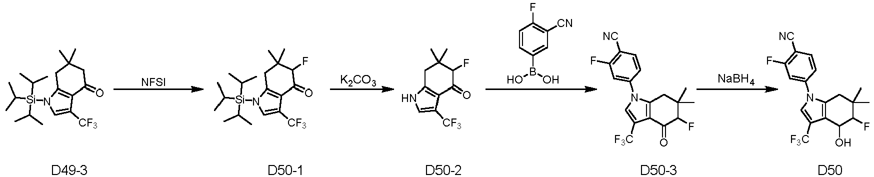

### Step 1: Synthesis of compound D50-1

Compound D49-3 (1.0 g) was dissolved in tetrahydrofuran (20 mL) under nitrogen protection, cooled to -78 °C, LDA in tetrahydrofuran solution (1.0 M, 3.9 mL) was added slowly and dropwise, after stirred for half an hour, a solution of NFSI (895 mg) in THF(10 mL) was added dropwise, and kept stirring at -78 °C for two hours. The reaction was quenched by the addition of saturated aqueous ammonium chloride, extracted twice with ethyl acetate, washed twice with saturated brine, dried and concentrated to obtain the crude product, which was purified by column chromatography (DCM = 100%) to obtain the target compound D50-1 (550 mg). ESI-MS m/z: 406.12[M+H]⁺.

### Step 2: Synthesis of compound D50-2

The crude compound D50-1 (550 mg) was dissolved in a mixed solvent of THF (10 mL)/water (10 mL), K₂CO₃ (375 mg) was added at room temperature, and stirring was continued for three hours. The reaction mixture was extracted with ethyl acetate, the organic layer was washed with brine, dried over Na₂SO₄, concentrated to obtain the crude product, which was purified by column chromatography (DCM = 100%) to obtain the target compound D50-2 (208 mg). ESI-MS m/z: 250.06[M+H]⁺.

### Step 3: Synthesis of compound D50-3

Compound D50-2 (208 mg) was dissolved in DCM (15 mL), copper acetate (150 mg), 3,5-difluorophenylboronic acid (206 mg), TEA compound (168 mg) were added, stirred at room temperature overnight, the reaction mixture was diluted with dichloromethane, washed twice with saturated brine, dried, filtered, and concentrated to obtain the crude product, which was purified by column chromatography (EA/PE=1:3) to obtain the target compound D50-3 (217 mg). ESI-MS m/z:
369.10[M+H]⁺.

### Step 4: Synthesis of compound D50

Compound D50-3 (100 mg) was dissolved in methanol (4 mL), NaBH₄ (21 mg) was added in an ice bath, after stirred at 0°C for 1 hour, ice water was added, the reaction mixture was extracted with ethyl acetate, the organic layer was washed with brine, dried over Na₂SO₄, concentrated to obtain the crude product, which was purified by Prep-TLC (EA/PE=1:4) to obtain the target compound D50 (82 mg). ESI-MS m/z: 353.12[M+H-H₂O]⁺.

### Example 103 Synthesis of the mixture of (4S,5S)-1-(3-(difluoromethyl)-4-fluorophenyl)-5-fluoro-3-(trifluoromethyl)-4,5,6,7-tetrahydro-1H-indol-4-ol and (4R,5R)-1-(3-(difluoromethyl)-4-fluorophenyl)-5-fluoro-3-(trifluoromethyl)-4,5,6,7-tetrahydro-1H-indol-4-ol ( compound D51-A, assumed structure)

### Example 104 Synthesis of (4S,5R)-1-(3-(difluoromethyl)-4-fluorophenyl)-5-fluoro-3-(trifluoromethyl)-4,5,6,7-tetrahydro-1H-indol-4-ol ( compound D51-B, assumed struture)

### Example 105 Synthesis of (4R, 5S)-1-(3-(difluoromethyl)-4-fluorophenyl)-5-fluoro-3-(trifluoromethyl)-4,5,6,7-tetrahydro-1H-indol-4-ol ( compound D51-C, assumed structure)

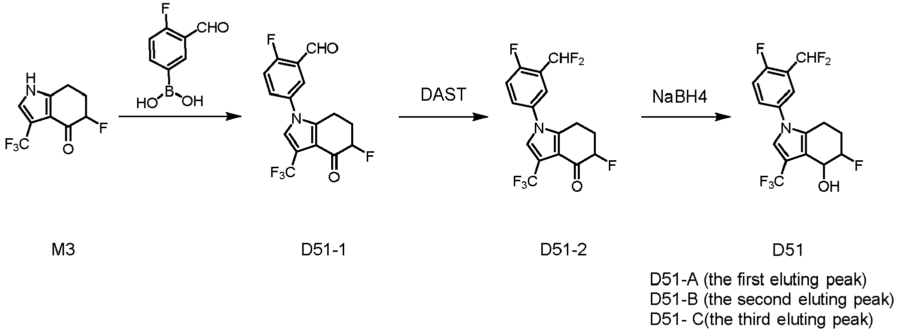

### Step 1: Synthesis of compound D51-1

Compound M3 (147 mg) was dissolved in DCM (15 mL), copper acetate (120 mg), 4-fluoro-3-aldophenylboronic acid (167 mg), TEA (136 mg) were added, stirred at room temperature overnight, the reaction mixture was diluted with dichloromethane, washed twice with saturated brine, dried, filtered, and concentrated to obtain the crude product, which was purified by column chromatography (EA/PE=1:3) to obtain the target compound D51-1 (152 mg). ESI-MS m/z: 344.08[M+H]⁺.

### Step 2: Synthesis of compound D51-2

Compound D51-1 (152 mg) was dissolved in DCM (10 mL), DAST (214 mg) was added in an ice bath, stirred at 0°C for 3 hours, diluted with dichloromethane, washed twice with saturated brine, dried, and filtered, concentrated to obtain the crude product, which was purified by column chromatography (EA/PE=1:3) to obtain the target compound D51-2 (129 mg). ESI-MS m/z: 344.08[M+H]⁺.

### Step 3: Synthesis of compound D51

Compound D51-2 (350 mg) was dissolved in MeOH (5 mL), sodium borohydride (0.15 g) was added in batches, stirred at room temperature for 6 hours, monitored by LC-MS and TLC, after the reaction was completed, the reaction solution was poured into water, extracted with ethyl acetate, dried, spin-dried, the crude product was chromatographed to give D51 (300 mg). D51 was separated by chiral preparation (column type: Daicel CHIRALPAK^{®} IF 250^{∗}20 mm, 5 µm; mobile phase A: n-hexane, Mobile phase B: ethanol; mobile phase A: mobile phase B=90:10 (V/V); detection wavelength: 254 nM; flow rate: 15 mL/min; column temperature: RT; running time: 25 min; column pressure: 35 Bar) to obtain three elution peaks.

D51-A (10 mg) was the first eluting peak, Rt=7.5 min, ESI-MS m/z: 350.12[M+H-H₂O]⁺, ¹H NMR (500 MHz, DMSO) δ 7.77 - 7.67 (m, 2H), 7.53-7.59 (m, 2H), 7.23 (t, *J* = 53.9 Hz, 1H), 5.44 (d, *J* = 6.7 Hz, 1H), 4.80 (d, *J* = 47.3 Hz, 1H), 4.64 (s, 1H), 2.64 - 2.54 (m, 1H), 2.48 - 2.51 (m, 1H), 2.18 - 1.94 (m, 2H). D51-B (70 mg) was the second eluting peak, Rt=11 min, ESI-MS m/z: 350.12[M+H-H₂O]⁺, ¹H NMR (500 MHz, DMSO) δ 7.71 (dd, *J* = 5.7, 2.8 Hz, 2H), 7.62 - 7.49 (m, 2H), 7.23 (t, *J* = 53.9 Hz, 1H), 5.19 (d, *J* = 6.1 Hz, 1H), 4.92 - 4.69 (m, 2H), 2.77 - 2.60 (m, 1H), 2.60 - 2.51 (m, 1H), 2.26 - 2.07 (m, 1H), 1.85 - 1.90 (m, 1H). D51-C (80 mg) was the third eluting peak, Rt=14.5 min, ESI-MS m/z: 350.12[M+H-H₂O]⁺, ¹H NMR (500 MHz, DMSO) δ 7.71 (dd, *J* = 5.7, 2.8 Hz, 2H), 7.62 - 7.49 (m, 2H), 7.23 (t, *J* = 53.9 Hz, 1H), 5.19 (d, *J* = 6.1 Hz, 1H), 4.92 - 4.69 (m, 2H), 2.77 - 2.60 (m, 1H), 2.60 - 2.51 (m, 1H), 2.26 - 2.07 (m, 1H), 1.85 - 1.90 (m, 1H).

### Example 106 Synthesis of 1-(3-chloro-5-fluorophenyl)-2,5,5-trifluoro-3-(trifluoromethyl)-4,5,6,7-tetrahydro-1H-indol-4-ol ( compound A41)

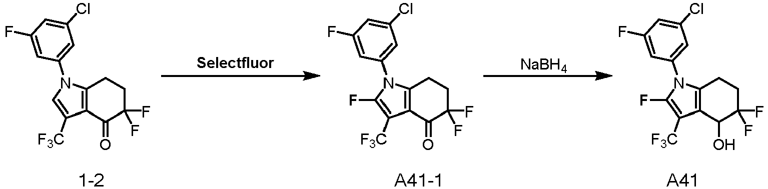

### Step 1: Synthesis of compound A41-1

Compound 1-2 (106 mg) was dissolved in acetonitrile (5 mL) under nitrogen protection, Selectfluor (133 mg) was added, the reaction mixture was stirred at 80°C for 2 hours, extracted with ethyl acetate, the organic layer was washed with brine, dried over Na₂SO₄, concentrated to obtain the crude product, which was purified by Prep-TLC (EA/PE=1:3) to obtain the target compound A41-1 (79 mg).

### Step 2: Synthesis of compound A41

Compound A41-1 (79 mg) was dissolved in a mixed solvent of THF (2 mL) and H₂O (1 mL), NaBH₄ (16 mg) was added in an ice bath, stirred at 0°C for 1 hour. The reaction mixture was added ice water, extracted with ethyl acetate, the organic layer was washed with brine, dried over Na₂SO₄, and concentrated to obtain the crude product, which was purified by Prep-TLC (EA/PE=1:3) to obtain the target compound A41 (45 mg). ESI-MS m/z: 370.02[M+H-H₂O]⁺. ¹H NMR (500 MHz, DMSO-*d₆*) δ 7.72-7.67 (m, 2H), 7.66-7.62 (m, 1H), 6.22 (d, *J* = 6.4 Hz, 1H), 4.55 (q, *J* = 6.6 Hz, 1H), 2.61-2.57 (m, 2H), 2.41-2.24 (m, 1H), 2.19-2.10 (m, 1H).

### Example 107 Synthesis of (S)-2-chloro-1-(3-chloro-5-fluorophenyl)-5,5-difluoro-3-(trifluoromethyl)-4,5,6,7-tetrahydro-1H-indol-4-ol ( compound A75)

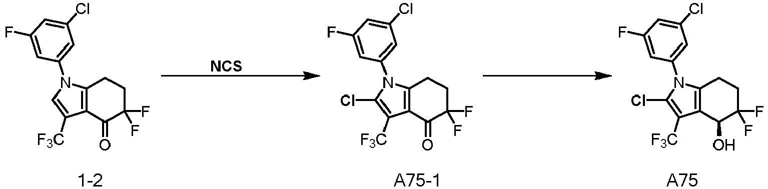

### Step 1: Synthesis of compound A75-1

Compound 1-2 (106 mg) was dissolved in acetonitrile (5 mL) under nitrogen protection, NCS (50 mg) was added, stirred at 80 °C for 2 h, the reaction mixture was extracted with ethyl acetate, the organic layer was washed with brine, dried over Na₂SO₄, concentrated to obtain the crude product, which was purified by Prep-TLC (EA/PE=1:3) to obtain the target compound A75-1 (81 mg). ESI-MS m/z:
401.9[M+H]⁺.

### Step 2: Synthesis of compound A75

Compound A75-1 (40 mg) was dissolved in dichloromethane (3 mL), formic acid (0.01 mL) and triethylamine (0.03 mL) were added, the reaction mixture was sparged with nitrogen, and [(R,R)-Ts-DPEN]RuCl(p-cymene) (12 mg) was added, stirred at room temperature under nitrogen protection overnight, concentrated, and the crude product was purified by Prep-TLC (EA/PE=1:3) to obtain the target compound A75 (15 mg). ESI-MS m/z: 387.85[M+H-H₂O]⁺.

### Example 108 Synthesis of 1-(3-(difluoromethyl)-4-fluorophenyl)-3-(trifluoromethyl)-4,5,6,7-tetrahydro-1H-indol-4-ol ( compound D95)

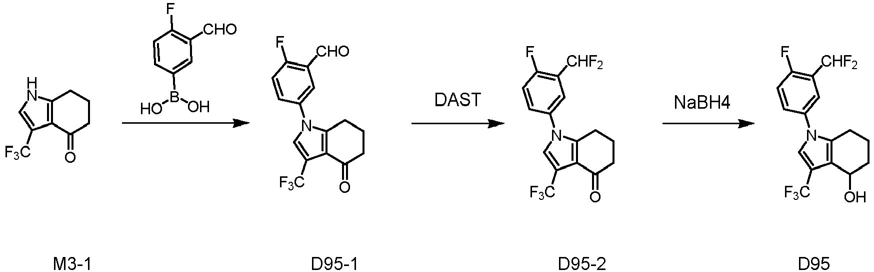

### Step 1: Synthesis of compound D95-1

(4-Fluoro-3-formylphenyl)boronic acid (2.0 g), compound M3-1 (3.63 g), copper acetate (2.16 g) and triethylamine (4.97 mL) were added to dichloromethane (40 mL)), the reaction mixture was stirred at room temperature under an oxygen atmosphere overnight, after the reaction was completed, the reaction mixture was diluted with dichloromethane, washed twice with saturated brine, dried, filtered, and concentrated to obtain a crude product, which was purified by column chromatography (EA/PE=1:3) to obtain the target compound D95-1 (2.1 g). ESI-MS m/z: 326.10[M+H]⁺.

### Step 2: Synthesis of compound D95-2

Compound D95-1 (500 mg) was added to dichloromethane (10 mL), DAST (520 mg) was added, stirred at room temperature, after the reaction was completed, the reaction mixture was diluted with dichloromethane, quenched with saturated sodium bicarbonate, the organic phase was washed with water, dried, filtered, concentrated to obtain the crude product, which was purified by column chromatography (EA/PE=1:3) to obtain the target compound D95-2 (163 mg). ESI-MS m/z: 348.05[M+H]⁺.

### Step 3: Synthesis of compound D95

Compound D95-2 (100 mg) was added to a mixed solution of tetrahydrofuran (2 mL) and water (1 mL), sodium borohydride (80 mg) was added in an ice-water bath, the reaction mixture was stirred at room temperature overnight, diluted with ethyl acetate, washed with saturated brine, the organic layer was dried, and purified by the HPLC to obtain the target product D95 (50 mg). ESI-MS m/z: 350.10[M+H]⁺.

Through different raw materials and/or intermediates, using a preparation method similar to example 1-example 108, the following example was obtained.

| **Example number** | **Structure** | **Chemical name** | **ESI-MS** |
|---|---|---|---|
| 109 | 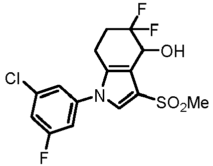 | 1-(3-chloro-5-fluorophenyl)-5,5-difluoro-3-(methylsulfonyl)-4,5,6,7-tetrahydro-1H-indol-4-ol | [M+H-H₂O]⁺: 362.02 |
| 110 | 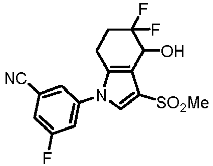 | 3-(5,5-difluoro-4-hydroxy-3 - (methylsulfonyl)-4,5,6,7-tetrahydro-1H-indol-1-yl)-5-fluorobenzonitrile | [M+H-H₂O]⁺: 353.06 |
| 111 | 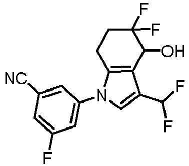 | 3-(3-(difluoromethyl)-5,5-difluoro-4-hydroxy-4,5,6,7-tetrahydro-1H-indol-1-yl)-5-fluorobenzonitrile | [M+H-H₂O]⁺: 325.10 |
| 112 | 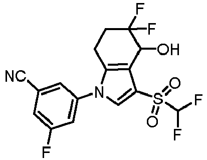 | 3-(3-((difluoromethyl)sulfonyl)-5,5-difluoro-4-hydroxy-4,5,6,7-tetrahydro-1H-indol-1-yl)-5-fluorobenzonitrile | [M+H-H₂O]⁺: 389.06 |
| 113 | 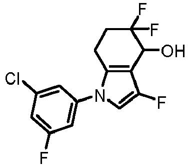 | 1-(3-chloro-5-fluorophenyl)-3,5,5-trifluoro-4,5,6,7-tetrahydro-1H-indol-4-ol | [M+H-H₂O]⁺ : 302.4 |
| 114 | 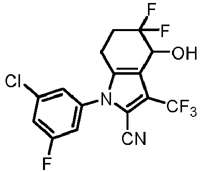 | 1-(3-chloro-5-fluorophenyl)-5,5-difluoro-4-hydroxy-3-(trifluoromethyl)-4,5,6,7-tetrahydro-1H-indole-2-carbonitrile | [M+H-H₂O]⁺: 377.03 |
| 115 | 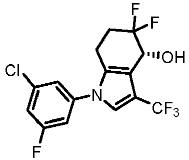 | (R)-1-(3-chloro-5-fluorophenyl)-5,5-difluoro-3-(trifluoromethyl)-4,5,6,7-tetrahydro-1H-indol-4-ol | [M+H-H₂O]⁺: 352.00 |
| 116 | 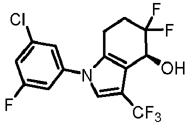 | (S)-1-(3-chloro-5-fluorophenyl)-5,5-difluoro-3-(trifluoromethyl)-4,5,6,7-tetrahydro-1H-indol-4-ol | [M+H-H₂O]⁺: 352.00 |
| 117 | 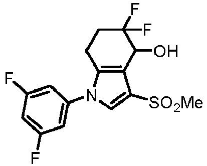 | 1-(3,5-difluorophenyl)-5,5-difluoro-3-(methylsulfonyl)-4,5,6,7-tetrahydro-1H-indol-4-ol | [M+H-H₂O]⁺: 346.20 |
| 118 | 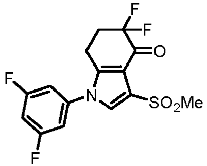 | 1-(3,5-difluorophenyl)-5,5-difluoro-3-(methylsulfonyl)-1,5,6,7-tetrahydro-4H-indol-4-one | [M+H]⁺: 362.18 |
| 119 | 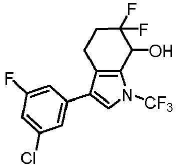 | 3-(3-chloro-5-fluorophenyl)-6,6-difluoro-1-(trifluoromethyl)-4,5,6,7-tetrahydro-1H-indol-7-ol | [M+H-H₂O]⁺: 352.10 |
| 120 | 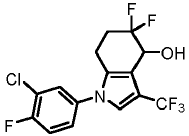 | 1-(3-chloro-4-fluorophenyl)-5,5-difluoro-3-(trifluoromethyl)-4,5,6,7-tetrahydro-1H-indol-4-ol | [M+H-H₂O]⁺: 352.00 |
| 121 | 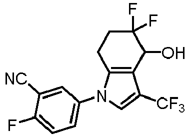 | 5-(5,5-difluoro-4-hydroxy-3 - (trifluoromethyl)-4,5,6,7-tetrahydro-1H-indol-1-yl)-2-fluorobenzonitrile | [M+H-H₂O]⁺: 343.05 |
| 122 | 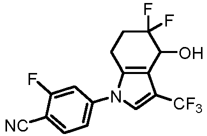 | 4-(5,5-difluoro-4-hydroxy-3-(trifluoromethyl)-4,5,6,7-tetrahydro-1H-indol-1-yl)-2-fluorobenzonitrile | [M+H-H₂O]⁺: 342.95 |
| 123 | 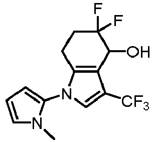 | 5,5-difluoro-1-(1-methyl-1H-pyrrol-2-yl)-3-(trifluoromethyl)-4,5,6,7-tetrahydro-1H-indol-4-ol | [M+H-H₂O]⁺: 303.09 |
| 124 | 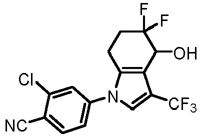 | 2-chloro-4-(5,5-difluoro-4-hydroxy-3-(trifluoromethyl)-4,5,6,7-tetrahydro-1H-indol-1-yl)benzonitrile | [M+H-H₂O]⁺: 358.95 |
| 125 | 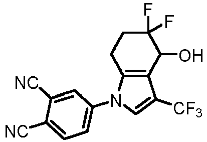 | 4-(5,5-difluoro-4-hydroxy-3-(trifluoromethyl)-4,5,6,7-tetrahydro-1H-indol-1-yl)phthalonitrile | [M+H-H₂O]⁺: 350.00 |
| 126 | 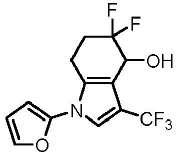 | 5,5-difluoro-1-(furan-2-yl)-3-(trifluoromethyl)-4,5,6,7-tetrahydro-1H-indol-4-ol | [M+H-H₂O]⁺: 290.06 |
| 127 | 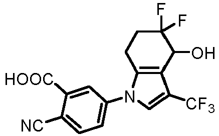 | 2-cyano-5-(5,5-difluoro-4-hydroxy-3-(trifluoromethyl)-4,5,6,7-tetrahydro-1H-indol-1-yl)benzoic acid | [M+H-H₂O]⁺: 369.07 |
| 128 | 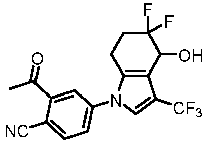 | 2-acetyl-4-(5,5-difluoro-4-hydroxy-3-(trifluoromethyl)-4,5,6,7-tetrahydro-1H-indol-1-yl)benzonitrile | [M+H-H₂O]⁺: 367.09 |
| 129 | 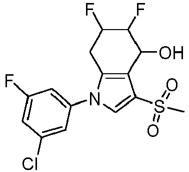 | 1-(3-chloro-5-fluorophenyl)-5,6-difluoro-3-(methylsulfonyl)-4,5,6,7-tetrahydro-1H-indol-4-ol | [M+H-H₂O]⁺: 362.02 |
| 130 | 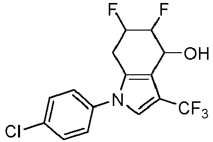 | 1-(4-chlorophenyl)-5,6-difluoro-3-(trifluoromethyl)-4,5,6,7-tetrahydro-1H-indol-4-ol | [M+H-H₂O]⁺: 334.04 |
| 131 | 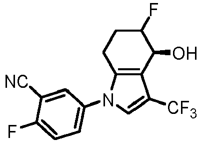 | 2-fluoro-5-((4S)-5-fluoro-4-hydroxy-3-(trifluoromethyl)-4,5,6,7-tetrahydro-1H-indol-1-yl)benzonitrile | [M+H-H₂O]⁺: 325.17 |
| 132 | 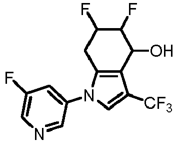 | 5,6-difluoro-1-(5-fluoropyridin-3-yl)-3-(trifluoromethyl)-4,5,6,7-tetrahydro-1H-indol-4-ol | [M+H-H₂O]⁺: 319.00 |
| 133 | 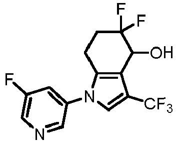 | 5,5-difluoro-1-(5-fluoropyridin-3-yl)-3-(trifluoromethyl)-4,5,6,7-tetrahydro-1H-indol-4-ol | [M+H-H₂O]⁺: 318.95 |
| 134 | 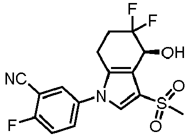 | (S)-5-(5,5-difluoro-4-hydroxy-3-(methylsulfonyl)-4,5,6,7-tetrahydro-1H-indol-1-yl)-2-fluorobenzonitrile | [M+H-H₂O]⁺: 352.95 |
| 135 | 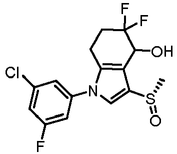 | 1-(3-chloro-5-fluorophenyl)-5,5-difluoro-3-((S)-methylsulfinyl)-4,5,6,7-tetrahydro-1H-indol-4-ol | [M+H]⁺: 364.16 |
| 136 | 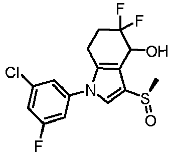 | 1-(3-chloro-5-fluorophenyl)-5,5-difluoro-3-((R)-methylsulfinyl)-4,5,6,7-tetrahydro-1H-indol-4-ol | [M+H]⁺: 364.13 |
| 137 | 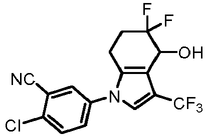 | 2-chloro-5-(5,5-difluoro-4-hydroxy-3-(trifluoromethyl)-4,5,6,7-tetrahydro-1H-indol-1-yl)benzonitrile | [M+H-H₂O]⁺: 359.04 |
| 138 | 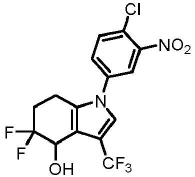 | 1-(4-chloro-3-nitrophenyl)-5,5-difluoro-3-(trifluoromethyl)-4,5,6,7-tetrahydro-1H-indol-4-ol | [M+H-H₂O]⁺: 379.00 |
| 139 | 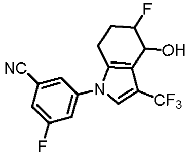 | 3-fluoro-5-(5-fluoro-4-hydroxy-3-(trifluoromethyl)-4,5,6,7-tetrahydro-1H-indol-1-yl)benzonitrile | [M+H-H₂O]⁺: 325.00 |
| 140 | 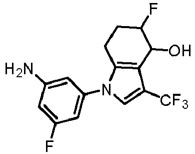 | 1-(3-amino-5-fluorophenyl)-5-fluoro-3-(trifluoromethyl)-4,5,6,7-tetrahydro-1H-indol-4-ol | [M+H-H₂O]⁺: 315.09 |
| 141 | 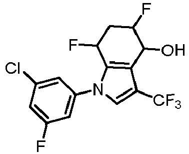 | 1-(3-chloro-5-fluorophenyl)-5,7-difluoro-3-(trifluoromethyl)-4,5,6,7-tetrahydro-1H-indol-4-ol | [M+H-H₂O]⁺: 352.03 |
| 142 | 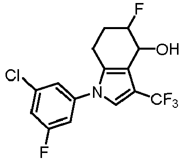 | 1-(3-chloro-5-fluorophenyl)-5-fluoro-3-(trifluoromethyl)-4,5,6,7-tetrahydro-1H-indol-4-ol | [M+H-H₂O]⁺: 334.11 |
| 143 | 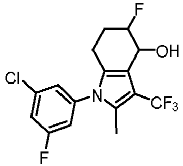 | 1-(3-chloro-5-fluorophenyl)-5-fluoro-2-methyl-3-(trifluoromethyl)-4,5,6,7-tetrahydro-1H-indol-4-ol | [M+H-H₂O]⁺: 348.06 |
| 144 | 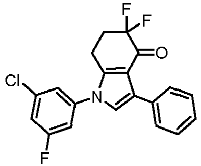 | 1-(3-chloro-5-fluorophenyl)-5,5-difluoro-3-phenyl-1,5,6,7-tetrahydro-4H-indol-4-one | [M+H]⁺: 376.23 |
| 145 | 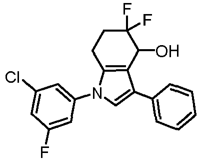 | 1-(3-chloro-5-fluorophenyl)-5,5-difluoro-3-phenyl-4,5,6,7-tetrahydro-1H-indol-4-ol | [M+H-H₂O]⁺ : 360.17 |
| 146 | 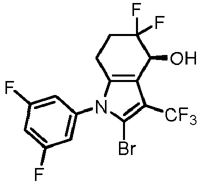 | (S)-2-bromo-1-(3,5-difluorophenyl)-5,5-difluoro-3-(trifluoromethyl)-4,5,6,7-tetrahydro-1H-indol-4-ol | [M+H-H₂O]⁺ : 433.93/431.94 |
| 147 | 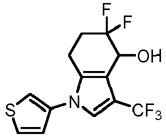 | 5,5-difluoro-1-(thiophen-3-yl)-3-(trifluoromethyl)-4,5,6,7-tetrahydro-1H-indol-4-ol | [M+H-H₂O]⁺ : 306.08 |
| 148 | 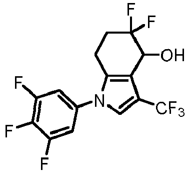 | 5,5-difluoro-3-(trifluoromethyl)-1-(3,4,5-trifluorophenyl)-4,5,6,7-tetrahydro-1H-indol-4-ol | [M+H-H₂O]⁺: 354.15 |
| 149 | 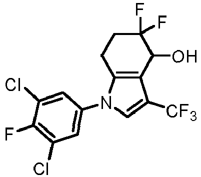 | 1-(3,5-dichloro-4-fluorophenyl)-5,5-difluoro-3-(trifluoromethyl)-4,5,6,7-tetrahydro-1H-indol-4-ol | [M+H-H₂O]⁺: 386.00 |
| 150 | 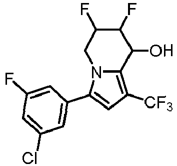 | 3-(3-chloro-5-fluorophenyl)-6,7-difluoro-1-(trifluoromethyl)-5,6,7,8-tetrahydroindolizin-8-ol | [M+H-H₂O]⁺: 352.03 |
| 151 | 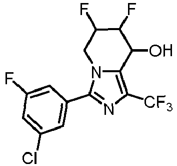 | 3-(3-chloro-5-fluorophenyl)-6,7-difluoro-1-(trifluoromethyl)-5,6,7,8-tetrahydroimidazo[1,5-a]pyridin-8-ol | [M+H-H₂O]⁺: 353.03 |
| 152 | 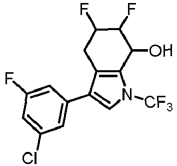 | 3-(3-chloro-5-fluorophenyl)-5,6-difluoro-1-(trifluoromethyl)-4,5,6,7-tetrahydro-1H-indol-7-ol | [M+H-H₂O]⁺: 352.03 |
| 153 | 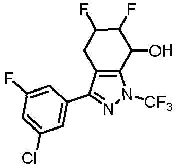 | 3-(3-chloro-5-fluorophenyl)-5,6-difluoro-1-(trifluoromethyl)-4,5,6,7-tetrahydro-1H-indazol-7-ol | [M+H-H₂O]⁺: 353.03 |
| 154 | 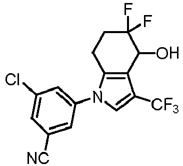 | 3-chloro-5-(5,5-difluoro-4-hydroxy-3-(trifluoromethyl)-4,5,6,7-tetrahydro-1H-indol-1-yl)benzonitrile | [M+H-H₂O]⁺: 359.00 |
| 155 | 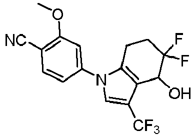 | 4-(5,5-difluoro-4-hydroxy-3-(trifluoromethyl)-4,5,6,7-tetrahydro-1H-indol-1-yl)-2-methoxybenzonitrile | [M+H-H₂O]⁺: 354.95 |
| 156 | 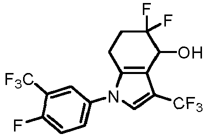 | 5,5-difluoro-1-(4-fluoro-3-(trifluoromethyl)phenyl)-3 - (trifluoromethyl)-4,5,6,7-tetrahydro-1H-indol-4-ol | [M+H-H₂O]⁺: 386.12 |
| 157 | 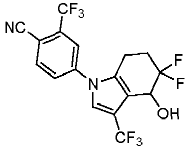 | 4-(5,5-difluoro-4-hydroxy-3-(trifluoromethyl)-4,5,6,7-tetrahydro-1H-indol-1-yl)-2-(trifluoromethyl)benzonitrile | [M+H-H₂O]⁺: 392.05 |
| 158 | 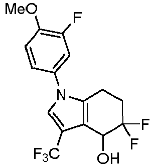 | 5,5-difluoro-1-(3-fluoro-4-methoxyphenyl)-3-(trifluoromethyl)-4,5,6,7-tetrahydro-1H-indol-4-ol | [M+H-H₂O]⁺: 348.18 |
| 159 | 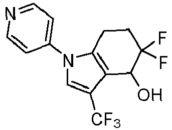 | 5,5-difluoro-1-(pyridin-4-yl)-3-(trifluoromethyl)-4,5,6,7-tetrahydro-1H-indol-4-ol | [M+H-H₂O]⁺: 319.00 |
| 160 | 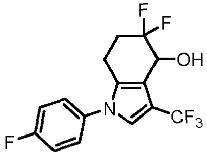 | 5,5-difluoro-1-(4-fluorophenyl)-3-(trifluoromethyl)-4,5,6,7-tetrahydro-1H-indol-4-ol | [M+H-H₂O]⁺: 318.02 |
| 161 | 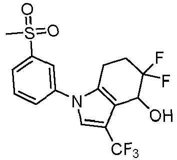 | 5,5-difluoro-1-(3-(methylsulfonyl)phenyl)-3-(trifluoromethyl)-4,5 ,6,7-tetrahydro-1H-indol-4-ol | [M+H-H₂O]⁺: 378.00 |
| 162 | 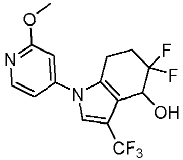 | 5,5-difluoro-1-(2-methoxypyridin-4-yl)-3 - (trifluoromethyl)-4,5,6,7-tetrahydro-1H-indol-4-ol | [M+H-H₂O]⁺: 349.35 |
| 163 | 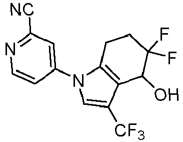 | 4-(5,5-difluoro-4-hydroxy-3 - (trifluoromethyl)-4,5,6,7-tetrahydro-1H-indol-1-yl)picolinonitrile | [M+H-H₂O]⁺: 344.00 |
| 164 | 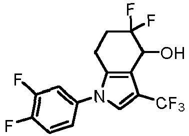 | 1-(3,4-difluorophenyl)-5,5-difluoro-3-(trifluoromethyl)-4,5,6,7-tetrahydro-1H-indol-4-ol | [M+H-H₂O]⁺: 336.23 |
| 165 | 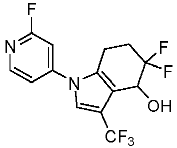 | 5,5-difluoro-1-(2-fluoropyridin-4-yl)-3-(trifluoromethyl)-4,5,6,7-tetrahydro-1H-indol-4-ol | [M+H-H₂O]⁺: 337.00 |
| 166 | 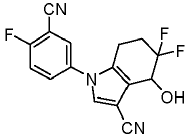 | 1-(3-cyano-4-fluorophenyl)-5,5-difluoro-4-hydroxy-4,5,6,7-tetrahydro-1H-indole-3-carbonitrile | [M+H-H₂O]⁺: 299.95 |
| 167 | 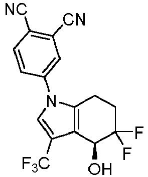 | (S)-4-(5,5-difluoro-4-hydroxy-3-(trifluoromethyl)-4,5,6,7-tetrahydro-1H-indol-1-yl)phthalonitrile | [M+H-H₂O]⁺: 350.00 |
| 168 | 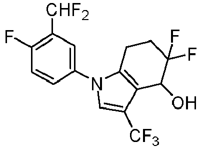 | 1-(3-(difluoromethyl)-4-fluorophenyl)-5,5-difluoro-3-(trifluoromethyl)-4,5,6,7-tetrahydro-1H-indol-4-ol | [M+H-H₂O]⁺: 368.00 |
| 169 | 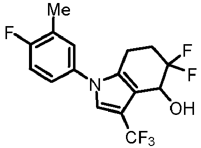 | 5,5-difluoro-1-(4-fluoro-3-methylphenyl)-3-(trifluoromethyl)-4,5,6,7-tetrahydro-1H-indol-4-ol | [M+H-H₂O]⁺: 332.30 |
| 170 | 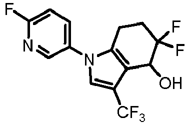 | 5,5-difluoro-1-(6-fluoropyridin-3-yl)-3-(trifluoromethyl)-4,5,6,7-tetrahydro-1H-indol-4-ol | [M+H-H₂O]⁺: 319.15 |
| 171 | 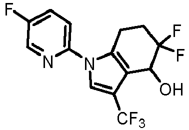 | 5,5-difluoro-1-(5-fluoropyridin-2-yl)-3-(trifluoromethyl)-4,5,6,7-tetrahydro-1H-indol-4-ol | [M+H-H₂O]⁺: 319.15 |
| 172 | 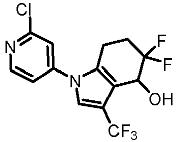 | 1-(2-chloropyridin-4-yl)-5,5-difluoro-3-(trifluoromethyl)-4,5,6,7-tetrahydro-1H-indol-4-ol | [M+H-H₂O]⁺: 335.00 |
| 173 | 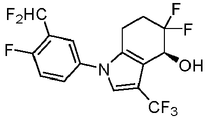 | (S)-1-(3-(difluoromethyl)-4-fluorophenyl)-5,5-difluoro-3-(trifluoromethyl)-4,5,6,7-tetrahydro-1H-indol-4-ol | [M+ HCOOH -H]⁻ : 430.05 |
| 174 | 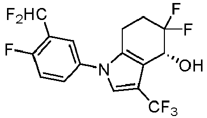 | (R)-1-(3-(difluoromethyl)-4-fluorophenyl)-5,5-difluoro-3-(trifluoromethyl)-4,5,6,7-tetrahydro-1H-indol-4-ol | [M+ HCOOH -H]⁻ : 430.2 |
| 175 | 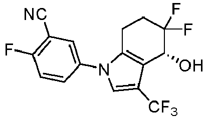 | (R)-5-(5,5-difluoro-4-hydroxy-3-(trifluoromethyl)-4,5,6,7-tetrahydro-1H-indol-1-yl)-2-fluorobenzonitrile | [M+ HCOOH -H]⁻ : 405.07 |
| 176 | 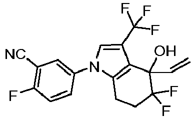 | 5-(5,5-difluoro-4-hydroxy-3 - (trifluoromethyl)-4-vinyl-4,5,6,7-tetrahydro-1H-indol-1-yl)-2-fluorobenzonitrile | [M+H-H₂O]⁺ : 369.20 |
| 177 | 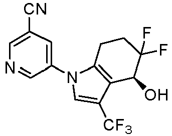 | (S)-5-(5,5-difluoro-4-hydroxy-3-(trifluoromethyl)-4,5,6,7-tetrahydro-1H-indol-1-yl)nicotinonitrile | [M+H]⁺ : 344.07 |
| 178 | 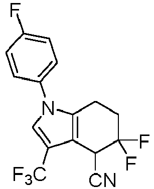 | 5,5-difluoro-1-(4-fluorophenyl)-3-(trifluoromethyl)-4,5,6,7-tetrahydro-1H-indole-4-carbonitrile | [M+H]⁺ : 345.06 |
| 179 | 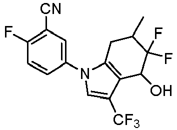 | 5-(5,5-difluoro-4-hydroxy-6-methyl-3-(trifluoromethyl)-4,5,6,7-tetrahydro-1H-indol-1-yl)-2-fluorobenzonitrile | [M+H-H₂O]⁺ : 357.00 |
| 180 | 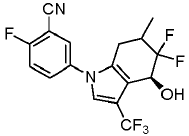 | 5-((4S)-5,5-difluoro-4-hydroxy-6-methyl-3-(trifluoromethyl)-4,5,6,7-tetrahydro-1H-indol-1-yl)-2-fluorobenzonitrile | [M+H-H₂O]⁺ : 357.02 |
| 181 | 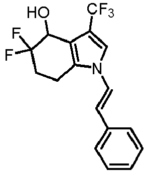 | (E)-5,5-difluoro-1-styryl-3-(trifluoromethyl)-4,5,6,7-tetrahydro-1H-indol-4-ol | [M+H-H₂O]⁺ : 326.09 |
| 182 | 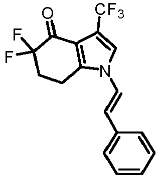 | (E)-5,5-difluoro-1-styryl-3-(trifluoromethyl)-1,5,6,7-tetrahydro-4H-indol-4-one | [M+H]⁺ : 342.12 |
| 183 | 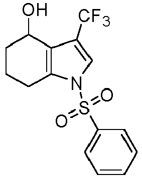 | 1-(phenylsulfonyl)-3-(trifluoromethyl)-4,5,6,7-tetrahydro-1H-indol-4-ol | [M+H]⁺ : 346.03 |
| 184 | 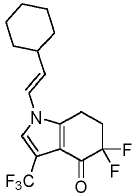 | (E)-1-(2-cyclohexylvinyl)-5,5-difluoro-3-(trifluoromethyl)-1,5,6,7-tetrahydro-4H-indol-4-one | [M+H]⁺ : 348.13 |
| 185 | 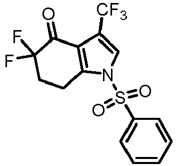 | 5,5-difluoro-1-(phenylsulfonyl)-3-(trifluoromethyl)-1,5,6,7-tetrahydro-4H-indol-4-one | [M+H]⁺ : 380.03 |
| 186 | 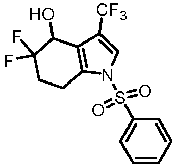 | 5,5-difluoro-1-(phenylsulfonyl)-3-(trifluoromethyl)-4,5,6,7-tetrahydro-1H-indol-4-ol | [M+H-H₂O]⁺ : 364.04 |
| 187 | 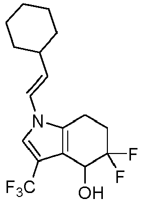 | (E)-1-(2-cyclohexylvinyl)-5,5-difluoro-3-(trifluoromethyl)-4,5,6,7-tetrahydro-1H-indol-4-ol | [M+H-H₂O]⁺ : 332.15 |
| 188 | 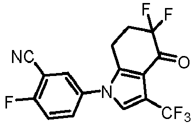 | 5-(5,5-difluoro-4-oxo-3 - (trifluoromethyl)-4,5,6,7-tetrahydro-1H-indol-1-yl)-2-fluorobenzonitrile | [M+H]⁻ : 359.05 |
| 189 | 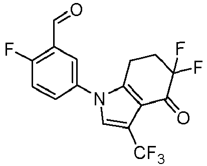 | 5-(5,5-difluoro-4-oxo-3 - (trifluoromethyl)-4,5,6,7-tetrahydro-1H-indol-1-yl)-2-fluorobenzaldehyde | [M+H]⁻ : 362.06 |
| 190 | 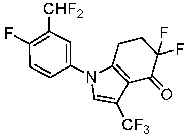 | 1-(3-(difluoromethyl)-4-fluorophenyl)-5,5-difluoro-3-(trifluoromethyl)-1,5,6,7-tetrahydro-4H-indol-4-one | [M-H]⁻ : 382.06 |
| 191 | 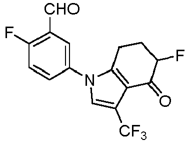 | 2-fluoro-5-(5-fluoro-4-oxo-3-(trifluoromethyl)-4,5,6,7-tetrahydro-1H-indol-1-yl)benzaldehyde | [M+H]⁺ : 344.06 |
| 192 | 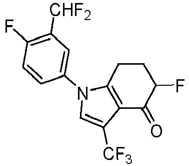 | 1-(3-(difluoromethyl)-4-fluorophenyl)-5-fluoro-3-(trifluoromethyl)-1,5,6,7-tetrahydro-4H-indol-4-one | [M-H]⁻ : 364.07 |
| 193 | 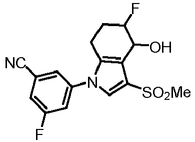 | 3-fluoro-5-(5-fluoro-4-hydroxy-3-(methylsulfonyl)-4,5,6,7-tetrahydro-1H-indol-1-yl)benzonitrile | [M+H-H₂O]⁺ : 335.07 |
| 194 | 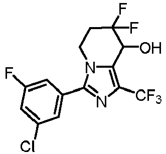 | 3-(3-chloro-5-fluorophenyl)-7,7-difluoro-1-(trifluoromethyl)-5,6,7,8-tetrahydroimidazo[1,5-a]pyridin-8-ol | [M+H-H₂O]⁺: 353.07 |
| 195 | 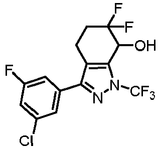 | 3-(3-chloro-5-fluorophenyl)-6,6-difluoro-1-(trifluoromethyl)-4,5,6,7-tetrahydro-1H-indazol-7-ol | [M+H-H₂O]⁺: 353.07 |
| 204 | 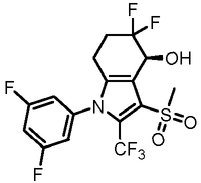 | (S)-1-(3,5-difluorophenyl)-5,5-difluoro-3-(methylsulfonyl)-2-(trifluoromethyl)-4,5,6,7-tetrahydro-1H-indol-4-ol | [M+H-H₂O]⁺: 414.04 |

### Example 196 and Example 197 Synthesis of (S)-2-fluoro-5-(4,5,5-trifluoro-3-(trifluoromethyl)-4,5,6,7-tetrahydro-1H-indol-1-yl)benzonitrile ( compound D54, assumed) and (R)-2-fluoro-5-(4,5,5-trifluoro-3-(trifluoromethyl)-4,5,6,7-tetrahydro-1H-indol-1-yl)benzonitrile ( compound D55, assumed)

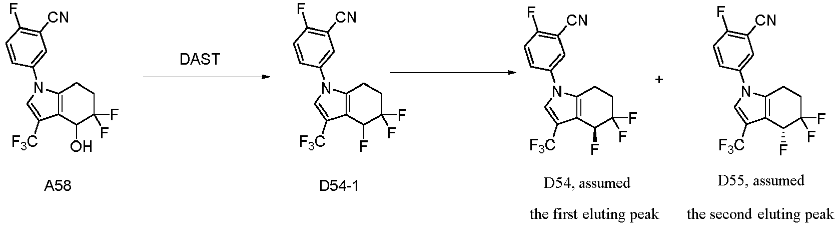

### Step 1: Synthesis of compound D54-1

A58 (360 mg) was dissolved in 20 mL of dichloromethane under nitrogen protection, DAST (412 mg) was added slowly and dropwise in an ice bath, the reaction was carried out at 0 °C for 2 hours, quenched by the addition of ice water, the organic layer was washed with saturated sodium bicarbonate and saturated brine successively, dried over anhydrous sodium sulfate and concentrated to obtain the crude product, the crude product was purified by column chromatography (EA/PE=1:3) to obtain the target compound D54-1 (285 mg). ESI-MS m/z:362.30[M+H]⁺.

### Step 2: Synthesis of compound D54 and D55

Compound D54-1 was separated and purified by chiral preparative column (column type Daicel CHIRALPAK^{®} IF 250^{∗}20 mm, 5µm, mobile phase A: n-hexane, mobile phase B: ethanol; mobile phase A: mobile phase B=85 : 15 (V/V), detection wavelength 254 nm; flow rate: 15 mL/min; column temperature: RT; running time: 28 min), example D54 was the first eluting peak, Rt=16.5min, ESI-MS m/z: 325.00 [M+H-H₂O]⁺ ; ¹H NMR (500 MHz, DMSO) δ 8.14 - 8.16 (m, 1H), 7.89-7.92 (m, 1H), 7.73 - 7.67 (m, 1H), 7.59 (s, 1H), 5.23 (d, *J* = 6.0 Hz, 1H), 4.88 - 4.70 (m, 2H), 2.56 - 2.76 (m, 2H), 2.23 - 2.13 (m, 1H), 1.85 - 1.92 (m, 1H); example D55 was the second eluting peak, Rt=20 min, ESI-MS m/z: 325.00 [M+H-H₂O]⁺.

### Example 198 Synthesis of (Z)-5-(5,5-difluoro-4-(hydroxyimino)-3-(trifluoromethyl)-4,5,6,7-tetrahydro-1H-indol-1-yl)-2-fluorobenzonitrile ( compound D56)

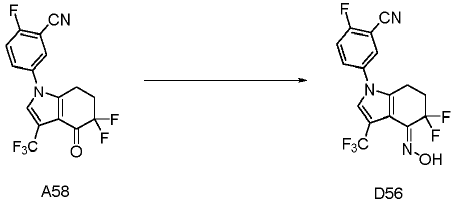

### Step 1: Synthesis of compound D56

Compound A58 (36 mg) and hydroxylamine hydrochloride (14 mg) were dissolved in 10 mL of ethanol, refluxed for 5 hours, concentrated to obtain the crude product, which was purified by Pre-TLC (EA/PE=1:3) to obtain the target compound D56 (16 mg). ESI-MS m/z:392.08 M+H-H₂O]⁺.

### Example 199 Synthesis of 2-fluoro-5-(5-fluoro-4-hydroxy-2-(trifluoromethyl)-4,5,6,7-tetrahydro-1H-indol-1-yl)benzonitrile (D58)

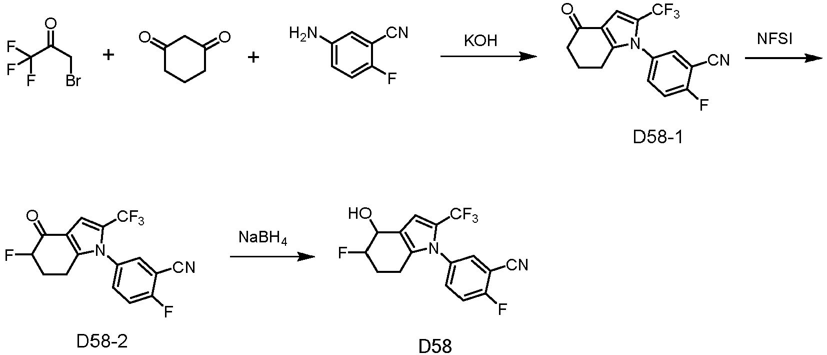

### Step 1: Synthesis of compound D58-1

1,3-cyclohexanedione (1.10 g) was dissolved in an aqueous solution (4 mL) of potassium hydroxide (0.57 g) in an ice bath, a solution of 3-bromo-1,1,1-trifluoroacetone (1.84 g) in methanol (10 mL) was added dropwise, stirred for 2 h and the methanol was spin-dried, water (5 mL) and 2-fluoro-5-aminobenzonitrile (1.2 g) were added to adjust the pH to 0, the reaction was refluxed for 14 h. The reaction mixture was spin-dried and extracted with ethyl acetate, the organic layer was washed with brine, dried over Na₂SO₄, and concentrated to obtain the crude product, which was purified by column chromatography (EA/PE=1:3) to obtain the target compound D58-1(254 mg, 8%). ESI-MS m/z: 323.07[M+H]⁺. ¹H NMR (500 MHz, CDCl₃) δ 7.66 (dd, *J* = 14.2, 11.6 Hz, 1H), 7.61 (dd, *J* = 8.4, 4.0 Hz, 1H), 7.41 (t, *J* = 8.4 Hz, 1H), 7.10 (s, 1H), 2.57-2.44 (m, 4H), 2.22-2.12 (m, 2H).

### Step 2: Synthesis of compound D58-2

Compound D58-1 (60 mg) and NFSI (90 mg) were dissolved in tetrahydrofuran (2.00 mL) under nitrogen protection, the temperature was cooled to -78 °C and lithium diisopropylamide (0.20 mL, 2.00 mol/L) was added, stirred for 2 hours. The reaction mixture was quenched with saturated ammonium chloride, extracted with ethyl acetate, and concentrated to obtain the crude product, which was purified by column chromatography (EA/PE=1:2) to obtain the target compound D58-2 (40 mg, 59%). ESI-MS m/z: 341.06[M+H]⁺.

### Step 3: Synthesis of compound D58

Compound D58-2 (40 mg) was dissolved in a mixed solvent of methanol (1 mL) and tetrahydrofuran (1 mL), sodium borohydride (10 mg) was added in an ice bath, and the mixture was stirred at room temperature for 2 hours. Water and ethyl acetate were added, the organic layer was concentrated to obtain the crude product, which was purified by column chromatography (EA/PE=1:2) to obtain the target compound D58 (20 mg). ESI-MS m/z: 336.20[M+H-H₂O]⁺.

### Example 200 Synthesis of (S)-5-(5,5-difluoro-4-hydroxy-3-(trifluoromethyl)-4,5,6,7-tetrahydro-1H-indol-1-yl-4-d)-2-fluorobenzonitrile ( compound D74)

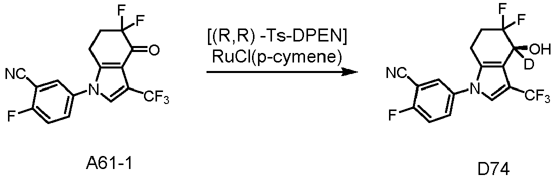

### Step 1: Synthesis of compound D74

Compound A61-1 (120 mg) was dissolved in dichloromethane (3 mL), deuterated formic acid (0.04 mL) and triethylamine (0.1 mL) were added, the reaction mixture was sparged with nitrogen, and [(R,R)-Ts-DPEN]RuCl(p-cymene) (22 mg) was added, the mixture was stirred at room temperature under nitrogen protection overnight, concentrated, and the crude product was purified by Prep-TLC (EA/PE=1:3) to obtain the target compound D74 (70 mg, ee>96%). ESI-MS m/z: 406.07[M+HCOOH-H]⁻. ¹H NMR (500 MHz, DMSO) δ 8.22 (dd, *J* = 5.4, 2.7 Hz, 1H), 8.02 - 7.85 (m, 1H), 7.80 - 7.49 (m, 2H), 6.16 - 5.82 (m, 1H), 2.86 - 2.60 (m, 2H), 2.42 - 2.26 (m, 1H), 2.26 - 2.06 (m, 1H).

### Example 201 Synthesis of 6-(3-chloro-5-fluorophenyl)-8-(trifluoromethyl)-5,6-dihydro-4H-isoxazolo[5,4-e]indole ( compound D100)

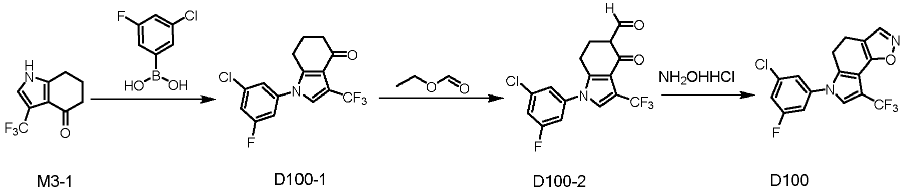

### Step 1: Synthesis of compound D100-1

Compound M3-1 (203 mg) was dissolved in DCM (20 mL), copper acetate (180 mg), 3-chloro-5-fluorophenylboronic acid (261 mg) and TEA (202 mg) were added, and the mixture was stirred at room temperature overnight. The reaction mixture was diluted with dichloromethane, washed twice with saturated brine, dried, and concentrated to obtain the crude product, which was purified by Pre-TLC (EA/PE=1:3) to obtain the target compound D100-1 (275 mg). ESI-MS m/z: 332.05[M+H]⁺.

### Step 2: Synthesis of compound D100-2

Compound D100-1 (100 mg) was dissolved in ethanol (5 mL) under nitrogen protection, cooled to 0 °C, sodium ethoxide (20 mg) was added, and ethyl formate (23 mg) was added dropwise after stirring for 10 minutes, stirred overnight at room temperature. The reaction solution was spin-dried and diluted with ethyl acetate, washed twice with saturated brine, dried, and concentrated to obtain the crude product, which was purified by column chromatography (EA/PE=1:3) to obtain the target compound D100-2 (35 mg). ESI-MS m/z: 360.17[M+H]⁺.

### Step 3: Synthesis of compound D100

Compound D100-2 (100 mg) was dissolved in acetic acid (5 mL), hydroxylamine hydrochloride (23 mg) was added, and the mixture was stirred at room temperature for 5 hours. The reaction solution was poured into water, extracted with ethyl acetate, washed twice with saturated brine, dried, concentrated to obtain the crude product, which was purified by Pre-TLC (EA/PE=1:3) to obtain the target compound D100 (42 mg). ESI-MS m/z: 357.12[M+H]⁺. ¹H NMR (500 MHz, DMSO-d₆) δ 8.49 (s, 1H), 7.82 (s, 1H), 7.67 - 7.56 (m, 3H), 2.96 (t, J = 8.5 Hz, 2H), 2.83 (t, J = 8.5 Hz, 2H).

### Example 202 Synthesis of 6-(3-chloro-5-fluorophenyl)-8-(trifluoromethyl)-1,4,5,6-tetrahydropyrrolo[2,3-g]indazole ( compound D101)

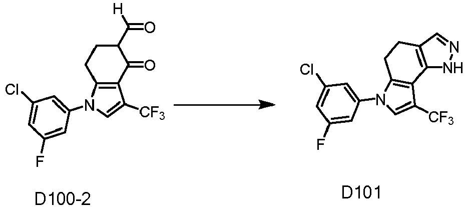

### Step 1: Synthesis of compound D101

Compound D100-2 (100 mg) was dissolved in acetic acid (5 mL), hydrazine hydrate (30 uL) was added, and the mixture was stirred at room temperature for 5 hours. The reaction solution was poured into water, extracted with ethyl acetate, washed twice with saturated brine, dried, concentrated to obtain the crude product, which was purified by Pre-TLC (EA/PE=1:3) to obtain the target compound D101 (30 mg). ESI-MS m/z: 356.12[M+H]⁺.

### ¹H NMR data for some exemplary compounds are shown below:

¹H NMR (500 MHz, DMSO-*d*₆) δ 8.13-8.11 (m, 1H), 7.88-7.85 (m, 1H), 7.74 - 7.62 (m, 1H), 7.26 (s, 1H), 5.89 (d, *J* = 6.4 Hz, 1H), 5.76 (s, 2H),4.31-4.27 (m, 1H), 2.84 - 2.77 (m, 1H), 2.80 - 2.74 (m, 1H), 2.39-2.32 (m, 1H), 2.12 (s, 1H) (Example 5).
¹H NMR (500 MHz, CDCl₃) δ 7.18 (d, J = 8.6 Hz, 1H), 7.13 (s, 2H), 6.96 (d, J = 8.6 Hz, 1H), 4.94 - 4.88 (m, 1H), 2.85 - 2.78 (m, 1H), 2.74 - 2.67 (m, 1H), 2.63 - 2.40 (m, 2H), 2.26 - 2.16 (m, 1H) (Example 7).
¹H NMR (500 MHz, DMSO-*d₆*) δ 7.67 (s, 1H), 7.42 - 7.30 (m, 3H), 6.07 (d, J = 6.7 Hz, 1H), 4.59 (q, J = 6.6 Hz, 1H), 2.91 - 2.69 (m, 2H), 2.44 - 2.23 (m, 1H), 2.17 - 2.12 (m, 1H) (Example 8).
¹H NMR (500 MHz, DMSO-*d₆*) δ 7.59-7.42 (m, 6H), 6.01 (d, J = 6.8 Hz, 1H),4.59(q, J = 6.9 Hz, 1H), 2.81-2.60 (m, 2H), 2.46-2.29 (m, 1H), 2.16-2.09 (m, 1H) (Example 9).
¹H NMR (500 MHz, CDCl₃) δ 7.41 - 7.34 (m, 2H), 7.25 (dt, J = 8.6, 2.1 Hz, 1H), 7.09 (s, 1H), 4.88 - 4.82(m, 1H), 2.80 - 2.70 (m, 1H), 2.66 - 2.59 (m, 1H), 2.55 (d, J = 2.5 Hz, 1H), 2.51 - 2.12 (m, 2H) (Example 12).
¹H NMR (500 MHz, DMSO-*d₆*) δ 7.96 (s, 1H), 7.62 - 7.47 (m, 3H), 3.36 (s, 3H), 3.09 (t, J = 6.0 Hz, 2H), 2.69 - 2.58 (m, 2H) (Example 15).
¹H NMR (500 MHz, DMSO) δ7.33 - 7.17 (m, 3H), 7.07 (d, *J* = 2.9 Hz, 1H), 6.23 (d, *J* = 3.0 Hz, 1H), 5.69 (d, *J* = 6.6 Hz, 1H), 4.61 - 4.53 (m, 1H), 2.92 - 2.75 (m, 2H), 2.41 - 2.24 (m, 1H), 2.20 - 2.08 (m, 1H) (Example 34).
¹H NMR (500 MHz, DMSO-*d*₆) δ 8.14-8.13 (m, 1H), 7.91-7.89 (m, 1H), 7.69 (t, *J* = 9.0 Hz, 1H), 7.52 (s, 1H), 4.78 (d, *J* = 6.3 Hz, 1H), 4.70-4.68 (m, 1H), 2.50 - 2.38 (m, 1H), 1.92 - 1.85 (m, 1H), 1.82 - 1.68 (m, 2H), 1.71 - 1.63 (m, 1H) (Example 46).
¹H NMR (500 MHz, DMSO-*d*₆) δ 7.53 - 7.45 (m, 3H), 7.43 - 7.36 (m, 2H), 7.33 (d, *J* = 3.5 Hz, 1H), 7.08 (dd, *J* = 5.1, 3.5 Hz, 1H), 6.01 (d, *J* = 6.5 Hz, 1H), 5.75 (s, 1H), 4.60 (d, *J* = 6.2 Hz, 1H), 2.90 (ddd, *J* = 17.0, 11.3, 6.3 Hz, 1H), 2.80 (dd, *J* = 16.2, 6.4 Hz, 1H), 2.19 - 2.11 (m, 1H) (Example 48).
¹H NMR (500 MHz, DMSO-*d*₆) δ 7.53 - 7.45 (m, 3H), 7.43 - 7.36 (m, 2H), 7.33 (d, *J* = 3.5 Hz, 1H), 7.08 (dd, *J* = 5.1, 3.5 Hz, 1H), 6.01 (d, *J* = 6.5 Hz, 1H), 5.75 (s, 1H), 4.60 (d, *J* = 6.2 Hz, 1H), 2.90 (ddd, *J* = 17.0, 11.3, 6.3 Hz, 1H), 2.80 (dd, *J* = 16.2, 6.4 Hz, 1H), 2.19 - 2.11 (m, 1H) (Example 59).
¹H NMR (500 MHz, DMSO-*d₆*) δ 7.67 (s, 1H), 7.43 - 7.36 (m, 3H), 6.07 (d, J = 6.7 Hz, 1H), 4.67 - 4.52 (m, 1H), 2.93 - 2.69 (m, 2H), 2.44 - 2.27 (m, 1H), 2.19 - 2.10 (m, 1H) (Example 64).
¹H NMR (500 MHz, DMSO) δ 8.30 - 8.24 (m, 1H), 8.04 - 7.98 (m, 1H), 7.81 - 7.68 (m, 2H), 6.13 (d, *J* = 6.6 Hz, 1H), 4.70 - 4.60 (m, 1H), 2.89 - 2.70 (m, 2H), 2.50 - 2.31 (m, 1H), 2.25 - 2.13 (m, 1H) (Example 95).
¹H NMR (500 MHz, DMSO) δ 7.75 - 7.58 (m, 3H), 6.22 (d, *J* = 6.4 Hz, 1H), 4.58 - 4.50 (m, 1H), 2.70 - 2.52 (m, 2H), 2.43 - 2.24 (m, 1H), 2.20 - 2.10 (m, 1H) (Example 106).
¹H NMR (500 MHz, DMSO-*d*₆) δ 7.69 (s, 1H), 7.62 - 7.54 (m, 3H), 6.37 (d, *J* = 6.1 Hz, 1H), 4.82 (q, *J* = 6.5 Hz, 1H), 3.24 (s, 3H), 2.85 - 2.79 (m, 1H), 2.75-2.70 (m, 1H), 2.29 (d, *J* = 8.9 Hz, 1H), 2.16 (s, 1H) (Example 109).
¹H NMR (500 MHz, DMSO-*d*₆) δ 7.68 (d, *J* = 1.6 Hz, 1H), 7.60 - 7.40 (m, 3H), 6.07 (d, *J* = 6.6 Hz, 1H), 4.59 (q, *J* = 6.8 Hz, 1H), 2.88 - 2.81 (m, 1H), 2.84 - 2.70 (m, 2H), 2.42 - 2.38 (m, 1H), 2.17 - 2.12 (m, 1H) (Example 115).
¹H NMR (500 MHz, DMSO-*d₆*) δ 7.96 (s, 1H), 7.62 - 7.47 (m, 3H), 3.36 (s, 3H), 3.09 (t, J = 6.0 Hz, 2H), 2.69 - 2.58 (m, 2H) (Example 118).
¹H NMR (500 MHz, DMSO) δ 7.88- 7.84(m, 1H), 7.67 - 7.48 (m, 3H), 6.03 (d, *J* = 7.0 Hz, 1H), 4.62 - 4.55 (m, 1H), 2.84 - 2.62 (m, 2H), 2.44 - 2.26 (m, 1H), 2.20 - 2.10 (m, 1H) (Example 120).
¹H NMR (500 MHz, DMSO) δ 7.88- 7.84(m, 1H), 7.67 - 7.48 (m, 3H), 6.03 (d, *J* = 7.0 Hz, 1H), 4.62 - 4.55 (m, 1H), 2.84 - 2.62 (m, 2H), 2.44 - 2.26 (m, 1H), 2.20 - 2.10 (m, 1H) (Example 124).
¹H NMR (500 MHz, DMSO) δ 7.83 - 7.70 (m, 1H), 7.65 - 7.55 (m, 2H), 7.36 - 7.42 (m, 1H), 6.05 (d, *J* = 6.6 Hz, 1H), 4.52 - 4.55 (m, 1H), 2.80 - 2.60 (m 2H), 2.45 - 2.26 (m, 1H), 2.20 - 2.05 (m, 1H) (Example 137).
¹H NMR (500 MHz, DMSO) δ 7.59 - 7.49 (m, 3H), 7.37 (t, *J* = 8.7 Hz, 2H), 6.01 (d, *J* = 6.8 Hz, 1H), 4.62 - 4.53 (m, 1H), 2.76 - 2.59 (m, 2H), 2.44 - 2.27 (m, 1H), 2.18 - 2.08 (m, 1H) (Example 160).
¹H NMR (500 MHz, DMSO) δ 7.83 - 7.70 (m, 1H), 7.65 - 7.55 (m, 2H), 7.36 - 7.42 (m, 1H), 6.05 (d, *J* = 6.6 Hz, 1H), 4.52 - 4.55 (m, 1H), 2.80 - 2.60 (m 2H), 2.45 - 2.26 (m, 1H), 2.20 - 2.05 (m, 1H) (Example 164).
¹H NMR (500 MHz, CDCl₃) δ 7.55 (m, 7.56-7.54, 1H), 7.43-7.40 (m, 1H), 7.30-7.28 (m, 1H), 7.12 (d, *J* = 1.4 Hz, 1H), 7.04-6.82 (m, 1H), 4.93 (q, *J* = 5.4 Hz, 1H), 2.78 - 2.72 (m, 1H), 2.66 - 2.61(m, 1H), 2.55 - 2.42 (m, 2H), 2.25 - 2.17 (m, 1H) (Example 168).
¹H NMR (500 MHz, DMSO-*d*₆) δ 9.10 (d, *J* = 1.8 Hz, 1H), 9.05 (d, *J* = 2.5 Hz, 1H), 8.63 (t, *J* = 2.2 Hz, 1H), 7.77 (d, *J* = 1.6 Hz, 1H), 6.11 (d, *J* = 6.7 Hz, 1H), 4.60 (q, *J* = 6.7 Hz, 1H), 2.86-2.72 (m, 2H), 2.16 (s, 1H) (Example 177).
¹H NMR (500 MHz, CDCl₃) δ 7.53 - 7.45 (m, 2H), 7.32 (t, *J* = 8.4 Hz, 1H), 7.04 (d, *J* = 1.4 Hz, 1H), 4.92 (q, *J* = 9.8 Hz, 1H), 2.55 - 2.43 (m, 2H), 2.38-2.28(m, 1H), 2.24 (dd, *J* = 8.4, 2.3 Hz, 1H), 0.02 (d, *J* =34.9 Hz, 3H) (Example 179).
¹H NMR (500 MHz, CDCl₃) δ 7.42 - 7.35 (m, 5H), 7.34-7.29 (m, 1H), 7.13 (d, *J* = 14.4 Hz, 1H), 6.69 (d, *J* = 14.4 Hz, 1H), 4.88 (t, *J* = 6.6 Hz, 1H), 2.96 - 2.80 (m, 2H), 2.63 - 2.46 (m, 1H), 2.45 - 2.41 (m, 1H), 2.32-2.24 (m, 1H) (Example 181).
¹H NMR (500 MHz, DMSO-*d₆*) δ 8.30-8.28 (m, 1H), 8.04-8.02 (m, 1H), 7.82 (d, J = 1.6 Hz, 1H), 7.74 (t, J = 9.0 Hz, 1H), 5.71-5.61 (m, 1H), 2.92 - 2.76 (m, 2H), 2.43 - 2.36 (m, 2H) (Example 196).

### Pharmacological experiments

### VEGFA ELISA assay (IC₅₀)

786-O cells in exponential growth phase were seeded in 96-well plate, the cell concentration was 65,000 cells per milliliter of culture medium, 180 ul per well. Compounds were serial diluted to the indicated concentration, and 20 ul compound solutions of different concentrations were added to the corresponding cell wells. The final compound concentrations were as follows (nM): 1.5, 4.6, 13.7, 41.2, 123.5, 370.4, 1111.1, 3333.3, 10000. After 24 hours of incubation, the cell culture supernatant was taken, and the VEGFA concentration was determined using an ELISA kit (purchased from abcam). Finally, the reaction was terminated, and the light absorbance value of each well was measured using a microplate reader at a wavelength of 450 nm, and IC₅₀ was calculated by GraphPad Prism. CellTiter-Glo reagent was used to measure cell viability.

The IC₅₀ data of the exemplary Example are provided in Table 1, wherein A means IC₅₀≤0.5µM; B means 0.5 µM<IC₅₀≤ 1.5µM; C means 1.5µM<IC₅₀<10µM, D means IC₅₀ > 1.5 µM.

**Table 1**

| **Example No.** | **IC₅₀ (µM)** | **Example No.** | **IC₅₀ (µM)** |
|---|---|---|---|
| 5 | B | 109 | A |
| 6 | B | 116 | 0.0277 |
| 7 | 0.0307 | 120 | 0.0389 |
| 12 | 0.0675 | 121 | 0.031 |
| 13 | A | 122 | 0.0426 |
| 15 | 0.0188 | 124 | 0.0085 |
| 19 | A | 125 | 0.034 |
| 20 | A | 130 | A |
| 22 | 0.0554 | 131 | A |
| 24 | A | 132 | A |
| 29 | C | 133 | B |
| 37 | A | 134 | A |
| 41 | B | 137 | 0.0421 |
| 48 | 0.0456 | 139 | A |
| 51 | B | 141 | A |
| 53 | A | 142 | A |
| 54 | B | 145 | C |
| 64 | A | 148 | A |
| 68 | A | 154 | A |
| 69 | 0.0442 | 156 | 0.0156 |
| 70 | 0.0454 | 160 | 0.0461 |
| 71 | 0.0138 | 164 | A |
| 75 | A | 167 | A |
| 76 | A | 168 | 0.0084 |
| 77 | 0.0162 | 170 | B |
| 88 | D | 173 | 0.0178 |
| 90 | D | 174 | B |
| 92 | D | 178 | A |
| 93 | D | 179 | B |
| 95 | 0.0145 | 180 | B |
| 96 | A | 181 | C |
| 97 | A | 190 | B |
| 100 | A | 193 | A |
| 103 | 0.0441 | 196 | A |
| 104 | 0.0062 | 197 | A |
| 106 | A | 200 | 0.0056 |
| 107 | A | 201 | D |
| 108 | A | 202 | D |

### Luciferase assay

Luciferase LUC gene was transfected into 786-O cells (purchased from ATCC) using Lipofectamine 3000 Transfection Reagent (purchased from Invitrogen) to construct HIF2α reporter cells (786-O-HIF2α-Luc cells). Experiments were performed when 786-O-HIF2α-Luc cells were in logarithmic growth phase, culture medium (RPMI MEDIUM 1640, purchased from Invitrogen) was abandoned, the cells were rinsed with PBS three times; trypsin (TrypLE, purchased from invitrogen) was added to digest cells, the digestion was terminated by washing the cells with culture medium, 10% fetal bovine serum, 1% penicillin and streptomycin. The cells were collected by centrifugation, rinsed twice with PBS to remove the phenol red in the medium, and then resuspended the cells to an appropriate concentration, and detected the cell density and viability, and made sure the cell viability is above 90% before it can be used for experiments.

The gradient concentration compound was transferred into 384-well plate, 25nl/well using Echo550 (non-contact sonic pipetting system, purchased from Labcyte); the cells were seeded in 384-well plate, 4500 cells/well, with 25 µl of culture medium, to make the final concentrations as follows: 10000, 3333, 1111, 370, 123, 41.1, 13.7, 4.6, 1.5, 0.5 nM. Cells were incubated at 37°C, 5% CO₂ for 18-20h; Steady-Glo^{™} Luciferase Assay System (purchased from Promega) was added to 384 well-plate, 25µl/well; the luminescence value was detected with Envision. The % inhibition rate was calculated according to the RLU (Record Luminescence) signal value of each well, and then the IC₅₀ of the corresponding compound was calculated by Graphpad 8.0.

The IC₅₀ data of the exemplary Example are provided in Table 2, wherein A means IC₅₀≤0.5µM; B means 0.5 µM<IC₅₀≤ 1.5µM; C means 1.5µM<IC₅₀≤10µM, D means IC₅₀ > 1.5µM.

**Table 2**

| **Example No.** | **IC₅₀ (µM)** | **Example No.** | **IC₅₀ (µM)** |
|---|---|---|---|
| 7 | 0.0577 | 106 | A |
| 12 | A | 116 | 0.0532 |
| 15 | A | 120 | 0.0757 |
| 19 | A | 121 | 0.0985 |
| 20 | A | 124 | A |
| 37 | A | 125 | A |
| 48 | A | 131 | A |
| 64 | A | 148 | A |
| 69 | 0.067 | 156 | 0.0705 |
| 70 | 0.0842 | 167 | 0.0699 |
| 75 | A | 168 | 0.0305 |
| 95 | 0.0376 | 173 | 0.0159 |
| 103 | A | 196 | A |
| 104 | 0.0220 | 200 | 0.0335 |

### Pharmacokinetics tset in vivo

Compound was dissolved in 5% DMSO, 5% Solutoland 90% NaCl. SD rats and Balb/c mice were selected as animals for administration, the intravenous dose was 1 mg/kg, the oral dose was 5 mg/kg, orbital blood was collected at 5min, 15min, 30min, 1h, 2h, 4h, 7h, and 24h, respectively. After blood collection, supernatant was collected by centrifugation at 4000rpm for 10 min, 200 µL internal standard solutions were added to 30 µL supernatant for precipitation, after vortexing, supernatant was collected by centrifugation at 12,000 rpm for 10 min, 100 µL supernatant solutions and purified water were mixed at a ratio of 1:1 and injected. The compound concentration in plasma was detected by high performance liquid chromatography-mass spectrometry, and the compound concentration in plasma samples was quantitatively analyzed by internal standard method. After the compound concentration was measured, relevant pharmacokinetic parameters including Cmax, AUC, etc. were calculated by Winnonln software. Experiments have found that the exemplary compounds of the present invention have better PK properties in vivo.

## Claims

1. A compound of formula (I), or a stereoisomer, tautomer, pharmaceutically acceptable salt, prodrug, chelate, non-covalent complex, or solvate thereof, 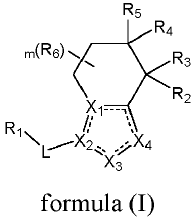 wherein,
-̅ -̅ -̅ -̅ -̅ -̅ represents a single bond or a double bond;
L is a bond, -O-, -NH-, -(CR_{d}Rₑ)ₙ-, -S-, -S(=O)-, -S(=O)₂-, -C=C-, -C≡C- or C₃-C₅ cycloalkyl;
X₁ and X₂ are each independently selected from C or N;
X₃ is CR_{f} or NR_{f};
X₄ is CRⱼ or NRⱼ; and at least one of X₁, X₂, X₃ or X₄ is N;
R₁ is selected from C₁-C₁₀ alkyl, C₂-C₁₀ alkenyl, C₂-C₁₀ alkynyl, C₁-C₁₀ alkoxyl, C₆-C₁₀ aryl, 5-18 membered heteroaryl, C₃-C₁₀ cycloalkyl, or 3-10 membered heterocyclyl; wherein, the 5-18 membered heteroaryl and 3-10 membered heterocyclyl optionally comprising 1, 2 or 3 hetero atoms independently selected from N, O or S; the C₁-C₁₀ alkyl, C₂-C₁₀ alkenyl, C₂-C₁₀ alkynyl, C₁-C₁₀ alkoxyl, C₆-C₁₀ aryl, 5-18 membered heteroaryl, C₃-C₁₀ cycloalkyl and 3-10 membered heterocyclyl are each optionally substituted with one or more halogen, -OH, -CN, oxo, amino, C₁-C₆ alkyl, C₁-C₆ alkoxyl, C₂-C₆ alkenyl, C₃-C₅ cycloalkyl, C₂-C₆ alkynyl, C₁-C₆ haloalkyl, -C₁-C₆ alkylene-OR_{c}, -C₀-C₆ alkylene-C=O-R_{c}, -NO₂,-OR_{c}, -SR_{c}, -NRₐR_{b}, -C(=O)R_{c}, -C(=O)OR_{c}, -C(=O)NRₐR_{b}, -NC(=O)R_{c},-S(=O)R_{c}, -S(=O)₂R_{C}, -S(=O)₂NRₐR_{b}, -S(=O)(=NRₐ)R_{b}, -P(=O)RₐR_{b} and/or-P(=S)RₐR_{b};
R₂ is selected from H, deuterium, halogen, -CN, -OH, =N-OH, C₁-C₅ haloalkyl, amino, C₁-C₁₀ alkoxyl, -O-C(=O)-C₁₋₃ alkyl, -C(=O)-O-C₁₋₃alkyl or -NRₐR_{b};
R₃ is selected from H, deuterium, halogen, -CN, -OH, =N-OH, C₁-C₁₀ alkyl, C₂-C₁₀ alkenyl, C₁-C₅ haloalkyl, C₁-C₁₀ alkoxyl, -O-C(=O)-C₁₋₃ alkyl, -C(=O)-O-C₁₋₃ alkyl or C₂-C₁₀ alkynyl, the C₁-C₁₀ alkyl, C₂-C₁₀ alkenyl, C₁-C₅ haloalkyl, C₁-C₁₀ alkoxyl, -O-C(=O)-C₁₋₃ alkyl, -C(=O)-O-C₁₋₃ alkyl are each optionally substituted with one or more H, halogen, -CN, -OH, amino, C₁-C₅ alkyl, C₂-C₆ alkenyl and/or C₁-C₅ haloalkyl; or R₂ and R₃ together form an oxo group;
R₄ and R₅ are each independently selected from H, halogen, -OH, C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl or 3-6 membered heterocyclyl; the C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl and 3-6 membered heterocyclyl are each optionally substituted with one or more halogen, -CN, -OH, amino, C₁-C₅ alkyl, C₂-C₆ alkenyl and/or C₁-C₅ haloalkyl; or R₄ and R₅ together with the C atom to which they are attached form a substituted or unsubstituted cyclopropyl;
R₆ is selected from H, -CN, halogen, -OH, -NO₂, -NH₂, oxo, C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl or 3-6 membered heterocyclyl; the C₁-C₆ alkyl, C₁-C₆ alkoxyl, C₁-C₆ haloalkyl, C₂-C₁₀ alkenyl, C₂-C₁₀ alkynyl, C₃-C₅ cycloalkyl and 3-6 membered heterocyclyl are each optionally substituted with one or more H, halogen, -CN, -OH, amino, oxo, C₁-C₅ alkyl, C₂-C₆ alkenyl and/or C₁-C₅ haloalkyl; or two R₆ together with the C atom to which they are attached form a substituted or unsubstituted C₃-C₅ cycloalkyl or 3-5 membered heterocyclyl;
or R₆ and R₅ together with the C atom to which they are attached form a substituted or unsubstituted C₃-C₄ cycloalkyl;
R_{d} and Rₑ are each independently selected from H, halogen, CN, -NRₐR_{b}, C₁-C₁₀ alkyl and C₃-C₁₀ cycloalkyl; or R_{d} and Rₑ together form an oxo group;
R_{f} is selected from absent, H, -CN, halogen, C₁-C₁₀ alkyl, C₁-C₁₀ haloalkyl, C₃-C₁₀ cycloalkyl, oxo, or -NRₐR_{b}; the C₁-C₁₀ alkyl, C₁-C₁₀ haloalkyl and C₃-C₁₀ cycloalkyl are each optionally substituted with one or more H, halogen, -CN,-OH, amino, C₁-C₅ alkyl, C₂-C₆ alkenyl and/or C₁-C₅ haloalkyl;
Rⱼ is selected from H, -NO₂, -CN, halogen, C₁-C₁₀ alkyl, C₂-C₁₀ alkenyl, C₂-C₁₀ alkynyl, C₁-C₁₀ haloalkyl, C₃-C₁₀ cycloalkyl, 3-10 membered heterocyclyl, C₆-C₁₀ aryl, 5-10 membered heteroaryl, -C₁-C₆ alkylene-OR_{c}, -OR_{c}, -SR_{c}, -NRₐR_{b},-C(=O)R_{c}, -C(=O)OR_{c}, -C(=O)NRₐR_{b}, -NC(=O)R_{c}, -S(=O)R_{c}, -S(=O)₂R_{c},-S(=O)₂NRₐR_{b}, -S(=O)(=NRₐ)R_{b}, P(=O)RₐR_{b}, or P(=S)RₐR_{b}; wherein, the 5-10 membered heteroaryl and 3-10 membered heterocyclyl optionally comprising 1, 2 or 3 heteroatoms independently selected from N, O or S; the C₁-C₁₀ alkyl, C₂-C₁₀ alkenyl, C₂-C₁₀ alkynyl, C₁-C₁₀ haloalkyl, C₃-C₁₀ cycloalkyl, 3-10 membered heterocyclyl, C₆-C₁₀ aryl and 5-10 membered heteroaryl are each optionally substituted with one or more halogen, hydroxyl, amino, P(=O)Me₂, P(=S)Me₂,-S(=O)₂-C₁-C₃ alkyl, -S(=O)₂-C₃-C₅ cycloalkyl, -S(=O)-C₁-C₃ alkyl, -S(=O)-C₃-C₅ cycloalkyl, CN, C₁-C₆ alkyl, C₁-C₆ alkoxyl and/or C₁-C₆ haloalkyl;
Rₐ, R_{b} and R_{c} are each independently selected from H, C₁-C₁₀ alkyl, C₁-C₁₀ haloalkyl, C₂-C₁₀ alkenyl, C₂-C₁₀ alkynyl, C₃-C₁₀ cycloalkyl, 3-10 membered heterocyclyl, C₆-C₁₀ aryl or 5-10 membered heteroaryl;
n is 1, 2 or 3;
m is 0, 1, 2, 3 or 4.

2. The compound, or the stereoisomer, tautomer, pharmaceutically acceptable salt, prodrug, chelate, non-covalent complex, or solvate of claim 1, wherein, the compound is of formula (II): 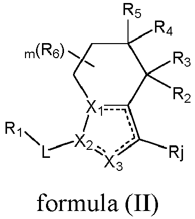 wherein, one and only one of X₁ or X₂ is N, and the other is C.

3. The compound, or the stereoisomer, tautomer, pharmaceutically acceptable salt, prodrug, chelate, non-covalent complex, or solvate of claim 1 or claim 2, wherein, the R₂ is selected from halogen, -CN, -OH, or =N-OH.

4. The compound or the stereoisomer, tautomer, pharmaceutically acceptable salt, prodrug, chelate, non-covalent complex, or solvate of any one of claims 1-3, wherein, the R₃ is selected from H, deuterium, C₁-C₁₀ alkyl, or C₂-C₁₀ alkenyl, the C₁-C₁₀ alkyl and C₂-C₁₀ alkenyl are each optionally substituted with H, halogen, -CN, -OH, amino, or C₁-C₅ haloalkyl; or R₂ and R₃ together form an oxo group.

5. The compound or the stereoisomer, tautomer, pharmaceutically acceptable salt, prodrug, chelate, non-covalent complex, or solvate of any one of claims 1-4, wherein, the R₃ is selected from H, deuterium, or C₁-C₃ alkyl, the C₁-C₃ alkyl and C₂-C₅ alkenyl are each optionally substituted with H, halogen, -CN, -OH, amino, or C₁-C₅ haloalkyl; or R₂ and R₃ together form an oxo group.

6. The compound or the stereoisomer, tautomer, pharmaceutically acceptable salt, prodrug, chelate, non-covalent complex, or solvate of any one of claims 1-5, wherein, R₄ and R₅ are each independently selected from H, halogen or C₁-C₆ alkyl, the C₁-C₆ alkyl is optionally substituted with H, halogen, -CN, -OH, amino, or oxo.

7. The compound or the stereoisomer, tautomer, pharmaceutically acceptable salt, prodrug, chelate, non-covalent complex, or solvate of any one of claims 1-6, wherein, R₄ and R₅ are each independently selected from H or halogen.

8. The compound or the stereoisomer, tautomer, pharmaceutically acceptable salt, prodrug, chelate, non-covalent complex, or solvate of any one of claims 1-7, wherein, the compound is of formula (IV-1) or formula (IV-2), 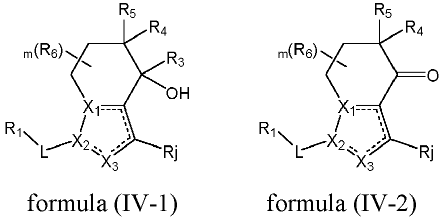 wherein, one and only one of X₁ or X₂ is N, and the other is C.

9. The compound or the stereoisomer, tautomer, pharmaceutically acceptable salt, prodrug, chelate, non-covalent complex, or solvate of any one of claims 1-8, wherein, the compound is of formula (V), 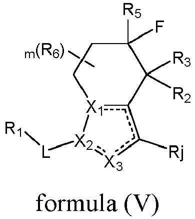 wherein, one and only one of X₁ or X₂ is N, and the other is C.

10. The compound or the stereoisomer, tautomer, pharmaceutically acceptable salt, prodrug, chelate, non-covalent complex, or solvate of any one of claims 1-9, wherein, L is a bond, -CH₂-, -S(=O)₂-, -C=C-, -C=O-, -C≡C- or -C₃-C₅ cycloalkyl.

11. The compound or the stereoisomer, tautomer, pharmaceutically acceptable salt, prodrug, chelate, non-covalent complex, or solvate of any one of claims 1-10, wherein, X₃ is CR_{f}.

12. The compound or the stereoisomer, tautomer, pharmaceutically acceptable salt, prodrug, chelate, non-covalent complex, or solvate of any one of claims 1-11, wherein, L is a bond.

13. The compound or the stereoisomer, tautomer, pharmaceutically acceptable salt, prodrug, chelate, non-covalent complex, or solvate of any one of claims 1-12, wherein, the compound is of formula (VI-1), formula (VI-2), formula (VI-3), formula (VI-4) or formula (VI-5): 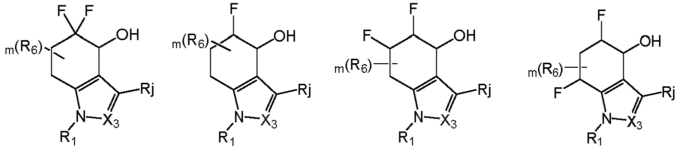 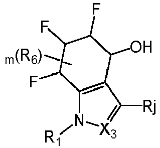 formula (VI-1) formula (VI-2) formula (VI-3) formula (VI-4) formula (VI-5).

14. The compound or the stereoisomer, tautomer, pharmaceutically acceptable salt, prodrug, chelate, non-covalent complex, or solvate of any one of claims 1-13, wherein, R₆ is H, -CN, halogen, C₁-C₆ alkyl, C₁-C₆ alkoxyl or C₁-C₆ haloalkyl, the C₁-C₆ alkyl, C₁-C₆ alkoxyl and C₁-C₆ haloalkyl are each optionally substituted with one or more H, halogen, -CN, -OH, oxo and/or amino.

15. The compound or the stereoisomer, tautomer, pharmaceutically acceptable salt, prodrug, chelate, non-covalent complex, or solvate of any one of claims 1-14, wherein, R₆ is H, halogen or C₁-C₆ alkyl, the C₁-C₆ alkyl is optionally substituted with one or more H, halogen, -CN, -OH, oxo and/or amino.

16. The compound or the stereoisomer, tautomer, pharmaceutically acceptable salt, prodrug, chelate, non-covalent complex, or solvate of any one of claims 1-15, wherein, the R_{f} is selected from H, -CN, halogen, -NH₂, C₁-C₃ alkyl, cyclopropyl or C₁-C₃ haloalkyl, the C₁-C₃ alkyl, cyclopropyl or C₁-C₃ haloalkyl are each optionally substituted with one or more H, halogen, -CN, -OH, amino, C₁-C₃ alkyl, C₂-C₄ alkenyl and/or C₁-C₃ haloalkyl.

17. The compound or the stereoisomer, tautomer, pharmaceutically acceptable salt, prodrug, chelate, non-covalent complex, or solvate of any one of claims 1-16, wherein, R_{f} is selected from H, -CN, halogen, C₁-C₃ alkyl or C₁-C₃ haloalkyl.

18. The compound or the stereoisomer, tautomer, pharmaceutically acceptable salt, prodrug, chelate, non-covalent complex, or solvate of any one of claims 1-17, wherein, R₁ is C₆-C₁₀ aryl, 5-18 membered heteroaryl or C₃-C₁₀ cycloalkyl; the 5-18 membered heteroaryl optionally comprising 1, 2 or 3 heteroatoms independently selected from N, O or S.

19. The compound or the stereoisomer, tautomer, pharmaceutically acceptable salt, prodrug, chelate, non-covalent complex, or solvate of any one of claims 1-18, wherein, R₁ is C₆-C₈ aryl, 5-8-membered heteroaryl or C₃-C₆ cycloalkyl, the 5-8-membered heteroaryl optionally comprising 1, 2 or 3 heteroatoms independently selected from N, O or S, the C₆-C₈ aryl, 5-8 membered heteroaryl or C₃-C₆ cycloalkyl are each optionally substituted with one or more halogen, -OH, -CN, oxo, amino, C₁-C₆ alkyl, C₁-C₆ alkoxyl, C₁-C₆ haloalkyl, -C₁-C₆ alkylene-OR_{c}, -C₀-C₆ alkylene-C=O-R_{c}, -NO₂, C(=O)OR_{c} and/or -S(=O)₂R_{c}.

20. The compound or the stereoisomer, tautomer, pharmaceutically acceptable salt, prodrug, chelate, non-covalent complex, or solvate of any one of claims 1-19, wherein, R₁ is phenyl, 5-6 membered heteroaryl or C₃-C₆cycloalkyl, the 5-6 membered heteroaryl optionally comprising 1, 2 or 3 heteroatoms independently selected from N, O or S; the phenyl, 5-6 membered heteroaryl and C₃-C₆ cycloalkyl are each optionally substituted with one or more halogen, -CN and/or C₁-C₄ haloalkyl.

21. The compound or the stereoisomer, tautomer, pharmaceutically acceptable salt, prodrug, chelate, non-covalent complex, or solvate of any one of claims 1-20, wherein, the Rⱼ is selected from H, -CN, halogen, C₁-C₁₀ alkyl, C₂-C₁₀ alkenyl, C₁-C₁₀ haloalkyl, C₃-C₁₀ cycloalkyl, C₆-C₁₀ aryl, 5-10 membered heteroaryl, - S(=O)R_{c}, -C₁-C₆ alkylene-OR_{c}, -S(=O)₂R_{c} or P(=O)RₐR_{b}; the 5-10 membered heteroaryl optionally comprising 1, 2 or 3 heteroatoms independently selected from N, O or S; the C₁-C₁₀ alkyl, C₂-C₁₀ alkenyl, C₁-C₁₀ haloalkyl, C₃-C₁₀ cycloalkyl, C₆-C₁₀ aryl, 5-10 membered heteroaryl are each optionally substituted with one or more halogen, hydroxyl, P(=O)Me₂, P(=S)Me₂, -S(=O)₂-C₁-C₃alkyl, -S(=O)₂-C₃-Cscycloalkyl, -S(=O)-C₁-C₃alkyl, -S(=O)-C₃-C₅cycloalkyl, CN, C₁-C₆ alkyl and/or C₁-C₆ haloalkyl;
the Rₐ and R_{b} independently selected from H, C₁-C₄ alkyl;
the R_{c} is selected from H, C₁-C₃ alkyl or C₁-C₃ haloalkyl.

22. The compound or the stereoisomer, tautomer, pharmaceutically acceptable salt, prodrug, chelate, non-covalent complex, or solvate of any one of claims 1-21, wherein, the Rⱼ is selected from H, halogen, -CN, C₁-C₄ haloalkyl, C₃-C₅ cycloalkyl, 5-membered heteroaryl, S(=O)R_{c}, -S(=O)₂R_{c}, -S(=O)(=NRₐ)R_{b} or P(=O)Me₂, the 5-membered heteroaryl optionally comprising 1, 2 or 3 heteroatoms independently selected from N, O or S; the C₃-C₅ cycloalkyl, 5-membered heteroaryl are each optionally substituted with one or more halogen, hydroxyl, C₁-C₃ alkyl, C₁-C₃ alkoxyl and/or C₁-C₃ haloalkyl;
the Rₐ and R_{b} independently selected from H, C₁-C₄ alkyl;
the R_{c} is selected from H, C₁-C₃ alkyl or C₁-C₃ haloalkyl.

23. The compound or the stereoisomer, tautomer, pharmaceutically acceptable salt, prodrug, chelate, non-covalent complex, or solvate of any one of claims 1-22, wherein, the Rⱼ is selected from C₁-C₄haloalkyl, CN, -S(=O)₂R_{c} or 5-membered heteroaryl, the 5-membered heteroaryl optionally comprising 1, 2 or 3 heteroatoms independently selected from N, O or S; the 5-membered heteroaryl is optionally substituted with one or more halogen, C₁-C₃alkyl and/or C₁-C₃haloalkyl;
the R_{c} is selected from H, C₁-C₃ alkyl or C₁-C₃ haloalkyl.

24. The compound or the stereoisomer, tautomer, pharmaceutically acceptable salt, prodrug, chelate, non-covalent complex, or solvate of claim 1, wherein, the compound is selected from:
1-(3-chloro-5-fluorophenyl)-5,5-difluoro-3-iodo-1,5,6,7-tetrahydro-4*H*-indol-4-one;
1-(3-chloro-5-fluorophenyl)-5,5-difluoro-3-iodo-4,5,6,7-tetrahydro-1*H*-indol-4-ol;
5,5-difluoro-3-iodo-1-(4-(trifluoromethyl)phenyl)-1,5,6,7-tetrahydro-4*H*-indol-4-one;
5,5-difluoro-3-iodo-1-(4-(trifluoromethyl)phenyl)-4,5,6,7-tetrahydro-1*H*-indol-4-ol;
5-(5,5-difluoro-4-hydroxy-3-iodo-4,5,6,7-tetrahydro-1H-indol-1-yl)-2-fluorobenzonitrile;
1-(3-chloro-5-fluorophenyl)-5,5-difluoro-3-(trifluoromethyl)-1,5,6,7-tetrahydro-4*H*-indol-4-one;
1-(3-chloro-5-fluorophenyl)-5,5-difluoro-3-(trifluoromethyl)-4,5,6,7-tetrahydro-1*H*-indol-4-ol;
1-(3,5-difluorophenyl)-5,5-difluoro-3-(trifluoromethyl)-4,5,6,7-tetrahydro-1*H-*indol-4-ol;
5,5-difluoro-1-phenyl-3-(trifluoromethyl)-4,5,6,7-tetrahydro-1*H*-indol-4-ol;
1-(3,5-difluorophenyl)-5,5-difluoro-3-(trifluoromethyl)-1,5,6,7-tetrahydro-4*H-*indol-4-one;
5,5-difluoro-1-phenyl-3-(trifluoromethyl)-1,5,6,7-tetrahydro-4H-indol-4-one;
3-(5,5-difluoro-4-hydroxy-3-(trifluoromethyl)-4,5,6,7-tetrahydro-1*H*-indol-1-yl)-5-fluorobenzonitrile;
4-(5,5-difluoro-4-hydroxy-3-(trifluoromethyl)-4,5,6,7-tetrahydro-1*H*-indol-1-yl)benzonitrile;
5,5-difluoro-1-(4-fluoro-3-methoxyphenyl)-3-(trifluoromethyl)-4,5,6,7-tetrahydro-1*H*-indol-4-ol;
1-(3-chlorophenyl)-5,5-difluoro-3-(trifluoromethyl)-4,5,6,7-tetrahydro-1*H*-indol-4-ol;
5,5-difluoro-1-(3-fluorophenyl)-3-(trifluoromethyl)-4,5,6,7-tetrahydro-1*H*-indol-4-ol;
1-(5-chloropyridin-3-yl)-5,5-difluoro-3-(trifluoromethyl)-4,5,6,7-tetrahydro-1*H-*indol-4-ol;
5,5-difluoro-1-(2-fluorophenyl)-3-(trifluoromethyl)-4,5,6,7-tetrahydro-1*H*-indol-4-ol;
3-(5,5-difluoro-4-hydroxy-3-(trifluoromethyl)-4,5,6,7-tetrahydro-1*H*-indol-1-yl)benzonitrile;
1-(3, 5-dichlorophenyl)-5, 5-difluoro-3-(trifluoromethyl)-4, 5, 6,7-tetrahydro-1*H-*indol-4-ol;
1-(4-chlorophenyl)-5,5-difluoro-3-(trifluoromethyl)-4,5,6,7-tetrahydro-1*H*-indol-4-ol;
1-(3-bromo-5-fluorophenyl)-5,5-difluoro-3-(trifluoromethyl)-4,5,6,7-tetrahydro-1*H*-indol-4-ol;
5,5-difluoro-1-(pyridin-3-yl)-3-(trifluoromethyl)-4,5,6,7-tetrahydro-1*H*-indol-4-ol;
5,5-difluoro-1-(3-fluoro-5-(trifluoromethyl)phenyl)-3-(trifluoromethyl)-4,5,6,7-tetrahydro-1*H*-indol-4-ol;
5, 5-difluoro-3-(trifluoromethyl)-1-(4-(trifluoromethyl)phenyl)-4, 5,6,7-tetrahydro-1*H*-indol-4-ol;
5,5-difluoro-1-(p-tolyl)-3-(trifluoromethyl)-4,5,6,7-tetrahydro-1*H*-indol-4-ol;
5,5-difluoro-1-(3-fluoro-5-hydroxyphenyl)-3-(trifluoromethyl)-4,5,6,7-tetrahydro-1*H*-indol-4-ol;
1-(3-chloro-5-fluorobenzyl)-5,5-difluoro-3-(trifluoromethyl)-1,5,6,7-tetrahydro-4*H*-indol-4-one;
1-(3-chloro-5-fluorobenzyl)-5,5-difluoro-3-(trifluoromethyl)-4,5,6,7-tetrahydro-1*H*-indol-4-ol;
5,5-difluoro-1-phenyl-3-(trifluoromethyl)-1,5,6,7-tetrahydro-4*H*-indol-4-ol;
1-((3,3-difluorocyclobutyl)methyl)-5,5-difluoro-3-(trifluoromethyl)-4,5,6,7-tetrahydro-1*H*-indol-4-ol;
(5,5-difluoro-4-hydroxy-3-(trifluoromethyl)-4,5,6,7-tetrahydro-1*H*-indol-1-yl)(3,3-difluorocyclobutyl)methanone;
1-(3,5-difluorophenyl)-5,5-difluoro-1,5,6,7-tetrahydro-4*H*-indol-4-one;
1-(3,5-difluorophenyl)-5,5-difluoro-4,5,6,7-tetrahydro-1*H*-indol-4-ol;
1-cyclohexyl-5,5-difluoro-3-(trifluoromethyl)-1,5,6,7-tetrahydro-4*H*-indol-4-one;
1-cyclohexyl-5,5-difluoro-3-(trifluoromethyl)-4,5,6,7-tetrahydro-1*H*-indol-4-ol;
1-(3,5-difluorophenyl)-3-(trifluoromethyl)-4,5,6,7-tetrahydro-1*H*-indol-4-ol;
3-fluoro-5-(3-(methylsulfonyl)-4-oxo-4,5,6,7-tetrahydro-1*H*-indol-1-yl)benzonitrile;
3-fluoro-5-(4-hydroxy-3-(methylsulfonyl)-4,5,6,7-tetrahydro-1*H*-indol-1-yl)benzonitrile;
1-(3,5-difluorophenyl)-4-hydroxy-4,5,6,7-tetrahydro-1*H*-indole-3-carbonitrile;
3-fluoro-5-(4-hydroxy-3-(trifluoromethyl)-4,5,6,7-tetrahydro-1*H*-indol-1-yl)benzonitrile;
1-(3-chloro-5-fluorophenyl)-3-(trifluoromethyl)-1,5,6,7-tetrahydro-4*H*-indol-4-one;
3-fluoro-5-(4-oxo-3-(trifluoromethyl)-4,5,6,7-tetrahydro-1*H*-indol-1-yl)benzonitrile;
1-(3,3-difluorocyclobutyl)-5,5-difluoro-3-(trifluoromethyl)-4,5,6,7-tetrahydro-1*H-*indol-4-ol;
1-(3,3-difluorocyclobutyl)-5,5-difluoro-3-(methylsulfonyl)-4,5,6,7-tetrahydro-1*H-*indol-4-ol;
2-fluoro-5-(4-hydroxy-3-(trifluoromethyl)-4,5,6,7-tetrahydro-1*H*-indol-1-yl)benzonitrile;
1-(3,5-difluorophenyl)-3-(1-methyl-1H-pyrazol-5-yl)-4,5,6,7-tetrahydro-1*H*-indol-4-ol;
(*S*)-1-(3-chloro-5-fluorophenyl)-5,5-difluoro-3-(thiophen-2-yl)-4,5,6,7-tetrahydro-1*H*-indol-4-ol;
1-(4-fluorobenzyl)-3-(1-methyl-1*H*-pyrazol-5-yl)-4,5,6,7-tetrahydro-1*H*-indol-4-ol;
1-(3,5-difluorophenyl)-5,5-difluoro-3-(thiophen-2-yl)-4,5,6,7-tetrahydro-1*H*-indol-4-ol;
1-(3-chloro-5-fluorophenyl)-5,5-difluoro-3-(1-methyl-1*H*-pyrrol-2-yl)-4,5,6,7-tetrahydro-1*H*-indol-4-ol;
1-(3-chloro-5-fluorophenyl)-3-cyclopropyl-5,5-difluoro-4,5,6,7-tetrahydro-1*H-*indol-4-ol;
1-(3-chloro-5-fluorophenyl)-5,5-difluoro-3-(furan-2-yl)-4,5,6,7-tetrahydro-1*H-*indol-4-ol;
5-(5,5-difluoro-4-hydroxy-3-(thiazol-5-yl)-4,5,6,7-tetrahydro-1*H*-indol-1-yl)-2-fluorobenzonitrile;
1-(3-chloro-5-fluorophenyl)-5,5-difluoro-3-(furan-3-yl)-1,5,6,7-tetrahydro-4*H-*indol-4-one;
1-(3-chloro-5-fluorophenyl)-5,5-difluoro-3-(furan-3-yl)-4,5,6,7-tetrahydro-1*H-*indol-4-ol;
1-(3-chloro-5-fluorophenyl)-5,5-difluoro-3-(1*H*-pyrazol-3-yl)-4,5,6,7-tetrahydro-1*H*-indol-4-ol;
1-(3-chloro-5-fluorophenyl)-5,5-difluoro-3-(5-methyl-1*H*-pyrazol-3-yl)-4,5,6,7-tetrahydro-1*H*-indol-4-ol;
1-(3-chloro-5-fluorophenyl)-5,5-difluoro-3-(1-methyl-1*H*-pyrazol-5-yl)-4,5,6,7-tetrahydro-1H-indol-4-ol;
1-(3-chloro-5-fluorophenyl)-5,5-difluoro-3-(thiophen-3-yl)-4,5,6,7-tetrahydro-1*H-*indol-4-ol;
1-(3-chloro-5-fluorophenyl)-5,5-difluoro-3-methyl-4,5,6,7-tetrahydro-1*H*-indol-4-ol;
1-(3-chloro-5-fluorophenyl)-5,5-difluoro-3-(thiazol-4-yl)-4,5,6,7-tetrahydro-1*H-*indol-4-ol;
1-(3-chloro-5-fluorophenyl)-5,5-difluoro-3-(thiazol-5-yl)-4,5,6,7-tetrahydro-1*H-*indol-4-ol;
(*S*)-1-(3,5-difluorophenyl)-5,5-difluoro-3-(trifluoromethyl)-4,5,6,7-tetrahydro-1*H-*indol-4-ol;
1-(3-chloro-5-fluorophenyl)-3-(difluoromethyl)-5,5-difluoro-4,5,6,7-tetrahydro-1*H*-indol-4-ol;
1-(3-chloro-5-fluorophenyl)-5,5-difluoro-3-vinyl-4,5,6,7-tetrahydro-1*H*-indol-4-ol;
1-(3-chloro-5-fluorophenyl)-5,5-difluoro-3-(hydroxymethyl)-4,5,6,7-tetrahydro-1*H*-indol-4-ol;
3-(3-chloro-5-fluorophenyl)-7-fluoro-1-(trifluoromethyl)-5,6,7,8-tetrahydroindolizin-8-ol;
5-(7,7-difluoro-8-hydroxy-1-(trifluoromethyl)-5,6,7,8-tetrahydroindolizin-3-yl)-2-fluorobenzonitrile;
(*S*)-5-(7,7-difluoro-8-hydroxy-1-(trifluoromethyl)-5,6,7,8-tetrahydroindolizin-3-yl)-2-fluorobenzonitrile;
3-(3-chloro-5-fluorophenyl)-7,7-difluoro-1-(trifluoromethyl)-5,6,7,8-tetrahydroindolizin-8-ol;
7-fluoro-3-phenyl-1-(trifluoromethyl)-5,6,7,8-tetrahydroindolizin-8-ol;
3-(3,5-difluorophenyl)-7-fluoro-1-(trifluoromethyl)-5,6,7,8-tetrahydroindolizin-8-ol;
7-fluoro-3-(4-fluoro-3-(trifluoromethyl)phenyl)-1-(trifluoromethyl)-5,6,7,8-tetrahydroindolizin-8-ol;
2-fluoro-5-(7-fluoro-8-hydroxy-1-(trifluoromethyl)-5,6,7,8-tetrahydroindolizin-3-yl)benzonitrile;
3-(3,5-difluorophenyl)-7,7-difluoro-1-(trifluoromethyl)-5,6,7,8-tetrahydroindolizin-8-ol;
3-(7,7-difluoro-8-hydroxy-1-(trifluoromethyl)-5,6,7,8-tetrahydroindolizin-3-yl)-5-fluorobenzonitrile;
(*E*)-3-(2-cyclohexylvinyl)-7,7-difluoro-1-(trifluoromethyl)-5,6,7,8-tetrahydroindolizin-8-ol;
3-(cyclopropylethynyl)-7,7-difluoro-1-(trifluoromethyl)-5,6,7,8-tetrahydroindolizin-8-ol;
3-(3-(difluoromethyl)-4-fluorophenyl)-7,7-difluoro-1-(trifluoromethyl)-5,6,7,8-tetrahydroindolizin-8-ol;
7,7-difluoro-1-(trifluoromethyl)-3-(3,4,5-trifluorophenyl)-5,6,7,8-tetrahydroindolizin-8-ol;
7,7-difluoro-3-(4-fluoro-3-(trifluoromethyl)phenyl)-1-(trifluoromethyl)-5,6,7,8-tetrahydroindolizin-8-ol;
3-(3-(difluoromethyl)-4-fluorophenyl)-7,7-difluoro-1-(trifluoromethyl)-6,7-dihydroindolizin-8(5*H*)-one;
7,7-difluoro-1-(trifluoromethyl)-3-(3,4, 5-trifluorophenyl)-6,7-dihydroindolizin-8(5*H*)-one;
7,7-difluoro-3-(4-fluoro-3-(trifluoromethyl)phenyl)-1-(trifluoromethyl)-6,7-dihydroindolizin-8(5*H*)-one;
7,7-difluoro-3-(1-phenylcyclopropyl)-1-(trifluoromethyl)-5,6,7,8-tetrahydroindolizin-8-ol;
7,7-difluoro-3-(phenylethynyl)-1-(trifluoromethyl)-5,6,7,8-tetrahydroindolizin-8-ol;
(1-(3-chloro-5-fluorophenyl)-5,5-difluoro-4-hydroxy-4,5,6,7-tetrahydro-1*H*-indol-3-yl)dimethylphosphine oxide;
(*S*)-5-(5,5-difluoro-4-hydroxy-3-(trifluoromethyl)-4,5,6,7-tetrahydro-1*H*-indol-1-yl)-2-fluorobenzonitrile;
1-(3-chloro-5-fluorophenyl)-5,5-difluoro-4-hydroxy-4,5,6,7-tetrahydro-1*H*-indole-3-carbonitrile;
1-(3-chloro-5-fluorophenyl)-5,6-difluoro-3-(trifluoromethyl)-4,5,6,7-tetrahydro-1*H*-indol-4-ol;
5,5-difluoro-1-(4-fluoro-3-(hydroxymethyl)phenyl)-3-(trifluoromethyl)-4,5,6,7-tetrahydro-1*H*-indol-4-ol;
5,5-difluoro-1-(6-fluoropyridin-2-yl)-3-(trifluoromethyl)-4,5,6,7-tetrahydro-1*H-*indol-4-ol;
5-(5,5-difluoro-4-hydroxy-4-methyl-3-(trifluoromethyl)-4,5,6,7-tetrahydro-1*H-*indol-1-yl)-2-fluorobenzonitrile;
5-(5,5-difluoro-4-hydroxy-6,6-dimethyl-3-(trifluoromethyl)-4,5,6,7-tetrahydro-1*H-*indol-1-yl)-2-fluorobenzonitrile;
2-fluoro-5-(5-fluoro-4-hydroxy-6,6-dimethyl-3-(trifluoromethyl)-4,5,6,7-tetrahydro-1*H*-indol-1-yl)benzonitrile;
(4*S*,5*S*)-1-(3-(difluoromethyl)-4-fluorophenyl)-5-fluoro-3-(trifluoromethyl)-4,5,6,7-tetrahydro-1*H*-indol-4-ol;
(4*R*,5*R*)-1-(3-(difluoromethyl)-4-fluorophenyl)-5-fluoro-3-(trifluoromethyl)-4,5,6,7-tetrahydro-1*H*-indol-4-ol;
(4*S*,5*R*)-1-(3-(difluoromethyl)-4-fluorophenyl)-5-fluoro-3-(trifluoromethyl)-4,5,6,7-tetrahydro-1*H*-indol-4-ol;
(4*R*,5*S*)-1-(3-(difluoromethyl)-4-fluorophenyl)-5-fluoro-3-(trifluoromethyl)-4,5,6,7-tetrahydro-1*H*-indol-4-ol;
1-(3-chloro-5-fluorophenyl)-2,5,5-trifluoro-3-(trifluoromethyl)-4,5,6,7-tetrahydro-1*H*-indol-4-ol;
(*S*)-2-chloro-1-(3-chloro-5-fluorophenyl)-5,5-difluoro-3-(trifluoromethyl)-4,5,6,7-tetrahydro-1*H*-indol-4-ol;
1-(3-(difluoromethyl)-4-fluorophenyl)-3-(trifluoromethyl)-4, 5, 6,7-tetrahydro-1*H-*indol-4-ol;
1-(3-chloro-5-fluorophenyl)-5,5-difluoro-3-(methylsulfonyl)-4,5,6,7-tetrahydro-1*H*-indol-4-ol;
3-(5,5-difluoro-4-hydroxy-3-(methylsulfonyl)-4,5,6,7-tetrahydro-1*H*-indol-1-yl)-5-fluorobenzonitrile;
3-(3-(difluoromethyl)-5,5-difluoro-4-hydroxy-4,5,6,7-tetrahydro-1*H*-indol-1-yl)-5-fluorobenzonitrile;
3-(3-((difluoromethyl)sulfonyl)-5,5-difluoro-4-hydroxy-4,5,6,7-tetrahydro-1*H-*indol-1-yl)-5-fluorobenzonitrile;
1-(3-chloro-5-fluorophenyl)-3,5,5-trifluoro-4,5,6,7-tetrahydro-1*H*-indol-4-ol;
1-(3-chloro-5-fluorophenyl)-5,5-difluoro-4-hydroxy-3-(trifluoromethyl)-4,5,6,7-tetrahydro-1*H*-indole-2-carbonitrile;
(*R*)-1-(3-chloro-5-fluorophenyl)-5,5-difluoro-3-(trifluoromethyl)-4,5,6,7-tetrahydro-1*H*-indol-4-ol;
(*S*)-1-(3-chloro-5-fluorophenyl)-5,5-difluoro-3-(trifluoromethyl)-4,5,6,7-tetrahydro-1*H*-indol-4-ol;
1-(3,5-difluorophenyl)-5,5-difluoro-3-(methylsulfonyl)-4,5,6,7-tetrahydro-1*H-*indol-4-ol;
1-(3,5-difluorophenyl)-5,5-difluoro-3-(methylsulfonyl)-1,5,6,7-tetrahydro-4*H-*indol-4-one;
3-(3-chloro-5-fluorophenyl)-6,6-difluoro-1-(trifluoromethyl)-4,5,6,7-tetrahydro-1*H*-indol-7-ol;
1-(3-chloro-4-fluorophenyl)-5,5-difluoro-3-(trifluoromethyl)-4,5,6,7-tetrahydro-1*H*-indol-4-ol;
5-(5,5-difluoro-4-hydroxy-3-(trifluoromethyl)-4,5,6,7-tetrahydro-1*H*-indol-1-yl)-2-fluorobenzonitrile;
4-(5,5-difluoro-4-hydroxy-3-(trifluoromethyl)-4,5,6,7-tetrahydro-1*H*-indol-1-yl)-2-fluorobenzonitrile;
5,5-difluoro-1-(1-methyl-1*H*-pyrrol-2-yl)-3-(trifluoromethyl)-4,5,6,7-tetrahydro-1*H*-indol-4-ol;
2-chloro-4-(5,5-difluoro-4-hydroxy-3-(trifluoromethyl)-4,5,6,7-tetrahydro-1*H-*indol-1-yl)benzonitrile;
4-(5,5-difluoro-4-hydroxy-3-(trifluoromethyl)-4,5,6,7-tetrahydro-1*H*-indol-1-yl)phthalonitrile;
5,5-difluoro-1-(furan-2-yl)-3-(trifluoromethyl)-4,5,6,7-tetrahydro-1*H*-indol-4-ol;
2-cyano-5-(5,5-difluoro-4-hydroxy-3-(trifluoromethyl)-4,5,6,7-tetrahydro-1*H-*indol-1-yl)benzoic acid;
2-acetyl-4-(5,5-difluoro-4-hydroxy-3-(trifluoromethyl)-4,5,6,7-tetrahydro-1*H-*indol-1-yl)benzonitrile;
1-(3-chloro-5-fluorophenyl)-5,6-difluoro-3-(methylsulfonyl)-4,5,6,7-tetrahydro-1*H*-indol-4-ol;
1-(4-chlorophenyl)-5,6-difluoro-3-(trifluoromethyl)-4,5,6,7-tetrahydro-1*H*-indol-4-ol;
2-fluoro-5-((4S)-5-fluoro-4-hydroxy-3-(trifluoromethyl)-4,5,6,7-tetrahydro-1*H*- indol-1-yl)benzonitrile;
5,6-difluoro-1-(5-fluoropyridin-3-yl)-3-(trifluoromethyl)-4,5,6,7-tetrahydro-1*H-*indol-4-ol;
5,5-difluoro-1-(5-fluoropyridin-3-yl)-3-(trifluoromethyl)-4,5,6,7-tetrahydro-1*H-*indol-4-ol;
(*S*)-5-(5,5-difluoro-4-hydroxy-3-(methylsulfonyl)-4,5,6,7-tetrahydro-1*H*-indol-1-yl)-2-fluorobenzonitrile;
1-(3-chloro-5-fluorophenyl)-5,5-difluoro-3-((S)-methylsulfinyl)-4,5,6,7-tetrahydro-1*H*-indol-4-ol;
1-(3-chloro-5-fluorophenyl)-5,5-difluoro-3-((R)-methylsulfinyl)-4,5,6,7-tetrahydro-1*H*-indol-4-ol;
2-chloro-5-(5,5-difluoro-4-hydroxy-3-(trifluoromethyl)-4,5,6,7-tetrahydro-1*H*- indol-1-yl)benzonitrile;
1-(4-chloro-3-nitrophenyl)-5,5-difluoro-3-(trifluoromethyl)-4,5,6,7-tetrahydro-1*H-*indol-4-ol;
3-fluoro-5-(5-fluoro-4-hydroxy-3-(trifluoromethyl)-4,5,6,7-tetrahydro-1H-indol-1-yl)benzonitrile;
1-(3-amino-5-fluorophenyl)-5-fluoro-3-(trifluoromethyl)-4,5,6,7-tetrahydro-1*H-*indol-4-ol;
1-(3-chloro-5-fluorophenyl)-5,7-difluoro-3-(trifluoromethyl)-4,5,6,7-tetrahydro-1*H*-indol-4-ol;
1-(3-chloro-5-fluorophenyl)-5-fluoro-3-(trifluoromethyl)-4,5,6,7-tetrahydro-1*H-*indol-4-ol;
1-(3-chloro-5-fluorophenyl)-5-fluoro-2-methyl-3-(trifluoromethyl)-4,5,6,7-tetrahydro-1*H*-indol-4-ol;
1-(3-chloro-5-fluorophenyl)-5,5-difluoro-3-phenyl-1,5,6,7-tetrahydro-4*H*-indol-4-one;
1-(3-chloro-5-fluorophenyl)-5,5-difluoro-3-phenyl-4,5,6,7-tetrahydro-1*H*-indol-4-ol;
(*S*)-2-bromo-1-(3,5-difluorophenyl)-5,5-difluoro-3-(trifluoromethyl)-4,5,6,7-tetrahydro-1*H*-indol-4-ol;
5,5-difluoro-1-(thiophen-3-yl)-3-(trifluoromethyl)-4,5,6,7-tetrahydro-1*H*-indol-4-ol;
5,5-difluoro-3-(trifluoromethyl)-1-(3,4,5-trifluorophenyl)-4,5,6,7-tetrahydro-1*H-*indol-4-ol;
1-(3,5-dichloro-4-fluorophenyl)-5,5-difluoro-3-(trifluoromethyl)-4,5,6,7-tetrahydro-1*H*-indol-4-ol;
3-(3-chloro-5-fluorophenyl)-6,7-difluoro-1-(trifluoromethyl)-5,6,7,8-tetrahydroindolizin-8-ol;
3-(3-chloro-5-fluorophenyl)-6,7-difluoro-1-(trifluoromethyl)-5,6,7,8-tetrahydroimidazo[1,5-a]pyridin-8-ol;
3-(3-chloro-5-fluorophenyl)-5,6-difluoro-1-(trifluoromethyl)-4,5,6,7-tetrahydro-1*H*-indol-7-ol;
3-(3-chloro-5-fluorophenyl)-5,6-difluoro-1-(trifluoromethyl)-4,5,6,7-tetrahydro-1*H*-indazol-7-ol;
3-chloro-5-(5,5-difluoro-4-hydroxy-3-(trifluoromethyl)-4,5,6,7-tetrahydro-1*H-*indol-1-yl)benzonitrile;
4-(5,5-difluoro-4-hydroxy-3-(trifluoromethyl)-4,5,6,7-tetrahydro-1*H*-indol-1-yl)-2-methoxybenzonitrile;
5,5-difluoro-1-(4-fluoro-3-(trifluoromethyl)phenyl)-3-(trifluoromethyl)-4,5,6,7-tetrahydro-1*H*-indol-4-ol;
4-(5,5-difluoro-4-hydroxy-3-(trifluoromethyl)-4,5,6,7-tetrahydro-1*H*-indol-1-yl)-2-(trifluoromethyl)benzonitrile;
5,5-difluoro-1-(3-fluoro-4-methoxyphenyl)-3-(trifluoromethyl)-4,5,6,7-tetrahydro-1*H*-indol-4-ol;
5,5-difluoro-1-(pyridin-4-yl)-3-(trifluoromethyl)-4,5,6,7-tetrahydro-1*H*-indol-4-ol;
5,5-difluoro-1-(4-fluorophenyl)-3-(trifluoromethyl)-4,5,6,7-tetrahydro-1*H*-indol-4-ol;
5,5-difluoro-1-(3-(methylsulfonyl)phenyl)-3-(trifluoromethyl)-4,5,6,7-tetrahydro-1*H*-indol-4-ol;
5,5-difluoro-1-(2-methoxypyridin-4-yl)-3-(trifluoromethyl)-4,5,6,7-tetrahydro-1*H-*indol-4-ol;
4-(5,5-difluoro-4-hydroxy-3-(trifluoromethyl)-4,5,6,7-tetrahydro-1*H*-indol-1-yl)picolinonitrile;
1-(3,4-difluorophenyl)-5,5-difluoro-3-(trifluoromethyl)-4,5,6,7-tetrahydro-1*H-*indol-4-ol;
5,5-difluoro-1-(2-fluoropyridin-4-yl)-3-(trifluoromethyl)-4,5,6,7-tetrahydro-1*H-*indol-4-ol;
1-(3-cyano-4-fluorophenyl)-5,5-difluoro-4-hydroxy-4,5,6,7-tetrahydro-1*H*-indole-3-carbonitrile;
(*S*)-4-(5,5-difluoro-4-hydroxy-3-(trifluoromethyl)-4,5,6,7-tetrahydro-1*H*-indol-1-yl)phthalonitrile;
1-(3-(difluoromethyl)-4-fluorophenyl)-5,5-difluoro-3-(trifluoromethyl)-4,5,6,7-tetrahydro-1*H*-indol-4-ol;
5,5-difluoro-1-(4-fluoro-3-methylphenyl)-3-(trifluoromethyl)-4,5,6,7-tetrahydro-1*H*-indol-4-ol;
5,5-difluoro-1-(6-fluoropyridin-3-yl)-3-(trifluoromethyl)-4,5,6,7-tetrahydro-1*H-*indol-4-ol;
5,5-difluoro-1-(5-fluoropyridin-2-yl)-3-(trifluoromethyl)-4,5,6,7-tetrahydro-1*H-*indol-4-ol;
1-(2-chloropyridin-4-yl)-5,5-difluoro-3-(trifluoromethyl)-4,5,6,7-tetrahydro-1*H-*indol-4-ol;
(*S*)-1-(3-(difluoromethyl)-4-fluorophenyl)-5,5-difluoro-3-(trifluoromethyl)-4,5,6,7-tetrahydro-1*H*-indol-4-ol;
(*R*)-1-(3-(difluoromethyl)-4-fluorophenyl)-5,5-difluoro-3-(trifluoromethyl)-4,5,6,7-tetrahydro-1*H*-indol-4-ol;
(*R*)-5-(5,5-difluoro-4-hydroxy-3-(trifluoromethyl)-4,5,6,7-tetrahydro-1*H*-indol-1-yl)-2-fluorobenzonitrile;
5-(5,5-difluoro-4-hydroxy-3-(trifluoromethyl)-4-vinyl-4,5,6,7-tetrahydro-1*H*-indol-1-yl)-2-fluorobenzonitrile;
(*S*)-5-(5,5-difluoro-4-hydroxy-3-(trifluoromethyl)-4,5,6,7-tetrahydro-1*H*-indol-1-yl)nicotinonitrile;
5,5-difluoro-1-(4-fluorophenyl)-3-(trifluoromethyl)-4,5,6,7-tetrahydro-1*H*-indole-4-carbonitrile;
5-(5,5-difluoro-4-hydroxy-6-methyl-3-(trifluoromethyl)-4,5,6,7-tetrahydro-1*H-*indol-1-yl)-2-fluorobenzonitrile;
5-((4*S*)-5,5-difluoro-4-hydroxy-6-methyl-3-(trifluoromethyl)-4,5,6,7-tetrahydro-1*H*-indol-1-yl)-2-fluorobenzonitrile;
(*E*)-5,5-difluoro-1-styryl-3-(trifluoromethyl)-4,5,6,7-tetrahydro-1*H*-indol-4-ol;
(*E*)-5,5-difluoro-1-styryl-3-(trifluoromethyl)-1,5,6,7-tetrahydro-4*H*-indol-4-one;
1-(phenylsulfonyl)-3-(trifluoromethyl)-4,5,6,7-tetrahydro-1*H*-indol-4-ol;
(*E*)-1-(2-cyclohexylvinyl)-5,5-difluoro-3-(trifluoromethyl)-1,5,6,7-tetrahydro-4*H-*indol-4-one;
5,5-difluoro-1-(phenylsulfonyl)-3-(trifluoromethyl)-1,5,6,7-tetrahydro-4*H*-indol-4-one;
5,5-difluoro-1-(phenylsulfonyl)-3-(trifluoromethyl)-4,5,6,7-tetrahydro-1*H*-indol-4-ol;
(*E*)-1-(2-cyclohexylvinyl)-5,5-difluoro-3-(trifluoromethyl)-4,5,6,7-tetrahydro-1*H-*indol-4-ol;
5-(5,5-difluoro-4-oxo-3-(trifluoromethyl)-4,5,6,7-tetrahydro-1*H*-indol-1-yl)-2-fluorobenzonitrile;
5-(5,5-difluoro-4-oxo-3-(trifluoromethyl)-4,5,6,7-tetrahydro-1*H*-indol-1-yl)-2-fluorobenzaldehyde;
1-(3-(difluoromethyl)-4-fluorophenyl)-5,5-difluoro-3-(trifluoromethyl)-1,5,6,7-tetrahydro-4H-indol-4-one;
2-fluoro-5-(5-fluoro-4-oxo-3-(trifluoromethyl)-4,5,6,7-tetrahydro-1*H*-indol-1-yl)benzaldehyde;
1-(3-(difluoromethyl)-4-fluorophenyl)-5-fluoro-3-(trifluoromethyl)-1,5,6,7-tetrahydro-4*H*-indol-4-one;
3-fluoro-5-(5-fluoro-4-hydroxy-3-(methylsulfonyl)-4,5,6,7-tetrahydro-1*H*-indol-1-yl)benzonitrile;
3-(3-chloro-5-fluorophenyl)-7,7-difluoro-1-(trifluoromethyl)-5,6,7,8-tetrahydroimidazo[1,5-a]pyridin-8-ol;
3-(3-chloro-5-fluorophenyl)-6,6-difluoro-1-(trifluoromethyl)-4,5,6,7-tetrahydro-1*H*-indazol-7-ol;
(*S*)-1-(3,5-difluorophenyl)-5,5-difluoro-3-(methylsulfonyl)-2-(trifluoromethyl)-4,5,6,7-tetrahydro-1*H*-indol-4-ol;
(*S*)-2-fluoro-5-(4,5,5-trifluoro-3-(trifluoromethyl)-4,5,6,7-tetrahydro-1*H*-indol-1-yl)benzonitrile;
(*R*)-2-fluoro-5-(4,5,5-trifluoro-3-(trifluoromethyl)-4,5,6,7-tetrahydro-1*H*-indol-1-yl)benzonitrile;
(*Z*)-5-(5,5-difluoro-4-(hydroxyimino)-3-(trifluoromethyl)-4,5,6,7-tetrahydro-1*H-*indol-1-yl)-2-fluorobenzonitrile; or
(*S*)-5-(5,5-difluoro-4-hydroxy-3-(trifluoromethyl)-4,5,6,7-tetrahydro-1*H*-indol-1-yl-4-d)-2-fluorobenzonitrile.

25. A pharmaceutical composition, wherein, comprising at least one compound of any one of claims 1-24 and at least one pharmaceutically acceptable excipient.

26. Use of the compound according to any one of claims 1-24 or the pharmaceutical composition of claim 25 for treating disease mediated by HIF-2α.

27. The use of claim 26, wherein, the disease is VHL syndrome, autoimmune disease, inflammatory disease and/or cancer.

28. The use of claim 27, wherein, the cancer is selected from hematological cancer, lymphoma, multiple myeloma, digestive system tumor, reproductive system tumor, brain tumor, nervous system tumor, neoplasm.

29. The use of claim 28, wherein, the cancer is selected from glioma, pheochromocytoma, paraganglioma, colon cancer, rectal cancer, prostate cancer, lung cancer, pancreatic cancer, liver cancer, kidney cancer, cervical cancer, uterine cancer, stomach cancer, ovarian cancer, breast cancer, skin cancer, brain cancer, meningioma, neurocytoma, meningioma and medulloblastoma.

30. A method for treating or preventing HIF-2α mediated disease, wherein, comprising administering a therapeutically effective amount of the compound of any one of claims 1-24, or the pharmaceutical composition to a subject.

31. The method of claim 30, wherein, the disease mediated by HIF-2α is cancer.

32. The method of claim 31, wherein, the cancer is selected from glioma, pheochromocytoma, paraganglioma, colon cancer, rectal cancer, prostate cancer, lung cancer, pancreatic cancer, liver cancer, kidney cancer, cervical cancer, uterine cancer, stomach cancer, ovarian cancer, breast cancer, skin cancer, brain cancer, meningioma, neurocytoma, meningioma and medulloblastoma.
